# EUROPEAN PATENT SPECIFICATION

(11) **EP 2 046 147 B1**
(45) Date of publication and mention of the grant of the patent: **01.05.2013**
(21) Application number: 07763961.5
(22) Date of filing: 24.07.2007
(51) Int. Cl.: A23L 1/226, C07D 317/24, C07D 317/26, C07D 319/06, C07D 405/04, C11C 3/06

(54) **METHOD OF USING ORGANIC COMPOUNDS**
VERFAHREN ZUR VERWENDUNG VON ORGANISCHEN VERBINDUNGEN
PROCÉDÉ D'UTILISATION DE COMPOSÉS ORGANIQUES

(30) Priority: 28.07.2006 SG 200605098
(43) Date of publication of application: 15.04.2009
(73) Proprietor: Givaudan SA, 1214 Vernier-Genève (CH)
(72) Inventor: GRAB, Willi, 2552 Orpund (CH)
(74) Representative: Givaudan Patents
(86) International application number: PCT/CH2007/000365
(87) International publication number: WO 2008/011742

(56) References cited:
- WO-A-01/02474
- WO-A-03/082850
- GB-A- 1 352 092
- JP-A- 2001 181 271
- DATABASE WPI Week 200246 Derwent Publications Ltd., London, GB; AN 2002-430038 XP002454696 & JP 2002 069068 A (KAO CORP) 8 March 2002 (2002-03-08)

## Description

The present invention relates to flavor precursors that can be added to food to slowly release the flavor upon heating and consumption.

Food products are rendered more palatable and more attractive for consumers by adding various flavors to them. However, when food products are processed or stored for a longer period of time, many flavors get lost at least partially, in particular the volatile flavors. For this reason, many attempts at encapsulation of flavors (binding to matrixes or coating to prevent premature release) have been made to retard the flavor release and prolong shelf life. These however are only partially successful, and there remains a need of a method orflavor product that keeps the main part of volatile flavors inside the food product to be slowly released upon consumption of the food by the consumer.

GB-A-1352092 - discloses that certain dioxolane derivatives may be used as flavourings in aroma compositions. WO 2003/082850 discloses that some aldoxane derivatives are capable of releasing an active aldehyde in a controlled manner, as well as protecting said aldehyde, from a chemically aggressive medium into which it has to be added prior to its release or use.

Applicant has now found that the use of flavor precursors, according to formula I (monoglyceride acetals and ketals) below in flavor compositions and food products provides a means to retard the premature release of volatile flavors and slowly release them upon consumption. Thereby an early unwanted flavor release, for example during storage or processing, is minimised, the shelf life of the flavoured food product is prolonged and the flavor of the food product is improved.

A flavor precursor compound according to formula I 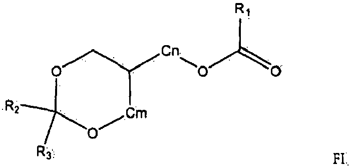 wherein n and m are selected from 0 and 1, and if n is 1 then m is 0, and if n is 0 then m is 1; wherein R1 is selected from the group consisting of
a straight-chain C7 to C17 alkyl, a straight-chain C7 to C17 alkenyl, a branched C7 to C17 alkyl, a branched C7 to C17 alkenyl,
a straight-chain C7 to C17 monoalkenyl, a branched C7 to C17 monoalkenyl,
a straight-chain C7 to C17 alkadienyl, a branched C7 to C17 alkadienyl,
a straight-chain C7 to C17 alkatrienyl, a branched C7 to C17 alkatrienyl,
a straight-chain C9 to C17 alkatetraenyl, a branched C9 to C17 alkatetraenyl,
and a straight-chain C11 to C17 alkapentaenyl, a branched C11 to C17 alkapentaenyl, and wherein R2 is selected from the group consisting of H, a C1 to C15 alkyl, a C1 to C15 oxoalkyl, a C1 to C15 hydroxyalkyl, C2 to C15 alkenyl, a C2 to C15 oxoalkenyl, and a C2 to C15 hydroxyalkenyl, and R3 is selected from the group consisting of C1 to C15 straight-chain alkyl comprising one or two substituents independently selected from O, OH, N, NH, phenyl, furanyl, C9 aryl, C10 aryl, SH, and SR4, wherein R4 is a straight-chain or branched C1 to C5 alkyl residue optionally selected from methyl, ethyl, propyl, isopropyl, butyl, isobutyl, pentyl and isopentyl, and the total numberof carbon atoms in R3 and R4 is up to 15, C1 to C15 straight-chain alkyl, comprising one or two atoms independently selected from O,S or N within the alkyl chain, C3 to C15 singly, doubly or multiply branched alkyl optionally substituted with one or two residues independently selected from O, OH, N, NH, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15,
C3 to C15 singly, doubly or multiply branched alkyl comprising one or two atoms independently selected from O,S or N within the alkyl chain, C2 to C15 straight-chain alkenyl, C3 to C15 straight-chain alkadienyl, C2 to C15 straight-chain alkenyl optionally substitued with one or more residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C2 to C15 straight-chain alkenyl, optionally comprising one or more atoms independently selected from O,S and N within the alkyl chain,
C3 to C15 branched alkenyl, optionally substituted with one or two alkyl groups,
C2 to C15 branched alkenyl comprising one or two alkyl groups optionally substituted with one or two residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C3 to C15 branched alkenyl comprising one or two alkyl groups and one or more atoms independently selected from O,Sand N within the alkyl chain,
C5 to C15 singly; doubly, or multiply branched alkadienyl, optionally substituted with one or two alkyl groups,
C4 to C15 singly, doubly, or multiply branched alkadienyl comprising one or more atoms independently selected from O,S and N within the alkenyl chain;
a 5- or 6-membered carbon ring residue comprising up to two heteroatoms independently selected from 0, S, and N, optionally subsituted with one or more residues independently selected from 0, OH, alkoxy, alkyl, alkenyl;
a C1 to C6 alkyl or alkenyl substituted with a 5- or 6-membered carbon ring residue comprising up to two heteroatoms independently selected from 0, S, and N, optionally subsituted with one or more residues independently selected from O, OH, alkoxy, alkyl, alkenyl; or wherein R2-C-R3 form a ring residue selected from oxacyclopentan optionally substituted with a residue selected from methyl, dimethyl, ethyl, hydroxy, and methoxy, oxacyclopentene optionally substituted with a residue selected from methyl, dimethyl, ethyl, hydroxy, and methoxy, hydroxycyclopentene, hydroxyalkylcyclopentene, hydroxymethylcyclopentene, hydroxydimethylcyclopentene, ethylhydroxycyclopentene, and ethylhydroxymethylcyclopentene, thiacyclopentane, alkylthiacyclopentane, and alkyl-3-thia-cyclopentane, oxa-cyclopentene, 2-oxacyclopentene, 3-oxacyclopentene, alkyloxacylcopentene, alkyl-3-oxacyclopentene, methyl-3-oxa-cyclopentene, alkyl-3-oxacyclopentene, dimethyl-3-oxacyclopentene, ethyl-3-oxacyclopentene, ethylmethyl-3-oxacyclopentene, hydroxyalkyl-3-oxacyclopentene, hydroxymethyl-3-oxacyclopentene, hydroxydimethyl-3-oxacyclopentene, hydroxyethyl-3-oxacyclopentene, ethylhydroxymethyl-3-oxacyclopentene, oxacyclopentane, 3-oxacyclopentan, alkyl-3-oxacyclopentan, methyl-3-oxa-cyclopentan, dimethyl-3-oxacyclopentan, ethyl-3-oxacyclopentan, ethylmethyl alkyl-3-oxacyclopentan, alkylcyclopenten, alkenylcyclopenten, alkylalkenylcyclopenten, methyhylopenten, dimethylcyclopenten, ethylmethylcyclopenten, propylmethylcyclopenten, butylmethylcyclopenten, butenylmethylcyclopenten, pentylmethylcyclopenten, pentenylmethylcyclopenten, alkylcarboxy-alkenyl-cyclopentan, alkylcarboxy-alkyl-cyclopentan, methylcarboxy-alkenyl-cyclopentan, methylcarboxy-alkyl-cyclopentan, alkylcarboxy-pentenyl-cyclopentan, alkylcarboxy-pentyl-cyclopentan, methylcarboxy-alkenyl-cyclopentan, and methylcarboxy-alkyl-cyclopentan, and wherein the alkyl is selected from methyl, ethyl, propyl, butyl, pentyl, pentenyl, hexyl, heptyl, and octyl, oxo-alkylcyclohexen, oxo-methylcyclohexen, oxo-trimethylcyclohexen, oxo-ethylcyclohexen, oxo-ethylmethylcyclohexen, oxo-propylcyclohexen, oxo-propylmethylcyclohexen, oxo-isopropylcyclohexen, oxo-isopropylmethylcyclohexen, oxo-thiopropylcyclohexen, oxo-thiopropylmethylcyclohexen, alkylcyclohexen, methylcyclohexen, trimethylcyclohexen, ethylcyclohexen, ethylmethylcyclohexen, propylcyclohexen, propylmethylcyclohexen, isopropylcyclohexen, isopropylmethylcyclohexen, thiopropylcyclohexen, thiopropylmethylcyclohexen, alkylcyclohexan, methylcyclohexan, trimethylcyclohexan, ethylcyclohexan, ethylmethylcyclohexan, propylcyclohexan, propylmethylcyclohexan, isopropylcyclohexan, isopropylmethylcyclohexan, thropropylcyclohexan, thiopropylmethylcyclohexan;
and wherein R2 and R3 together have a total number of carbon atoms of up to 15, and R2-C-R3 has a total number of carbon atoms of up to 16,

Certain alpha monoglyceride acetals/ketals of Fl with m=0 and n=1, and their synthesis, have been described in JP2001181271; these are useful as intermediates for the synthesis of monoglycerides, which are used as emulsifiers or moisturizers in cosmetics.

### Summary of the invention

In a first aspect, there is provided a method of providing a flavored food product, wherein at least one flavor precursor of formula I 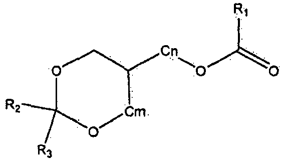 wherein n and m are selected from 0 and 1, and if n is 1 then m is 0, and if n is 0 then m is 1;
wherein R1 is selected from the group consisting of
a straight-chain C7 to C17 alkyl, astraight-chain C7 to C17 alkenyl, a branched C7 to C17 alkyl, a branched C7 to C17 alkenyl,
a straight-chain C7 to C17 monoalkenyl, a branched C7 to C17 monoalkenyl,
a straight-chain C7 to C17 alkadienyl, a branched C7 to C17 alkadienyl,
a straight-chain C7 to C17 alkatrienyl, a branched C7 to C17 alkatrienyl,
a straight-chain C9 to C17 alkatetraenyl, a branched C9 to C17 alkatetraenyl;
and a straight-Chain C11 to C17 alkapentaenyl, a branched C11 to C17 alkapentaenyl,
wherein the
R2 is selected from the group consisting of H, a C1 to C15 alkyl, a C1 to C15 oxoalkyl, a C1 to C15 hydroxyalkyl, C2 to C15 alkenyl, a C2 to C15 oxoalkenyl, and a C2 to C15 hydroxyalkenyl, and R3 is selected from the group consisting of C1 to C15 straight-chain alkyl comprising one or two substituents independently selected from O, OH, N, NH, phenyl, furanyl, C9 aryl, C10 aryl, SH, and SR4, wherein R4 is a straight-chaih or branched C1 to C5 alkyl residue optionally selected from methyl, ethyl, propyl, isopropyl, butyl, isobutyl, pentyl and isopentyl, and the total number of carbon atoms in R3 and R4 is up to 15, C1 to C15 straight-chain alkyl, comprising one or two atoms independently selected from O,S or N within the alkyl chain, C3 to C15 singly, doubly or multiply branched alkyl optionally substituted with one or two residues independently selected from O, OH, N, NH, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15,
C3 to C15 singly, doubly or multiply branched alkyl comprising one or two atoms independently selected from O,S or N within the alkyl chain, C2 to C15 straight-chain alkenyl, C3 to C15 straight-chain alkadienyl, C2 to C15 straight-chain alkenyl optionally substitued with one or more residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C2 to C15 straight-chain alkenyl, optionally comprising one or more atoms independently selected from O,S and N within the alkyl chain,
C3 to C15 branched alkenyl, optionally substituted with one or two alkyl groups,
C2 to C15 branched alkenyl comprising one or two alkyl groups optionally substituted with one or two residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C3 to C15 branched alkenyl comprising one or two alkyl groups and one or more atoms independently selected from O,Sand N within the alkyl chain,
C5 to C15 singly, doubly, or multiply branched alkadienyl, optionally substituted with one or two alkyl groups, C4 to C15 singly, doubly, or multiply branched alkadienyl comprising one or more atoms independently selected from O,S and N within the alkenyl chain;
a 5- or 6-membered carbon ring residue comprising up to two heteroatoms independently selected from O,S, and N, optionally subsituted with one or more residues independently selected from O, OH, alkoxy, alkyl; alkenyl;
a C1 to C6 alkyl or alkenyl substituted with a 5- or 6-membered carbon ring residue comprising up to two heteroatoms independently selected from 0, S, and N, optionally subsituted with one or more residues independently selected from O, OH, alkoxy, alkyl, alkenyl; or wherein R2-C-R3 form a ring residue selected from oxacyclopentan optionally substituted with a residue selected from methyl, dimethyl, ethyl, hydroxy, and methoxy, oxacyclopentene optionally substituted with a residue selected from methyl, dimethyl, ethyl, hydroxy, and methoxy, hydroxycyclopentene, hydroxyalkylcyclopentene, hydroxymethylcyclopentene, hydroxydimethylcyclopentene, ethylhydroxycyclopentene, and ethylhydroxymethylcyclopentene, thiacyclopentane, alkythiacyclopentane, and alkyl-3-thia-cyclopentane, oxa-cyclopentene, 2-oxacyclopentene, 3-oxacyclopentene, alkyloxacylcopentene, alkyl-3-oxacyclopentene, methyl-3-oxa-cyclopentene, alkyl-3-oxacyclopentene, dimethyl-3-oxacyclopentene, ethyl-3-oxacyclopentene, ethylmethyl-3-oxacyclopentene, hydroxyalkyl-3-oxacyclopentene, hydroxymethyl-3-oxacyclopentene, hydroxydimethyl-3-oxacyclopentene, hydroxyethyl-3-oxacyclopentene, ethylhydroxymethyl-3-oxacyclopentene, oxacyclopentane, 3-oxacyclopentan, alkyl-3-oxacyclopentan, methyl-3-oxa-cyclopentan, dimethyl-3-oxacyclopentan, ethyl-3-oxacyclopentan, ethylmethyl alkyl-3-oxacyclopentan, alkylcyclopenten, alkenylcyclopenten, alkylalkenylcyclopenten, methylcylopenten, dimethylcyclopenten, ethylmethylcyclopenten, propylmethylcyclopenten, butylmethylcyclopenten, butenylmethylcyclopenten, pentylmethylcyclopenten, pentenylmethylcyclopenten, alkylcarboxy-alkenyl-cyclopentan, alkylcarboxy-alkylcyclopentan, methylcarboxy-alkenyl-cyclopentan, methylcarboxy-alkyl-cyclopentan, alkylcarboxy-pentenyl-cyclopentan, alkylcarboxy-pentyl-cyclopentan, methylcarboxy-alkenyl-cyclopentan, and methylcarboxy-alkyl-cyclopentan, and wherein the alkyl is selected from methyl, ethyl, propyl, butyl, pentyl, pentenyl, hexyl, heptyl, and octyl, oxo-alkylcyclohexen, oxo-methylcyclohexen, oxo-trimethylcyclohexen, oxo-ethylcyclohexen, oxo-ethylmethylcyclohexen, oxo-propylcyclohexen, oxo-propylmethylcyclohexen, oxo-isopropylcyclohexen, oxo-isopropylmethylcyclohexen, oxo-thiopropylcyclohexen, oxo-thiopropylmethylcyclohexen, alkylcyclohexen, methylcyclohexen, trimethylcyclohexen, ethylcyclohexen, ethylmethylcyclohexen, propylcyclohexen, propylmethylcyclohexen, isopropylcyclohexen, isopropylmethylcyclohexen, thiopropylcyclohexen; thiopropylmethylcyclohexen, alkylcyclohexan, methylcyclohexan, trimethylcyclohexan, ethylcyclohexan, ethylmethylcyclohexan, propykyclohexan, propylmethylcyclohexan, isopropylcyclohexan, isopropylmethylcyclohexan, thiopropylcyclohexan, thiopropylmethylcyclohexan;
and wherein R2 and R3 together have a total number of carbon atoms of up to 15, and R2-C-R3 has a total number of carbon atoms of up to 16,
is admixed to a food product in a sufficient concentration to release a flavor of noticeable aroma upon consumption and/or heating of the food product.

In another aspect, there is provided a flavor composition comprising at least one flavor precursor defined as described herein-above.

In another aspect, there is provided a flavor composition comprising a mixture of flavor precursors as defined herein formed by reacting at least one flavor compound comprising one or more carbonyl group with at least one monoglyceride in an acid catalyzed reaction.

In another aspect, there is provided a food product comprising at least one flavor precursor defined as described herein-above.

In another aspect, there is provided a food product comprising a mixture of flavor precursors as defined in any one of claims 1 to 7 formed by reacting at least one flavor compound comprising one or more carbonyl group with at least one monoglyceride in an acid catalyzed reaction.

In another aspect, there is provided at least one flavor pecursor compound defined as described herein-above wherein m=1 and n=0.

In another aspect, there is provided a process of producing the flavor precursor of claim 10 or claim 11 by reacting at least one flavor compound comprising one or more carbonyl group with at least one monoglyceride in an acid catalyzed reaction.

### Detailed description of the invention:

There is provided a method as described herein-above wherein said flavor is selected from the group consisting of flavors as described herein-below.

There is provided a method as described herein-above wherein
R2 is selected from the group consisting of H, a C1 to C15 alkyl, a C1 to C15 oxoalkyl, a C1 to C15 hydroxyalkyl, C2 to C15 alkenyl, a C2 to C15 oxoalkenyl, and a C2 to C15 hydroxyalkenyl,
R3 is selected from the group consisting of C1 to C15 straight-chain alkyl comprising one or two substituents independently selected from O, OH, N, NH, phenyl, furanyl, C9 aryl, C10 aryl, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15, C1 to C15 straight-chain alkyl, comprising one or two atoms independently selected from O,S or N within the alkyl, chain, C3 to C15 singly, doubly or multiply branched alkyl optionally substituted with one or two residues independently selected from O, OH, N, NH, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15,
C3 to C15 singly, doubly or multiply branched alkyl comprising one or two atoms independently selected from O,S or N within the alkyl chain, C2 to C15 straight-chain alkenyl, C3 to C15 straight-chain alkadienyl,
C2 to C15 straight-chain alkenyl optionally substitued with one or more residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C2 to C15 straight-chain alkenyl, optionally comprising one or more atoms independently selected from O,S and N within the alkyl chain,
C3 to C15 branched alkenyl, optionally substituted with one or two alkyl groups,
C2 to C15 branched alkenyl comprising one or two alkyl groups optionally substituted with one or two residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C3 to C15 branched alkenyl comprising one or two alkyl groups and one or more atoms independently selected from O,S and N within the alkyl chain,
C5 to C15 shingly, doubly, or multiply branched alkadienyl, optionally substituted with one or two alkyl groups, C4 to C15 singly, doubly, or multiply branched alkadienyl comprising one or more atoms independently selected from O,S and N within the alkenyl chain;
a 5- or 6-membered carbon ring residue comprising up to two heteroatoms independently selected from 0, S, and N, optionally-subsituted with one or more residues independently selected from 0, OH, alkoxy, alkyl, alkenyl;
a C1 to C6 alkyl or alkenyl substituted with a 5- or 6-membered carbon ring residue comprising up to two heteroatoms independently selected from O, S, and N, optionally subsituted with one or more residues independently selected from 0, OH, alkoxy, alkyl, alkenyl;
a R2-C-R3 ring residue selected from oxacyclopentan optionally substituted with a residue selected from methyl, dimethyl, ethyl, hydroxy, and methoxy, oxacydopentene optionally substituted with a residue selected from methyl, dimethyl, ethyl, hydroxy, and methoxy, hydroxycyclopentene, hydroxyalkylcyclopentene, hydroxymethylcyclopentene, hydroxydimethylcyclopentene, ethylhydroxycyclopentene, and ethylhydroxymethylcyclopentene, thiacyclopentane, alkylthiacyclopentane, and alkyl-3-thia-cyclopentane, oxa-cyclopentene, 2-oxacyclopentene, 3-oxacyclopentene, alkyloxacyclopentene, alkyl-3-oxacyclopentene, methyl-3-oxa-cyclopentene, alkyl-3-oxacyclopentene, dimethyl-3-oxacyclopentene, ethyl-3-oxacyclopentene, ethytmethyl-3-oxyclopentene, hydroxyalkyl-3-oxacyclopentene, hydroxymethyl-3-oxacyclopentene, hydroxydimethyl-3-oxacyclopentene, hydroxyethyl-3-oxacyclopentene, ethylhydroxymethyl-3-oxacyclopentene, oxacyclopentane, 3-oxacyclopentan, alkyl-3-oxacyclopentan, methyl-3-oxa-cyclopentan, dimethyl-3-oxacyclopentan, ethyl-3-oxacyclopentan, ethylmethyl alkyl-3-oxacyclopentan, alkylcyclopenten, alkenylcyclopenten, alkylalkenylcyclopenten, methylcylopenten, dimethylcyclopenten, ethylmethylcyclopenten, propylmethylcyclopenten, butylmethyclopenten, butenylmethylcyclopenten, pentylmethylcyclopenten, pentenylmethylcyclopenten, alkylcarboxy-alkenyl-cyclopentan, alkylcarboxy-alkyl-cyclopentan, methylcarboxy alkenyl-cyclopentan, methylcarboxy-alkyl-cyclopentan, alkylcarboxy-pentenyl-cyclopentan, alkylcarboxy-pentyl-kyclopentan, methylcarboxy-alkenyl-cyclopentan, and methylcarboxy-alkyl-cyclopentan, arid wherein the alkyl is selected from methyl, ethyl, propyl, butyl, pentyl, pentenyl, hexyl, heptyl, and octyl, oxo-alkylcyclohexen, oxo-methylcyclohexen, oxo-trimethylcyclohexen, oxo-ethylcyclohexen, oxo-ethylmethylcyclohexen, oxo-propylcyclohexen, oxo-propylmethlycyclohexen, oxo-isopropylcyclohexen, oxo-isopropylmethylcyclohexen, oxo-thiopropylcyclohexen, oxo-thiopropylmethylcyclohexen, alkylcyclohexen, methylcyclohexen, trimethylcyclohexen, ethylcyclohexen, ethylmethylcyclobexen, propylcyclohexen, propylmethylcyclohexen, isopropylcyclohexen, isopropylmethylcyclohexen, thiopropylcyclohexen, thiopropylmethylcyclohexen, alkylcyclohexan, methylcyclohexan, trimethylcyclohexan, ethylcyclohexan, ethylmethylcyclohexan, propylcyclohexan, propylmethylcyclohexan, isopropylcyplohexan, isopropylmethylcyclohexan, thiopropylcyclohexan, thiopropylmethylcyclohexan;
and wherein R2 and R3 together have a total number of carbon atoms of up to 15, and the R2-C-R3 has a total number of carbon atoms of up to 16.

In one embodiment, there is provided a method as described herein-above wherein R1 is an alkyl selected from the group consisting of C7, C8, C9, C10, C11, C13, C15, and C17 alkyl.

In another embodiment, there is provided a method as described herein-above wherein R1 is an Alkenyl selected from the group consisting of C7, C8, C9, C10, C11, C13, C15, and C17 alkenyl, a C17-8en (oleic acid residue) alkenyl, a C17-8,11 alka-dienyl (linoleic acid residue), and a C17-8, 11, 14-trienyl (linolenic acid residue).

In another embodiment, there is provided a method as described herein-above wherein R2 is selected from the group consisting of H, methyl and ethyl.

In another embodiment, there is provided a method as described herein-above wherein R3 is selected from the group consisting of a C1 to C8 straight-chain alkyl, a C1 to C8 branched alkyl comprising up to two alkyl groups, wherein the alkyl residue may contain one or more further alkyl residues, a C1 to C8 straight-chain alkyl comprising one or two substituents selected from O, and SR4, wherein R4 is an alkyl residue selected from methyl, and ethyl, a C2 to C8 straight-chain alkenyl, and a ring selected from a 5 membered and a 6 membered ring, comprising up to two N heteroatoms, wherein the ring may be further substituted with one or more alkyl groups selected from methyl, ethyl, propyl and isopropyl.

In another embodiment, there is provided a method as described herein-above wherein R1 is an alkyl selected from the group consisting of C7, C9, C11, C13, G15 and C17 alkyl, R2 is selected from the group consisting of H, methyl and ethyl, and R3 is selected from the group consisting of a C1 to C8 straight-chain alkyl, a C1 to C8 branched alkyl comprising one or two alkyl groups, wherein the alkyl residue may contain one or more further alkyl residues, a C1 to C8 straight-chain alkyl comprising one or two substituents selected from and SR4, wherein R4 is an alkyl residue selected from methyl, and ethyl, a C2 to C8 straight-chain alkenyl, and a ring selected from a 5 membered and 6 membered ring comprising up to two N heteroatoms, wherein the ring may be further substituted with one or more alkyl groups selected from methyl, ethyl, propyl and isopropyl.

The flavor precursors of formula I are acetals (for R2=H) or ketals (for R2=optionally substituted alkyl or alkenyl) of formula I shown herein-below. They slowly release volatile flavors (aroma) and thereby protect the chemically bound flavor from an undesired excessive release of the volatile flavor compound, for example during processing and storage of a food product. The flavor is slowly released in presence of water and enzymes that are present naturally in the mouth during consumption.

Flavor precursors for use in the present invention can be formed by reaction of flavor compounds comprising one or more carbonyl group with monoglycerides (1-acylglycerides and 2-acylglycerides) in an acid-catalyzed. reaction (compare reaction scheme below).

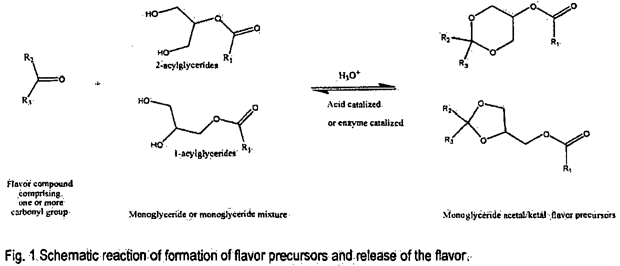

The compounds in the upper part show a 2-acylglyceride and its product, the compounds in the bottom part show the 1-acylglyceride and its product. Formation of the flavor precursors occurs in the presence of H⁺, see left to right direction of the scheme.

These flavor precursors will then slowly release the flavor (R2-CO-R3) in a reversed reaction when exposed to aqueous acidic condifions (compare right to left direcion, of the scheme), for example upon heating in the presence of water, for example by cooking or by baking and in particular in the mouth which contains enzymes that speed up the flavor release.

The reacted and later released flavors comprise the residues R2 and R3 and a carbonyl group (R2-CO₋R3). For example, a precursor of formula I wherein R2=Hydrogen and R3= pent-2-en-1-yl will release 3-hexenal, a precursor of formula I wherein R2=methyl and R3=1-oxobutyl will release 2,3-hexandione, a precursor of formula I wherein R2=H and R3=2-methylthioethyl will release methional.

Monoglycerides can be prepared from natural sources including plant and animal sources by enzymatic or chemical hydrolysis, as is well-known in the art. Some monoglycerides are available commercially as a mixture or in pure form (either extracted or synthesized). For example, without limitation, plant sources of monoglycerides include palm oil, oil seeds, sunflower seeds, nuts, cacoa beans, coconuts, hazelnuts, peanuts, and many more. For example, without limitation, aminal sources of monoglycerides include milk, butter, meat, chicken, beef, porc, lamb, fish, and many more.

A typical mixture of monoglycerides derived from natural sources includes monoglycerides bound to saturated fatty acids including laurylmonoglyceride (C12 - forms FI with R1=C11), myristylmonoglyceride (C14 - forms FI with R1=C13), palmitylmonoglyceride (C16 - forms FI with R1=C15), and stearoylmonoglyceride (C18 - forms FI with R1=C17). Monoglycerides comprising unsaturated fatty acids include oleylmonoglyceride, linolylmonoglyceride, and alpha-linolenylmonoglyeride.

A typical mixture of monoglycerides derived from cocoa butter includes palmitylmonoglyceride (C16), oleylmonoglyceride (C18-1, with one doubloe bond), and stearoylmonoglyceride (C18).

A typical mixture of monoglycerides derived from miglyol, a commonly used oil from vegetable sources), includes C8, C10, and C12 monoglycerides.

A typical mixture of monoglycerides derived from butter includes mainly C8, C10, C12, C14, C16, C18 saturated monoglycerides.

When used in food, monoglycerides or their residues (R1 in formula I) can partially degrade over time and release free fatty acid, which may turn a product rancid. When choosing a particular precursor for a particular food product, R1 of said percursor will be chosen so that upon degradation it releases e.g. caprylic acid that contribute to the desired flavor note rather than, for example, lauric acid, that will result in an undesirable soapy off-note in the chosen food product. The specific choice of appropriate R1 is well within the experience of the skilled person and depends on the food product and desired flavor note.

Commercial monoglycerides are mixtures of mainly 1-acylglycerides with variable amounts of 2-acylglycerides and other glycerides. 1-acylglycerides when reacted with flavor compounds as described herein will form flavor precursors of formula I wherein n=1 and m=0. 2-acylglycerides when reacted with flavor compounds as described herein will form favor precursors of formula I wherein n=0 and m=1.

Flavors are compounds that can be detected by the human olfactory system. To provide sensory properties, a flavor must have the following molecular properties: some water solubility, a sufficiently high vapor pressure, low polarity, and some ability to dissolve in fat (lipophilicity). Flavor compounds have a molecular weight of up to 294 (no larger compounds are known to trigger the human olfactory system).

Flavors useful for the methods described herein are compounds that comprise one or more carbonyl group and that can be reacted with the monoglycerides as described herein. These flavors include but are not limited to aldehydes, ketones, and other flavor classes provided they comprise one or more carbonyl groups.

Useful flavors include natural and artificial flavors, and extracts from natural sources that contain a mixture of flavor compounds, and flavor compounds as such. A number of suitable flavors can be found, for example, in the BACIS database (Boelens Aroma Chemical Information Service), which includes the Flavor-Base 2004 database (Leffingwell & Associates, Canton, Georgia, USA), in the listing of Flavor chemicals on the FDA (Food & Drug Administration, USA) & FEMA GRAS lists (FEMA - Flavor and Extracts Manufacturers Association, GRAS -Generally Recognised As Safe), and the European Community (EC) Register list.

Examples of useful flavor compounds include, but are not limited to (+)-8,9-DEHYDRONOOTKATONE; (1 R-CIS AND TRANS)-2-(1-ACETYLTHIO-1-METHYL)ETHYL-5-METHYLCYCLOHEXANONE; (E)-2-(2-OCTENYL)CYCLOPENTANONE; (E)-5-ISOPROPOXY-2-DECENAL; (E,E)-3,5-OCTADIEN-2-ONE; (METHYLTHIO)ACETONE; (RAC)-3-ACETYLOXY-5-METHYL-2-HEXANONE; 1-(2,5,5-TRIMETHYL-CYCLOPENT-1-EN-1-YL)-3-METHYL-2-BUTEN-1=ONE; 1-(3,5,5,-TRIMETHYL-1-CYCLOHEXENYL)-3-METHYL-2-BUTEN-1-ONE; 1(5-METHYLFURYL-2)-PROPANE-1,2-DIONE; 1-(METHYLTHIO)-2-BUTANONE; 1-(METHYLTHIO)-3-PENTANONE; 1-(METHYLTHIO)-OCTAN-3-ONE; 1-(P-METHOXYPHENYL)-2-PROPANONE; 1,4-DODEC-6-ENOLACTONE; 1,5,5-TRIMETHYL-8-ETHYL-G-OXABICYCLO (4.3.0) NON-6-EN-3-ONE; 1.5-[Z]-OCTADIEN-3-ONE; 12-METHYL-TRIDECANAL. 14-METHYL-PENTADECANAL; 1-HYDROXY-2-BUTANONE; 1-MERCAPTO-2-PROPANONE; 1-MERCAPTO-3-PENTANONE; 1-PENTEN-3-ONE; 2-(3,3-DIMETHYLCYCLOHEXYLIDEN-ETHANAL; 2-(METHYLTHIO)METHYL-2-BUTENAL; 2-(METHYLTHIOMETHYL)-3-PHENYLPROPENAL; 2,10-UNDECADIENAL; 2,3-DIHYDRO-2,3,3-TRIMETHYL-1H-INDEN-1-ONE; 2,4-DIMETHYL-HEPT-4-ENE-3-ONE; 2,4-PENTADIENAL; 2,5-DIETHYL-4-HYDROXY-5-METHYL-3(2H)-FURANONE; 2,5-DIMETHYL-3(2H)-FURANONE; 2,5-DIMETHYL-4-HYDROXY-6-HEPTANONE; 2,5-DIMETHYL-TETRAHYDRO-3-FURANONE; 2,6-DIMETHYL-3-HYDROXY-4-PYRONE; 2,6-DIMETHYL-4-HEPTANONE; 2,6-DIMETHYLBICYCLO[3.2.1]OCT-2-EN-7-ONE; 2-AMINO-AGETOPHENONE; 2-BUTYL-2-BUTENAL 2-ETHYL-4-METHYL-PENT-2-ENAL; 2-HEPTEN-4-ONE: 2-HEPTYL-BUTYROLACTONE; 2-HEXENAL. 2-HYDROXY-1-(4-HYDROXY-3-METHOXY-PHENYL)-ETHANONE; 2-HYDROXY-2-CYCLOHEXEN-1-ONE;2-HYDROXY-3,4,5-TRIMETHYL-2-CYCLOPENTEN-1-ONE; 2-HYDROXY-3,5,5-TRIMETHYL-2-CYCLOHEXENONE; 2-HYDROXYACETOPHENONE; 2-ISOPROPYL-5-CAPROLACTONE; 2-ISOPROPYL-DIOXOLAN-4-ONE; 2-METHOXY-3,4,5-TRIMETHYL-2-CYCLOPENTEN-1-ONE; 2-METHYL-2-OCTENAL; 2-METHYL-3-(P-METHYLPHENYL)PROPANAL; 2-METHYL-6-PHENYL-OCT-2-ENE-6-ONE; 2-METHYLCYCLOHEXANONE; 2-METHYLHEPTAN-3-ONE; 2-METHYLHEXAN-3-ONE; 2-METHYLNONANAL; 2-METHYL-SPIRO(5,5)-UNDECAN-1-ONE; 2-OCTEN-4-ONE; 2-PHENYL-3-(2-FURYL)PROP-2-ENAL; 2-PHENYL-4-PENTENAL; 2-TETRADECENAL; 2-TRANS-4^{:}CIS-7-CIS-TRIDECATRIENAL; 3-((2-METHYL-3-FURYL)THIO)-4-HEPTANONE; 3(2)-HYDROXY-4-METHYL-HEXAN-2(3)-ONE; 3-(3-METHOXY-PHENYL)-PROPAN-2-ONE; 3-(5-METHYL-2-FURYL)BUTANAL; 3(Z),6(Z),9(Z)-DODECATRIENAL; 3(Z),6(Z)-DODECADIENAL; 3,3-DIETHOXY-2-BUTANONE; 3,3-DIMETHYLCYCLOHEXANONE; 3,4,5,6-TETRAHYDRO PSEUDO IONONE; 3,4-DIMETHYL-BUTYROLACTONE; 3,5-DIMETHYLA-THIA-HEPTAN-2,6-DIONE; 3,6,DIMETHYL-5,6-DIHYDRO-2(4H)BENZOFURANONE; 3,6-DIMETHYL-2-HYDROXY-2-CYCLOHEXEN-1-ONE; 3,7-DIMETHYL-6-OCTEN-2-ONE: 3A,4,5,7A-TETRAHYDRO-3,6-DIMETHYL-2(3H)BENZOFURANONE; 3-ACETYLOXY-2-OCTANONE; 3-ACETYLOXY-2-PENTANONE; 3-ACETYLSULFANYL-2-ETHYLHEXANAL; 3-DECANONE; 3-ETHOXY HEXANAL; 3-ETHYL-2-HYDROXY-4-METHYLCYCLOPENT-2-EN-1-ONE; 3-ETHYL-4,5,5-TRIMETHYL-2-(5H)-FURANONE; 3-HEPTYLDIHYDRO-5-METHYL-2(3H)-FURANONE; 3-HEXANONE; 3-HYDROXY-2-PENTANONE; 3-HYDROXY-4,5,5-TRIMETHYL-2-(5H)-FURANONE; 3-HYDROXY-4-METHYL-5-PENTY6-2(5H)-FURANONE; 3-HYDROXY-4-METHYL-ALPHA-PYRONE; 2-ACETYL-4,5-DIMETHYL-THIAZOLE; 2-METHYL-3-ACETYLPYRIDINE; 1-ETHYL-2-ACETYL-PYRROLE; 1-METHYL-2-ACETYLPYRROLE; 2,4-DIMETHYL-5-ACETYL THIAZOLE; 3-ACETYL-2,5-DIMETHYLFURAN; 1,4-DIMETHYL-4-ACETYL-1-CYCLOHEXENE; 4-ACETYL-6-T-BUTYL-1,1-DIMETHYLINDAN; 4-ACETYL-2-METHYLPYRIMIDINE; ACETYL-2-PYRAZINE; ACETYL-2-FURAN; ACETYL-2-PYRROLE; ACETYL-2-PYRIDINE; ACETYL-2-DIMETHYL-PYRAZINE; ACETYL-2-THIAZOLE; ACETYL-3-PYRIDIN; ACETYL-3-DIMETHYL-2.5-THIOPHENE; ACETYL-2 METHYL-5 FURAN; ACETYL-2-THIAZOLINE-2; ACETYL-2 METHYL-3 PYRAZINE; PROPIONYL PYRAZINE; PROPIONYL-2-METHYL-3- FURANE; 2-PROPIONYLTHIAZOLE; 2-PROPIONYLPYRROLE; 3-HYDROXY-4-PHENYL-2-BUTANONE; 3-HYDROXY-5-HEXYL-4-METHYL-2(5H)-FURANONE; 3-HYDROXY-6-HYDROXYMETHYL-PYRAN-2-ONE; 3-HYDROXYMETHYL-2-OCTANONE; 3-ISOAMYL-2,4-PENTANDIONE; 3-MERCAPTO-2-METHYLPENTANAL; 3-MERCAPTO-2-OCTANONE; 3-MERCAPTO-2-PENTANONE; 3-MERCAPTO-4-PHENYL-2-BUTANONE; 3-MERCAPTO-5-METHYL-HEXAN-2-ONE; 3-METHYL NON-2(E)-EN-4ONE; 3-METHYL-1-CYCLOPENATADEGANONE; 3-METHYL-2-CYCLOHEXEN-1-ONE; 3-METHYL-2-CYCLOPENTEN-1-ONE; 3-METHYL-2-HEPTEN-4,5-DIONE; 3-METHYL-3-(3-METHYL-BUT-2-ENYL)-DIHYDRO-FURAN-2-ONE; 3-METHYL-4-PHENYL-3-BUTENE-2-ONE; 3-METHYL-5-PROPYL-2-CYCLOHEXEN-1=ONE; 3-METHYLCYCLOHEXANONE; 3-METHYLENE-2-OCTANONE_{;} 3-METHYL-GAMMA-DECALACTONE; 3-METHYLPENTANAL, 3-METHYLTHIOHEXANAL; 3-NONANONE: 3-NONEN-2-ONE; 3-PENTANONE; 3-PENTEN-2-ONE; 3-PENTYL-4,5,5-TRIMETHYL-2-(5H)-FURANONE; 3-PHENYL-4-PENTENAL; 3-PROPYL-2-CYCLOPENTEN-1-ONE; 3-PROPYLIDEN-HEPTAN-2-ONE; 4-(2,6,6-TRIMETHYL-2-CYCLOHEXEN-1-YL)BUTAN-2-ONE; 4-(2,6,6-TRIMETHYL-CYCLOHEXA-1 ,3-DIENYL)-BUT-3-EN-2-ONE; 4-(FURAN-2-YL)-BUTAN-2-ONE; 4-(METHYLTHIO)BUTANAL; 4-(P-TOLYL)-2-BUTANONE; 4,4-DIMETHOXY-3-HEXANONE; 4,4-DIMETHYL-1,3-OXATHIANE-2-ONE; 4,8-DIMETHYL-3,7-NONADIEN-2-ONE; 4-[(2-FURANMETHYL)THIO]-2-PENTANONE; 4-ACETOXY-HEX-4-EN-3-ONE; 4-EPOXY-1,1,3-TRIMETHYLCYCLOHEX-5-EN-2-ONE; 4-ETHYL-1,3-OXATHIAN-2-ONE; 4-ETHYL-5-METHYL-1,3-OXATHIAN-2-ONE; 4-HEPTYL-3-METHYLBUTYROLACTONE; 4-HYDROXY-2,2,5-TRIMETHYL-3[2H]-FURANONE; 4-HYDROXY-3,5,5-TRIMETHYL-CYCLOHEX-5,5-EN-1-ONE; 4-HYDROXY-4-METHYL-5-HEXENOIC-ACID LACTONE; 4-HYDROXY-4-METHYL-CIS-7-DECANOICACID-4-LACTONE; 4-MERCAPTO-4-METHYL-PENTAN-2-ONE; 4-METHYL-2-PENTENAL; 4-METHYL-3-PENTEN-2-ONE; 4-METHYL-4-FURFURYLTHIO-2-PENTANONE; 4-METHYL-5-PENTYL-DIHYDRO-2(3H)-FURANONE; 4-METHYLCYCLOHEXANONE; 4-METHYLTHIO-2-PENTANONE; 4-OXO-BETA DAMASCONE; 4-PHENYL-3,5-DITHIA-2-HEXANONE; 5 ALPHA-ANDROST-16-EN-3-ONE; 5,5-DIMETHYL-2(5H)-FURANONE; 5,5-DIMETHYL-2-METHOXY-CYCLOHEX-2-ENONE; 5-ETHYL-2-HYDROXY-3,4-DIMETHYL-2-CYCLOPENTEN-1-ONE; 5-ETHYL-2-METHOXY-3,4-DIMETHYL-2-CYCLOPENTEN-1-ONE; 5-ETHYLDIHYDRO-5-METHYL-2(3H)-FURANONE; 5-HYDROXY-4-OCTANONE; 5-HYDROXY-8-UNDECENOIC ACID D-LACTONE; 5-METHYL-2-PROPYL-[1,3]-DIOXOLAN-4-ONE; 5-METHYL-3-(3-METHYL-BUT-2-ENYL)-DIHYDRO-FURAN-2-ONE; 5-METHYL-3-HEXEN-2-ONE; 5-METHYL-4-HEXENE-2,3-DIONE; 5-METHYL-5-HEXEN-2-ONE; 5-PENTYL-ALPHA-PYRONE; 5-P-TOLUYL-2(3H)-FURANONE; 6-(1-PENTENYL)-2H-PYRAN-2-ONE; 6-HEPT-1-ENYL-5,6-DIHYDRO-PYRAN-2-ONE; 6-HYDROXY-3,7-DIMETHYLOCTANOIC ACID LACTONE; 6-METHYL-3-HEPTEN-2-ONE; 6-METHYL-NON-5-EN-4-ONE; 6-METHYL-OCTANAL; 6-PROPYL-[1,3]OXATHIAN-2-ONE; 6-UNDECANONE; 7-METHYL-2,3,4.4A,5,6-HEXAHYDRO-2-NAPHTHALENONE, 7-METHYL-6-OCTEN-2-ONE; 7-OCTENAL; 8-(2,2-DIMETHYLCYCLOPROPYL)-OCTA-3,5-DIEN-2-ONE; 8A-METHYL-3,4,4A,5,8,8A-HEXAHYDRO-1(2H)-NAPHTHALENONE; 8-METHYL NONANAL; 8-METHYL-TRIDEC-7-EN-6-ONE; 8-NONEN-2-ONE; 8-NONENAL; 9-DECENAL; ACETONE; ACETOPHENONE; A-P-DIMETHYLANISALACETONE; AR-TURMERONE; BENZOPHENONE; BENZOYLACETONE; BENZYL DIPROPYL KETONE; BENZYL-ACETONE; BENZYLIDENE ACETONE; BETA-IRONE; BICYCLO-IRONE; BICYCLONE; BICYGLONONALACTONE; BUTYRO-1,4-LACTONE; CARYOPHYLLEN; 12-AL; CIS,CIS-4,7-DECADIEN-1-AL; CIS-2-NONENAL; CIS-3-HEXENAL; CIS-3-NONENAL; CIS-4-HEPTEN-2-ONE; CIS-4-HEXENAL; CIS-4-NONENAL; CIS-5-NONENAt; CIS-5-OCTENAL; CIS-7-NONENAL; CITRONELLENE LACTONE; CLONAL; COGNAC LACTONE; CYCLOBUTONE; CYCLOHEPTADECA-9-EN-1-ONE; CYCLOHEXANONE;. CYCLOIONONE; CYCLOPENTANONE; CYCLOHEPTADECANONE; D-1-(2,6,6-TRIMETHYL-3-CYCLOHEXEN-1-YL)-2-BUTEN-1-ONE; DAMASCENONE; DECANAL; DECENYLCYCLOPENTANONE; DIBENZYL KETONE; DIBUTYL-4,4 GAMMA BUTYROLACTONE; DIHYDRO FILBERTONE; DIHYDRO-5-METHYL-5-PROPYL-2(3H)-FURANONE; DIHYDROCARVONE; DIHYDRO·DIHYDRO-GAMMA-IONONE; DIHYDROJASMONE LACTONE; DIHYDROLAVENDELLACTONE; DIMETHYL HYDROQUINONE; DIMETHYL METHOXY FURANONE; DIMETHYL-2,4 ACETOPHENONE; DIMETHYL-2,6 OCTANAL; DIMETHYL-3,6-BENZO-2(3H)-FURANONE: DODEC-2-EN-1,5-LACTONE; DODEC-2-EN-4-ONE; DODECANAL; E-DECALACTONE; E-DODECALACTONE; ETHYL-2-HEPTEN-2-AL; ETHYL-3 CYCLOPENTANDIONE; FARNESYL ACETONE; FURFURYLIDENE ACETONE; GAMMA DAMASCONE; GAMMA IONONE; GERANYL ACETONE; GINGERONE HEPT-3-EN-2-ONE; HEPTANAL; HEXANAL; HEXEN 5 ONE; HEXENYL METHYL KETONE; HEXYLIDENE CYCLOPENTANONE; HOMOCORYLONE; HYDROBICYCLONE; HYDROXY-3(2)-HEPTAN-2(3)-ONE; HYDROXY-5-DECADIENOIC ACID-2,4 LACTONE; ISO JASMONE; ISOLONGIFOLENE-KETONE; ISOPHORONE; JASMIN LACTONE; LAVENDELLACTONE; MASSOIA LACTONE; MELONAL, DIMETHYL HEPTENAL; MENTHONE; METHIONAL; METHOXY-P-PHENYL-PENTEN-3-ONE; ETHONE; METHYL 2 OCTANAL; METHYL 2-PENTENE-1-AL; METHYL THIO- METHYLTHIOMETHYL -2- PENTENAL; METHYL-2-UNDECANAL; METHYL-2-BUTANAL; METHYL-2-PENT-2-ENAL; METHYL-2-TETRAHYDRO-FURANONE; METHYL-4-HYDROXY-3/2H/-FURANONE; METHYL-5-HEPT-2-EN-4-ONE; METHYL-6-HEPTA-3,5-DIENONE; METHYLTHIO-3-BUTANAL; METHYLTHIO-4-BUTAN-2-ONE; MINTLACTONE; MYRTENAL; NEOFOLIONE; NEOHESPERIDINE DIHYDROCHALCONE; NONANAL; NOOTKATONE; OCTAN-2-ONE; OCTANAL; PENNYROYAL OIL 84% PULEGONE; PENTADECANONE; PENTANAL; PENTYL 2-FURYL KETONE; PHENYL-2-BUTEN-2-AL, PHENYL-3-PROPANAL; PIPERITENONE; PIPERONYL ACETONE; P-MENTH-1-ENAL; P-MENTH-1-ENE-9-AL; P-MENTHAN-2-ONE; PRENAL; PROPANAL; PROPYL-ISO PARA ACETOPHENONE; RAC-3-HYDROXY-5-METHYL-2-(5H)-FURANONE; RASPBERRY KETONE; SAFRANAL; SAGE OIL 27% THUJONE; SOLANONE; SPIRO-(6,5)-DODECAN-1-ONE; TETRAHYDROIRONE; TETRAHYDRONOOTKATONE; TRANS-4-HEXENAL; TRANS-5-NONENAL; TRANS-6-DECEN-1-AL; TRANS-6-NONENAL; TRANS-7-ME7HYL-3-OCTEN-2-ONE; TRANS-7-NONENAL; TRIDECAN-2-ONE; TRIMETHYL-3,5,5-HEXANAL; TRITHIOACETONE; UNDECANAL; VANILLYLIDENE ACETONE; and VERBENONE:

Useful flavor precursors according to formula I are, without limitation, the following groups of compounds below with a particular selection of R1, R2, R3 and a R2-C-R3 ring residue (N and m are as defined in FI. either n=1 and m=0 or n=0 and m=1).

Where branched compounds are indicated, these may be singly, doubly or multiply branched (having one, two or more alkyl groups).

### Without limitation, useful groups of compounds are as follows:

**R1 and R2 according to formula I:**
Compounds of formula I with R3 selected as indicated in the table below.

**R1 and R3 according to formula I:**
Compounds of formula I with R2 selected from H, methyl, ethyl, butyl, 2-butenyl, and vinyl.

**R2 and R3 according to formula I:**
Compounds of formula I with R1 selected from C7 to C17 alkyl, C7 to C17 alkenyl, C7 to C17 monoalkenyl and C7 to C17 alkadienyl.

Compounds of formula I with R1 selected from straight-chain C7 to C17 alkyl, straight-chain C7 to C17 alkenyl, straight chain C7 to C17 monoalkenyl, and straight-chain C7 to C17 alkadienyl.

Compounds of formula I with R1= alkenyl selected from C7, C8, C9, C10, C11, C13, C15, and C17 alkenyl, C17-8en alkenyl (oleic acid residue), C17-8,11 alka-dienyl (linoleic acid residue), and C17-8,11,14-trienyl (linolenic acid residue).

Compounds of formula I with R1= straight-chain alkenyl selected from C7, C8, C9, C10, C11, C13, C15, and straight-chain C17 alkenyl, straight-chain C17-8en alkenyl (oleic acid residue), straight-chain C17-8,11 alka-dienyl (linoleic acid residue), and C17-8,11,14-trienyl (linolenic acid residue).

Compounds of formula I with R1= heptyl, nonyl, undecyl, tridecyl, pentadecyl, heptadecyl.

**R1 according to formula I:**
Compounds of formula I with R2 selected from H, methyl, ethyl, 2-butenyl, or vinyl, and R3 as indicated in the table below.

Compounds of formula I with R2=H, and R3 selected as indicated in the table below.

Compounds of formula I with R2=methyl, and R3 selected as indicated in the table below.

R2 according to formula **I:**
Compounds of formula I with R1 selected from C7 to C17 alkyl, C7 to C17 alkenyl, C7 to C17 monoalkenyl and C7 to C17 alkadienyl, and R3 selected as indicated in the table below.

Compounds of formula I with R1 selected from heptyl, nonyl, undecyl, tridecyl, pentadecyl, heptadecyl, and R3 selected as indicated in the table below.

R3 according to formula I:
Compounds of formula I with R1 selected from heptyl, nonyl, undecyl, tridecyl, pentadecyl, and heptadecyl, and R2 selected from H, methyl, ethyl, 2-butenyl, and vinyl.

Compounds **with R1,** R2 and R3 (table) as **defined** below:
Compounds of formula I with R1 selected from C7 to C17 alkyl, C7 to C17 alkenyl, C7 to C17 monoalkenyl and C7 to C17 alkadienyl, and R2 selected from H, methyl, ethyl, butyl, 2-butenyl, or vinyl, and R3 selected as indicated in the table below.

Compounds of formula I with R1= heptyl, nonyl, undecyl, tridecyl, pentadecyl, and heptadecyl, wherein R2 is H, and R3 is selected as indicated in the table below.

Compounds of formula **I** with R1 selected from heptyl, nonyl, undecyl, tridecyl, pentadecyl, and heptadecyl, R2=methyl, and R3 selected as indicated in the table below.

**Table 1 Examples groups of R3 residues of formula I and the groups of compounds defined herein-above.**

| **R3 =** |
|---|
| C3 to C15 branched alkyl comprising one or two alkyl groups, wherein the alkyl residue optionally contains one or more further alkyl residues |
| C1 to C15 straight-chain alkyl comprising one or two substituents selected from O, OH, N, NH, SH, and SR4, wherein R4 is an alkyl residue selected from methyl, ethyl, propyl, isopropyl, butyl, isobutyl, pentyl and isopentyl and the total number of carbon atoms in R3 and R4 is up to 15, |
| C3 to C15 branched alkyl comprising one or two alkyl groups and substituted with one or two residues independently selected from 0, OH, N, NH, SH, and SR4, wherein R4 is an alkyl group selected from methyl, ethyl, propyl, isopropyl, butyl, isobutyl, pentyl and isopentyl, and the total number of carbon atoms in R3 and R4 is up to 15 |
| C1 to C15 straight-chain alkyl, comprising one or two atoms independently selected from O,S and N within the alkyl chain |
| C3 to C15 branched alkyl comprising one or two alkyl groups and comprising one or two atoms independently selected from O,S and N within the alkyl chain |
| C2 to C15 straight-chain alkenyl, |
| C3 to C15 straight-chain alkadienyl |
| C3 to C15 branched alkenyl, substituted with one or two alkyl groups, C5 to C15 branched alkadienyl, substituted with one or two alkyl groups |
| C2 to C15 straight-chain alkenyl comprising one or two substituents selected from 0, OH, N, NH, SH, SR4 wherein R4 is an alkenyl residue selected from ethenyl, propenyl, butenyl, and pentenyl, and the total number of carbon atoms in R3 and R4 is up to 15, |
| C2 to C15 branched alkenyl comprising one or two alkyl groups comprising one or two substituents selected from O, OH, N, NH, SH, and SR4, wherein R4 is a alkenyl residue selected from ethenyl, propenyl, butenyl, and pentenyl, and the total number of carbon atoms in R3 and R4 is up to 15 |
| C2 to C15 straight-chain alkenyl, comprising one or two atoms independently selected from O,S and N within the alkyl chain |
| C3 to C15 branched alkenyl comprising one or two alkyl groups and comprising one or two atoms independently selected from O,S and N within the alkyl chain |
| C4 to C15 branched alkadienyl, comprising one or two alkyl groups and comprising one or two atoms independently selected from O,S and N within the alkyl chain |
| Straight-chain C1 to C15 alkyl, straight-chain C2 to C 15 alkenyl, branched C1 to C15 alkyl, branched C3 to C 15 alkenyl |
| C2 to C5 alkyl substituted in position 1 with a substituent selected from O and OH |
| C2 to C13 alkyl |
| C2 to C10 alkyl |
| C3 to C13 alkenyl |
| C2 to C10 alkenyl |
| compounds as defined in Fl wherein R3 has a maximum of up to 13 carbon atoms |
| straight-chain C1 to C13 alkyl |
| straight-chain C1 to C10 alkyl |
| straight-chain C2 to C13 alkenyl |
| straight-chain C2 to C10 alkenyl |
| branched C1 to C13 alkyl comprising one or two branching alkyl groups |
| branched C1 to C10 alkyl comprising one or two branching alkyl groups |
| singly or doubly branched C4 to C13 alkenyl comprising one or two branching alkyl groups |
| singly or doubly branched C4 to C10 alkenyl comprising one or two branching alkyl groups |
| C3 to C13 alkenyl selected from 1-alkenyl, a 3-alkenyl, and a 1,3-alkadienyl |
| C3 to C10 alkenyl selected from 1-alkenyl, a 3-alkenyl, and a 1,3-alkadienyl |
| C3 to C13 alkenyl selected from propenyl, butenyl, pentenyl, hexenyl, heptenyl, and octenyl |
| C3 to C10 alkenyl selected from propenyl, butenyl, pentenyl, hexenyl, heptenyl, and octenyl |
| singly, doubly, or multiply branched C3 to C13 alkenyl |
| singly, doubly or multiply branched C3 to C10 alkenyl |
| 4-methylpent-3-enyl or 4-methylpent-1,3-dienyl |
| methylpentenyl and methylpentadienyl |
| straight-chain C3 to C13 alkenyl selected from propenyl, butenyl, pentenyl, hexenyl, heptenyl, and octenyl |
| straight-chain C3 to C10 alkenyl selected from propenyl, butenyl, pentenyl, hexenyl, heptenyl, and octenyl |
| prop-1-enyl, but-1-enyl, pent-1-enyl, hex-1-enyl, hept-1-enyl, and oct-1-enyl |
| pent-1,3-dienyl, hex-1,3-dienyl, hept-1,3-dienyl, and oct-1,3-dienyl |
| 1-1-hydroxyethyl, 1-oxoethyl,1-oxopropyl, 1-oxobutyl, 1-oxopentyl, 1-oxohexyl |
| C2 to C7 alkyl, C3 alkenyl |
| C3 alkenyl prop-1-enyl |
| prop-1-enyl |
| C3 alkenyl |
| straight-chain C2 to C7 alkyl |
| straight-chain C3 to C 15 alkenyl |
| straight-chain C3 to C 15 monoalkenyl |
| straight-chain 1-monoalkenyl |
| straight-chain C5 to C 13 alkadienyl |
| straight-chain 1,3-alkadienyl |
| straight-chain C8 to C 12 alkatrienyl |
| singly or doubly branched C3 to C 12 alkyl comprising one or two branching alkyl groups |
| 1-methyl alkyl and 3-methylalkyl |
| singly, doubly or multiply branched alkyl selected from 1-methyl alkyl, 3-methylalkyl, 1,3-methylalkyl, 1-ethylalkyl, 1,1-diethylalkyl, and 1-isopropylalkyl |
| 3,6-methylalkyl |
| singly, doubly or multiply branched C3 to C 13 monoalkenyl, singly, doubly or multiply branched C3 to C 13 alkadienyl and branched C3 to C 13 alkatrienyls |
| thioalkyl including thiomethyl, thioethyl, thiopropyl, thiobutyl, thiopentyl, thiohexyl |
| methylthioalkyl including methylthiomethyl, methylthioethyl, methylthiopropyl, methylthiobutyl, methylthiopentyl, methylthiohexyl |
| thioalkyl, thiomethyl, thioethyl, thiopropyl, thiobutyl, thiopentyl, thiomethylethyl, thiomethylpropyl, thiomethylbutyl, alkyl(methylthio)alkyl, methyl(methylthio)alkyl, methyl(methylthio)ethyl, methyl(methylthio)propyl, methyl(methylthio)butyl |
| branched alkylthioalkyl including methyl-methylthioalkyl and dimethylthioalkyl (including methyl-methylthiopropyl, methyl-methylthiobutyl, methyl-methylthiopentyl, thiohexyl) |
| methylthiomonoalkenyl including methylthioethenyl, methylthiopropenyl, methylthiobutenyl, methylthiopentenyl, thiohexenyl |
| thiaalkyl including thiamethyl, thiaethyl, thiapropyl, thiabutyl, thiapentyl, thiahexyl |
| methylthiaalkyl including methylthiamethyl, methylthiaethyl, methylthiapropyl, methylthiabutyl, methylthiapentyl, methyl-thiahexyl |
| singly, doubly or multiply branched alkylthiaalkyl including methyl-methylthiaalkyl and dimethylthiaalkyl (including methylthiapropyl, methylthiabutyl, methylthiapentyl, thiahexyl) (for example: 1,1 dimethyl 2,3dithiabutyl) |
| methylthiamonoalkenyl including methylthiaethenyl, methylthiapropenyl, methylthiabutenyl, methylthiapentenyl, thiahexenyl |
| dithiaalkyl including dithiamethyl, dithiaethyl, dithiapropyl, dithiabutyl, dithiapentyl, dithiahexyl |
| methyldithiaalkyl including methyldithiamethyl, methyldithiaethyl, methyldithiapropyl, methyldithiabutyl, methyldithiapentyl, methyldithiahexyl |
| singly, doubly or multiply branched alkylthiaalkyl including methylmethylthiaalkyl and dimethylthiaalkyl (including methylthiapropyl, methylthiabutyl, methylthiapentyl, thiahexyl) (for example: 1,1 dimethyl 2,3dithiabutyl) |
| methylthioalkenyl including methylthiomethenyl, methylthioethenyl, methylthiopropenyl, methylthiobutenyl, methylthiopentenyl, methylthiohexenyl |
| singly, doubly or multiply branched alkylthioalkenyl including methyl-methylthioalkenyl and dimethylthioalkenyl |
| (including methylthiopropenyl, methylthiobutenyl, methylthiopentenyl, thiohexenyl) |
| methylthiomonoalkenyl including methylthioethenyl, methylthiopropenyl, methylthiobutenyl, methylthiopentenyl, thiohexenyl |
| thiaalkenyl including thiaethenyl, thiapropenyl, thiabutenyl, thiapentenyl, thiahexenyl |
| methylthiaalkenyl including methylthiaethenyl, methylthiapropenyl, methylthiabutenyl, methylthiapentenyl, thiahexenyl |
| shingly, doubly or multiply branched alkylthiaalkenyl including methyl-methylthiaalkenyl and dimethylthiaalkenyl (including methylthiapropenyl, methylthiabutenyl, methylthiapentenyl, thiahexenyl) (for example: 1,1-dimethyl 2,3-dithiabutyl) |
| methylthiamonoalkenyl including methylthiapropenyl, methylthiabutenyl, methylthiapentenyl, thiahexenyl |
| dithiaalkenyl including dithiaethenyl, dithiapropenyl, dithiabutenyl, dithiapentenyl, dithiahexenyl |
| methyldithiaalkenyl including methyldithiaethenyl, methyldithiapropenyl, methyldithiabutenyl, methyldithiapentenyl, methyldithiahexenyl |
| branched alkylthiaalkenyl including methyl-methylthiaalkenyl and dimethylthiaalkenyl (including methylthiapropenyl, methylthiabutenyl, methylthiapentenyl, thiahexenyl) (for example: 1,1 dimethyl 2,3dithiabutenyl) |
| methylthiamonoalkenyl including methylthiaethenyl, methylthiapropenyl, methylthiabutenyl, methylthiapentenyl, thiahexenyl |
| phenyl, alkylphenyl, and substituted phenyl with the substituent selected from OH, and alkoxy |
| methylphenyl, ethylphenyl, propylphenyl, isopropylphenyl, butylphenyl, isobutylphenyl, and pentylphenyl |
| substituted phenyl selected from hydroxyphenyl, methoxyphenyl, and ethoxyphenyl |
| doubly substituted phenyl selected from hydroxy-alkoxyphenyl, hydroxymethoxyphenyl, alkoxy-alkylphenyl, methoxy-alkylphenyl, hydroxy-alkylphenyl, and hydroxymethoxyphenyl |
| furanyl, alkylfuranyl, methylfuranyl, ethylfuranly, propylfuranyl, isopropylfuranyl, butylfuranyl, isobutylfuranyl |
| thiophenyl, alkylthiophenyl, methylthiophenyl, ethylthiophenyl, dialkylthiophenyl, dimethylthiophenyl, ethylmethylthiophenyl |
| phenylalkyl and phenylalkenyl |
| phenylalkyl selected from benzyl, phenylethyl, phenylpropyl, phenylbutyl, phenylpentyl, phenylhexyl, phenylheptyl |
| phenylalkenyl selected from phenylethenyl, phenylpropenyl, phenylbutenyl, phenylpentenyl, phenylhexenyl, phenylheptenyl |
| alkylthioalkyl, alkylthioalkenyl, alkyldisulfanylalkyl, alkyldisulfanylalkenyl, methylthioalkyl, methyl-methylthioalkyl, methylthioalkenyl, methyl-methylthioalkenyl, methylthioalkyl, methyl-methylthioalkyl, methylthioalkenyl, methyl-methylthioalkenyl, methyldisulfanylalkyl, methyl-methyldisulfanylalkyl, methyldisulfanylalkenyl, methyl-methyldisulfanylalkenyl |
| residue having a maximum number of C atoms of 12 and selected from singly branched hydroxyalkyl, doubly branched hydroxyalkyl, multiply branched hydroxyalkyl, hydroxydialkylalkyl, hydroxydimethylalkyl, hydroxydimethylbutyl, hydroxydimethylpentyl, hydroxydimethylhexyl, hydroxydimethylheptyl, hydroxydimethyloctyl, hydroxydimethylnonyl |
| |
| furanyl-substituted straight-chain or branched alkyl including methyl, ethyl, propyl, butyl, and pentyl, optionally substituted with one or more of OH, O, and SH; straight-chain or branched furanyl-substituted alkylthioalkyl, furanyl-substituted methylthioalkyl |
| straight-chain or branched substituted alkyl substituted with one or more of O, and OH; a straight-chain or branched substituted alkyl substituted with one or more of 0 and OH wherein the alkyl is selected from ethyl, propyl, butyl, pentyl, hexyl, heptyl, and octyl; a straight-chain or branched (alkoxycarbonyl)alkyl; a straight-chain or branched (alkoxycarbonyl)alkyl wherein the alkyl is selected from ethyl, propyl, butyl, pentyl, hexyl, heptyl, and octyl |
| alkylphenyl, phenylalkyl, C9 arylalkyl, C10 arylalkyl, phenylalkenyl, C9 arylalkenyl, C10 arylalkenyl, methoxyphenyl, methoxyphenylalkyl, methoxyphenylalkenyl, hydroxyphenylalkyl, hydroxyphenylalkenyl, hydroxyphenylalkyl |
| a C1 to C6 alkyl or alkenyl substituted with a ring residue selected from cyclohexyl, cyclohexenyl, cyclopentyl, cyclopentenyl, oxacyclohexyl, oxacyclohexenyl, oxacyclopentyl, oxacyclopentenyl, thiacyclohexyl, thiacyclohexenyl, thiacyclopentyl, thiacyclopentenyl, azacyclohexyl, azacyclohexenyl, azacyclopentyl, azacyclopentenyl, dioxacyclohexyl, dioxacyclohexenyl, dioxacyclopentyl, dioxacyclopentenyl, dithiacyclohexyl, dithiacyclohexenyl, dithiacyclopentyl, dithiacyclopentenyl, diazacyclohexyl, diazacyclohexenyl, diazacyclopentyl, diazacyclopentenyl, oxathiacyclohexyl, oxathiacyclohexenyl, oxathiacyclopentyl, oxathiacyclopentenyl, oxathiacyclohexyl, oxathiacyclohexenyl, oxathiacyclopentyl, oxathiacyclopentenyl, oxathiacyclohexyl, oxathiacyclohexenyl, oxathiacyclopentyl, oxathiacyclopentenyl, azaoxacyclohexyl, azaoxacyclohexenyl, azaoxacyclopentyl, azaoxacyclopentenyl, azaoxacyclohexyl, azaoxacyclohexenyl, azaoxacyclopentyl, azaoxacyclopentenyl, azaoxacyclohexyl, azaoxacyclohexenyl, azaoxacyclopentyl, azaoxacyclopentenyl, azathiacyclohexyl, azathiacyclohexenyl, azathia cyclopentyl, azathia cyclopentenyl, azathia cyclohexyl, azathia cyclohexenyl, azathia cyclopentyl, azathia cyclopentenyl, azathiacyclohexyl, azathiacyclohexenyl, azathiacyclopentyl, azathiacyclopentenyl, wherein the ring residue may optionally be further substituted with one or more substituents selected independently from methyl, ethyl, propyl, isopropyl, and a ketogroup. |
| a ring residue selected from |
| cyclohexyl, cyclohexenyl, cyclopentyl, cyclopentenyl, oxacyclohexyl, oxacyclohexenyl, oxacyclopentyl, oxacyclopentenyl, thiacyclohexyl, thiacyclohexenyl, thiacyclopentyl, thiacyclopentenyl, azacyclohexyl, azacyclohexenyl, azacyclopentyl, azacyclopentenyl, dioxacyclohexyl, dioxacyclohexenyl, dioxacyclopentyl, dioxacyclopentenyl, dithiacyclohexyl, dithiacyclohexenyl, dithiacyclopentyl, dithiacyclopentenyl, diazacyclohexyl, diazacyclohexenyl, diazacyclopentyl, diazacyclopentenyl, oxathiacyclohexyl, oxathiacyclohexenyl, oxathiacyclopentyl, oxathiacyclopentenyl, oxathiacyclohexyl, oxathiacyclohexenyl, oxathiacyclopentyl, oxathiacyclopentenyl, oxathiacyclohexyl, oxathiacyclohexenyl, oxathiacyclopentyl, oxathiacyclopentenyl, azaoxacyclohexyl, azaoxacyclohexenyl, azaoxacyclopentyl, azaoxacyclopentenyl, azaoxacyclohexyl, azaoxacyclohexenyl, azaoxacyclopentyl, azaoxacyclopentenyl, azaoxacyclohexyl, azaoxacyclohexenyl, azaoxacyclopentyl, azaoxacyclopentenyl, azathiacyclohexyl, azathiacyclohexenyl, azathia cyclopentyl, azathia cyclopentenyl, azathia cyclohexyl, azathia cyclohexenyl, azathia cyclopentyl, azathia cyclopentenyl, azathiacyclohexyl, azathiacyclohexenyl, azathiacyclopentyl, azathiacyclopentenyl, wherein the ring residue may optionally be further substituted with one or more substituents selected independently from methyl, ethyl, propyl, isopropyl, and a ketogroup. |
| a ring residue selected from cyclohexyl, cyclohexenyl, cyclopentyl, cyclopentenyl wherein the ring is substituted with at least one ketogroup and further substituted with an alkyl selected from methyl and ethyl. |
| a ring residue selected from thiacyclohexyl, thiacyclohexenyl, thiacyclopentyl, thiacyclopentenyl wherein the ring is substituted with a ketogroup, and wherein the ring may optionally be further substituted with one or two alkyl groups selected from methyl, and ethyl |
| a ring residue selected from oxacyclohexyl, oxacyclohexenyl, oxacyclopentyl, oxacyclopentenyl wherein the ring is substituted with a ketogroup, and wherein the ring may optionally be further substituted with one or two alkyl groups selected from methyl, and ethyl |
| oxacyclopentan, substituted oxacyclopentyl |
| furanyl |
| thiacyclopentyl, substituted thiacyclopentyl |
| thiophenyl |
| azacyclopentyl, substituted azacyclopentyl |
| pyrrolyl, dihydropyrrolyl |
| azacyclopentyl, substituted diazacyclopentyl |
| azathiacyclopentenyl including a dihydrothiazolyl (for example: 2-thiadihydrothiazolyl) |
| thiazolyl, 2-thiazolyl |
| alkylthiazolyl including methylthiazolyl, ethylthiazolyl, and dimethylthiazolyl |
| azaoxacyclopentyl |
| oxazolyl, 2-oxazolyl, 5-oxazolyl |
| oxacyclohexyl |
| thiacyclohexyl |
| azacyclohexyl |
| pyridinyl, alkylpyridinyl, methylpyridinyl, dimethylpyridinyl, ethylpyridinyl, trimethylpyridinyl, tetramethylpyridinyl, ethylmethylpyridinyl, ethyldimethylpyridinyl |
| diazacyclohexyl |
| pyrazinyl, alkylpyrazinyl, methylpyrazinyl, dimethylpyrazinyl, trimethylpyrazinyl, ethylmethylpyrazinyl, ethyldimethylpyrazinyl, ethylpyrazinyl, diethylpyrazinyl, diethylmethylpyrazinyl |
| azathiacyclohexyl |
| azaoxacyclohexyl |

### Group of compounds with R1, R2 and R3 as defined below:

Compounds of formula I with R1 selected from heptyl, nonyl, undecyl, tridecyl, pentadecyl, and heptadecyl, R2=methyl, and R3 selected from a 1,1-hydroxyethyl, 1-oxoethyl, and 1-oxopropyl.

Compounds of formula I with R1 selected from straight-chain C7 to C17 alkyl, straight-chain C7 to C17 alkenyl, branched C7 to C17 alkyl, branched C7 to C17 alkenyl, R2= methyl, and R3 selected from straight-chain C1 to C15 alkyl, straight-chain C1 to C 15 alkenyl, branched C1 to C15 alkyl, and branched C1 to C 15 alkenyl.

### Groups of compounds with a R2-C-R3 ring residue:

The following compounds are compounds wherein the R2-C-R3 part of formula I forms a ring residue which is bound to the two ring oxygen atoms of formula I. For all residues, substituents and double bonds are counted starting from the C atom in position 1 (C¹, compare structural formula below) which is the C atom between R2 and R3. Double bonds may be in cis or trans position. To illustrate, the following structural formula shows a compound of formula I wherein R2-C-R3 is 2,4-dimethyl-4,5-dihydro-(2H)-furanone:

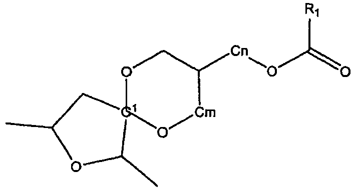

Compounds of formula I with R1 as defined herein-above and R2-C-R3 selected as indicated in the table below.

Compounds of formula I with R1 selected from C7 to C17 alkyl, C7 to C17 alkenyl, C7 to C17 monoalkenyl, C7 to C17 alkadienyl, straight-chain C7 to C17 alkyl, straight-chain C7 to C17 alkenyl, straight-chain C7 to C17 monoalkenyl, straight-chain C7 to C17 alkadienyl, branched C7 to C17 alkyl, branched C7 to C17 alkenyl, branched C7 to C17 monoalkenyl, branched C7 to C17 alkadienyl, and R2-C-R3 selected as indicated in the table below.

Compounds of formula I with R1 selected from C7 to C17 alkyl, C7 to C17 alkenyl, C7 to C17 monoalkenyl, C7 to C17 alkadienyl, and R2-C-R3 selected as indicated in the table below.

Compounds of formula I with R1 selected from C7, C9, C11, C13, C15, or C17 straight- chain alkyl and C7, C9, C11, C13, C15, or C17 branched alkyl

Compounds of formula I with R1 selected from heptyl, nonyl, undecyl, tridecyl, pentadecyl, and heptadecyl, and R2-C-R3 selected as indicated in the table below.

**Table 2: Groups of R2-C-R3 residues**

| **R2-C-R3 ring residue is selected from:** |
|---|
| oxacyclopentan optionally substituted with a residue selected from methyl, dimethyl, ethyl, hydroxy, and methoxy |
| oxacyclopentene optionally substituted with a residue selected from methyl, dimethyl, ethyl, hydroxy, and methoxy |
| hydroxycyclopentene, hydroxyalkylcyclopentene, hydroxymethylcyclopentene, hydroxydimethylcyclopentene, ethylhydroxycyclopentene, and ethylhydroxymethylyclopentene |
| thiacyclopentane, alkylthiacyclopentane, and alkyl-3-thia-cyclopentane |
| oxa-cyclopentene, 2-oxacyclopentene, 3-oxacyclopentene, alkyloxacylcopentene, alkyl-3-oxacyclopentene, methyl-3-oxa-cyclopentene, alkyl-3-oxacyclopentene, dimethyl-3-oxacyclopentene, ethyl-3-oxacyclopentene, ethylmethyl-3-oxacyclopentene, hydroxyalkyl-3-oxacyclopentene, hydroxymethyl-3-oxacyclopentene, hydroxydimethyl-3-oxacyclopentene, hydroxyethyl-3-oxacyclopentene, ethylhydroxymethyl-3-oxacyclopentene |
| oxacyclopentane, 3-oxacyclopentan, alkyl-3-oxacyclopentan, methyl-3-oxa-cyclopentan, dimethyl-3-oxacyclopentan, ethyl-3-oxacyclopentan, ethylmethyl alkyl-3-oxacyclopentan. |
| alkylcyclopenten, alkenylcyclopenten, alkylalkenylcyclopenten, methylcylopenten, dimethylcyclopenten, ethylmethylcyclopenten, propylmethylcyclopenten, butylmethylcyclopenten, butenylmethylcyclopenten, pentylmethylcyclopenten, pentenylmethylcyclopenten |
| alkylcarboxy-alkenyl-cyclopentan, alkylcarboxy-alkyl-cyclopentan, methylcarboxy-alkenyl-cyclopentan, methycarboxy-alkyl-cyclopentan, alkylcarboxy-pentenyl-cyclopentan, alkylcarboxy-pentyl-cyclopentan |
| methylcarboxy-alkenyl-cyclopentan, and methylcarboxy-alkyl-cyclopentan, and wherein the alkyl is selected from methyl, ethyl, propyl, butyl, pentyl, pentenyl, hexyl, heptyl, and octyl |
| oxo-alkylcyclohexen, oxo-methylcyclohexen, oxo-trimethylcyclohexen, oxo-ethylcyclohexen, oxo-ethylmethylcyclohexen, oxo-propylcyclohexen, oxo-propylmethylcyclohexen, oxo-isopropylcyclohexen, oxo-isopropylmethylcyclohexen, oxo-thiopropylcyclohexen, oxo-thiopropylmethylcyclohexen |
| alkylcyclohexen, methylcyclohexen, trimethylcyclohexen, ethylcyclohexen, ethylmethylcyclohexen, propylcyclohexen, propylmethylcyclohexen, isopropylcyclohexen, isopropylmethylcyclohexen, thiopropylcyclohexen, thiopropylmethylcyclohexen, |
| alkylcyclohexan, methylcyclohexan, trimethylcyclohexan, ethylcyclohexan, ethylmethylcyclohexan, propylcyclohexan, propylmethylcyclohexan, isopropylcyclohexan, isopropylmethylcyclohexan, thiopropylcyclobexan, thiopropylmethylcyclohexan |

### In the following, further groups of compounds of formula I comprising R2-C-R3 useful In the invention are given:

A compound according to formula I wherein the R2-C-R3 subsituted cyclopentene residue is selected from the group consisting of 2-hydroxy-3-methylcyclopent-2-ene, 2-hydroxy-4-methylcyclopent-2-ene, 3-ethyl-2-hydroxycyclopent-2-ene, 2-hydroxy-3,4-dimethylcyclopent-2-ene, 3-ethyl-2-hydroxycyclopent-2-ene, 4-ethyl-2-hydroxy-3-methylyclopent-2-ene. These compounds will release hydroxy-alkyl-cyclopentenones.

A compound according to formula I wherein the R2-C-R3 thiacycloalkane ring residue is 3-thiacyclopentane.

A compound according to formula I wherein the R2-C-R3 substituted oxacyclopentene ring residue is selected from 2-methoxy-4-oxa-3,5-dimethylcyclopent-2-ene, 2-hydroxy-3,5-dimethyl-4-oxacyclopent-2-ene, 5-ethyl-2-hydroxy-3-methyl-4-oxa-pent-2-ene, 2,5-dimethyl-3-oxa-cyclopent-4-ene.

A compound according to formula I wherein the R2-C-R3 substituted oxacyclopentane ring residue is selected from the group consisting of 2,4-dimethyl-3-oxa-cyclopentan, and 2-methyl-3-oxa-cyclopentan.

A compound according to formula I wherein the R2-C-R3 alkyl/alkenyl/alkylalkenyl substituted cyclopentene ring residue is selected from 2-((Z)-pent-2-enyl)-3-methylcyclopent-2-en, 2-(-pent-2-enyl)-3-methylcyclopent-2-en.

A compound according to formula I wherein the R2-C-R3 alkylcarboxyalky/alkylcarboxyalkenyl-cylopentan ring residue is selected from 3-(methoxycarbonylmethyl)-2-pentyl-cyclopentan, 3-(methoxycarbonylmethyl)-2(Z)-pent-2-enyl-cyclopentan, cis 3-(methoxycarbonylmethyl)-2(Z)-pent-2-enyl-cyclopentan, trans 3-(methoxycarbonylmethyl)-2(Z)-pent-2-enyl-cyclopentan, and 2R3S-cis-3-(methoxycarbonylmethyl)-2(Z)-pentyl-cyclopentan.

A compound according to formula I wherein the R2-C-R3 alkyl/thioalkyl substituted oxo-cyclohexan/hexene ring residue is selected from 4-oxo-3,5,5-trimethylcyclohex-2-en, 3,5,5-trimethylcyclohex-2-en, 4-isopropyl-cyclohex-2-en, 2-isopropyl-5-methylcyclohexan, 4-isopropylcyclohexan, 2-(2-thiopropan-2-yl)-5-methylcyclohexan.

A compound according to formula I wherein R2 is H and R3 is a methyl-cyclohexdienyl including but not limited to 2,6,6-trimethyl-cyclohex-1,3-dienyl. Alternatively, R2 may be selected from methyl, ethyl, propyl, butyl, butenyl, pentyl, hexyl, heptyl.

### The following groups of compounds are example groups of precursor compounds that will release aldehyde flavors:

A compound according to formula I wherein R2 is H and R3 is selected from phenyl, alkylphenyl, and substituted phenyl with the substituent selected from OH, and alkoxy.

A compound according to formula I wherein R2 is H and wherein R3 is an alkylphenyl selected from methylphenyl, ethylphenyl, propylphenyl, isopropylphenyl, butylphenyl, isobutylphenyl, and pentylphenyl.

A compound according to formula I wherein R2 is H and wherein R3 is a substituted phenyl selected from hydroxyphenyl, methoxyphenyl, and ethoxyphenyl.

A compound according to formula I wherein R2 is H and wherein R3 is a doubly substituted phenyl selected from hydroxy-alkoxyphenyl, hydroxymethoxyphenyl, alkoxy-alkylphenyl, methoxy-alkylphenyl, hydroxy-alkylphenyl, and hydroxymethoxyphenyl.

A compound according to formula I wherein R2 is H and wherein R2-C-R3 is selected from phenyl , 4-methylphenyl, 4-methoxy phenyl, 4-hydroxyphenyl, 4-isopropylphenyl, 4-hydroxy-3-methoxyphenyl, and piperonyl.

A compound according to formula I wherein R2 is H and wherein R3 is selected from furanyl, alkylfuranyl, methylfuranyl, ethylfuranly, propylfuranyl, isopropylfuranyl, butylfuranyl, isobutylfuranyl.

A compound according to formula I wherein R2 is H and wherein R3 is selected from furan-2-yl, 5-methylfuran-2-yl, 3-methylfuran-2-yl, furan-3-yl (from: acetyl NSO).

A compound according to formula I wherein R2 is H and wherein R3 is selected from thiophenyl, alkylthiophenyl, methylthiophenyl, ethylthiophenyl, dialkylthiophenyl, dimethylthiophenyl, ethylmethylthiophenyl.

A compound according to formula I wherein R2 is H and wherein R3 is selected from thiophen-2-yl, 5-methylthiophen-2-yl, 5-ethylthiophen-2-yl, thiophen-3-yl, 2-methylthiophen-3-yl, 2,5-dimethylthiophen-3-yl.

A compound according to formula I wherein R2 is H and wherein R3 is selected from phenylalkyl and phenylalkenyl.

A compound according to formula I wherein R2 is H and wherein R3 is a phenylalkyl selected from benzyl, phenylethyl, phenylpropyl, phenylbutyl, phenylpentyl, phenylhexyl, phenylheptyl, phenyloctyl, phenylnonyl.

A compound according to formula I wherein R2 is H and wherein R3 is a phenylalkenyl selected from phenylethenyl, phenylpropenyl, phenylbutenyl, phenylpentenyl, phenylhexenyl, phenylheptenyl, phenyloctenyl, phenylnonenyl.

A compound according to formula I wherein R2 is H and wherein R3 is selected from 1-phenylethyl, 4-methyl-2-phenylbut-2-enyl, (Z)-4-methyl-2-phenylbut-2-enyl, 5-methyl-2-phenylpent-2-enyl, (Z)-5-methyl-2-phenylpent-2-enyl, benzyl, 2-phenylprop-2-enyl, (Z)-2-phenylprop-2-enyl, 1-phenylvinyl, 2-phenylvinyl.

A compound according to formula I wherein R2 is H and wherein R3 is selected from alkylthioalkyl, alkylthioalkenyl, alkyldisulfanylalkyl, alkyldisulfanylalkenyl, methylthioalkyl, methyl-methylthioalkyl, methylthioalkenyl, methyl-methylthioalkenyl, methylthioalkyl, methyl-methylthioalkyl, methylthioalkenyl, methyl-methylthioalkenyl, methyldisulfanylalkyl, methyl-methyldisulfanylalkyl, methyldisulfanylalkenyl, methyl-methyldisulfanylalkenyl.

A compound according to formula I wherein R2 is H and wherein R3 is selected from 3-(methylthlo)propyl, 2-(methylthio)propyl, 4-(methylthio)but-2-en-2-yl, (Z)-4-(methylthio)but-2-en-2-yl, 2-methyl-5-(methylthio)pent-2-en-2-yl, (E)-2-methyl-5-(methylthio)pent-2-en-2-yl, 1-((methyl(thio)methyl)-but-2-en-2-yl, (Z)-1-((methyl(thio)methyl)-but-2-en-2-yl, 2-(methylthio)ethyl, and 2-(2-methyl(disulfanyl)-prop-2-yl.

A compound according to formula I wherein R2 is H and wherein R3 has a maximum number of C atoms of 12 and is selected from singly branched hydroxyalkyl, doubly branched hydroxyalkyl, multiply branched hydroxyalkyl, hydroxydialkylalkyl, hydroxydimethylalkyl, hydroxydimethylbutyl, hydroxydimethylpentyl, hydroxydimethylhexyl, hydroxydimethylheptyl, hydroxydimethyloctyl, hydroxydimethylnonyl.

A compound according to formula I wherein R2 is H and wherein R3 is selected from 6-hydroxy-3,6-dimethylheptyl.

Alternatively, the groups of compounds above may have instead of the indicated R2 (H) a R2 selected from methyl, ethyl, propyl, butyl, pentyl, hexyl, heptyl, octyl, nonyl, decyl. These groups of precursor compounds will release, instead of the flavor aldehydes, flavor ketone compounds.

For the groups of precursor compounds releasing flavor aldehydes described above, or the alterntive groups of compounds described above that release flavor ketones, R1 is selected as indicated herein-above or selected from heptyl, nonyl, undecyl, tridecyl, pentadecyl, and heptadecyl.

### Groups of precursor compounds that release acetyl flavor compounds:

The following groups of compounds may be formed by reacting flavor compounds with a ketone to form the R2-C-R3 part of the compound of formula I, wherein C is the former carbonyl C-atom of the ketone educt The se precursor compounds will release acetyl flavor compounds accordingly. The resulting R3 residues are indicated below:

A compound according to formula I wherein R2 is methyl and R3 is selected from pyrazinyl, alkylpyrazinyl, methylpyrazinyl, dimethylpyrazinyl, trimethylpyrazinyl, ethylmethylpyrazinyl, ethyldimethylpyrazinyl, ethylpyrazinyl, diethylpyrazinyl, diethylmethylpyrazinyl.

A compound according to formula I wherein R2 is methyl and R3 is selected from pyrazinyl, 3-methylpyrazin-2-yl, 6-methylpyrazin-2-yl, 2,3-dimethylpyrazin-6-yl, 3,5-dimethylpyrazin-2-yl, 5-dimethylpyrazin-2-yl, 2-ethyl-pyrazin-6-yl, 5-ethyl-3-methylpyrazin-2-yl, 2-ethyl-5-methylpyrazin-3-yl, 2-ethyl-5,6-dimethylpyrazin-3-yl, 2-ethyl-3,5-dimethylpyrazin-6-yl, 3-ethyl-5-methylpyrazin-2-yl, 2-ethyl-pyrazin-5-yl, 2-ethyl-pyrazin-3-yl.

A compound according to formula I wherein R2 is methyl and R3 is selected from alkylpyridinyl, methylpyridinyl, dimethylpyridinyl, trimethylpyridinyl, tetramethylpyridinyl, ethylmethylpyridinyl, ethyldimethylpyridinyl.

A compound according to formula I wherein R2 is methyl and R3 is selected from pyridin-2-yl, 3-methylpyridin-2-yl , 5-methylpyridin-2-yl, 6-methylpyridin-2-yl, 3-ethylpyridin-2-yl, 3,5-dimethylpyridin-2-yl, 5,6-dimethylpyridin-2-yl, 3-ethyl-5-methylpyridin-2-yl, 3-ethyl-6-methylpyridin-2-yl, 3,5,6-trimethylpyridin-2-yl, 3-ethyl-5,6,-dimethylpyridin-2-yl, 2-ethyl-3,5-dimethylpyridin-6-yl, pyridin-3-yl, 2-methylpyridin-3-yl, 5-methylpyridin-3-yl, 6-methylpyridin-3-yl, 2,3-dimethylpyridin-5-yl, 2,6-dimethylpyridin-3-yl, 2,3,4,5-tetrahydropyridin-6-yl, 1,2,3,4-tetrahydropyridin-6-yl.

A compound according to formula I wherein R2 is methyl and R3 is selected from oxazolyl, thiazolyl, dihydrothiazolyl.

A compound according to formula I wherein R2 is methyl and R3 is selected from oxazol-2-yl, oxazol-4-yl, oxazol-5-yl, thiazol-2-yl, thiazol-4-yl, thiazol-5-yl, 4,5-dihydro-thiazol-2-yl, 2,5-dihydro-thiazol-2-yl.

A compound according to formula I wherein R2 is ethyl and R3 is selected from oxazolyl, thiazolyl, dihydrothiazolyl.

A compound according to formula I wherein R2 is ethyl and R3 is selected from thiazol-2-yl, oxazol-2-yl, oxazol-4-yl, oxazol-5-yl, thiazol-2-yl, thiazol-4-yl, thiazol-5-yl, 4,5-dihydro-thiazol-2-yl, 2,5-dihydro-thiazol-2-yl.

A compound according to formula I wherein R2 is methyl and R3 is selected from pyrrolyl, and dihydropyrrolyl.

A compound according to formula I wherein R2 is methyl and R3 is selected from *1H*-pyrrol-2-yl, *1H*-pyrol-3-yl, 3,4-dihydro-*2H-*pyrrol-5-yl, 2,3-dihydro-*1H*-pyrrol-5-yl.

A compound according to formula I wherein R2 is methyl and R3 is selected from thioalkyl, thiomethyl, thioethyl, thiopropyl, thiobutyl, thiopentyl, thiomethylethyl, thiomethylpropyl, thiomethylbutyl, alkyl(methylthio)alkyl, methyl(methylthio)alkyl, methyl(methylthio)ethyl, methyl(methylthio)propyl, methyl(methylthio)butyl.

A compound according to formula I wherein R2 is methyl and R3 is selected from 1-thiopropyl, 2-thiopropyl, 3-thiopropyl, thiomethyl, 2-thio-2-methylpropyl, 2-methyl-2-(methylthio)propyl.

A compound according to formula I wherein R2 is methyl and R3 is selected from furanyl-substituted straight-chain or branched alkyl including methyl, ethyl, propyl, butyl, and pentyl, optionally substituted with one or more of OH, 0, and SH; straight-chain or branched furanyl-substituted alkylthioalkyl, and furanyl-substituted methylthioalkyl

A compound according to formula I wherein R2 is methyl and R3 is selected from 1-((furan-2-yl)methylthio)-1-hydroxyethyl, and 2-((furan-2-yl)methylthio)-2-methylpropyl.

A compound according to formula I wherein R2 is methyl and R3 is selected from straight-chain or branched substituted alkyl substituted with one or more of O, and OH, a straigh-chain or branched substituted alkyl substituted with one or more of O and OH wherein the alkyl is selected from ethyl, propyl, butyl, pentyl, hexyl, heptyl, and octyl, a straight-chain or branched (alkoxycarbonyl)alkyl, a straigh-chain or branched (alkoxycarbonyl)alkyl wherein the alkyl is selected from ethyl, propyl, butyl, pentyl, hexyl, heptyl, and octyl.

A compound according to formula I wherein R2 is methyl and R3 is selected from 1-oxopentyl, 1-oxobutyl,1-oxopropyl, 3-oxo-oct-2-yl, 2-(ethoxycarbonyl)ethyl.

A compound according to formula I wherein R2 is methyl and R3 is selected from alkylphenyl, phenylalkyl, C9 arylalkyl, C10 arylalkyl, phenylalkenyl, C9 arylalkenyl, C10 arylalkenyl, methoxyphenyl, methoxyphenylalkyl, methoxyphenylalkenyl, hydroxyphenylalkyl, hydroxyphenylalkenyl, hydroxyphenylalkyl.

A compound according to formula I wherein R2 is methyl and R3 is selected from 4-methylphenyl, 2-phenylethenyl, 4-methoxyphenyl, 2-(4-methoxyphenyl)ethyl, 2-(4-hydroxyphenyl)ethyl, and benzoyl.

Alternatively, the groups of compounds above may have instead of the indicated R2 (methyl) a R2 selected from H (in which case the reacting flavor compound/educt that forms the R2-C-R3 residue of the precursor and the released flavor compound is an aldehyde), ethyl, propyl, butyl, pentyl, hexyl, heptyl, octyl, pentyl, decyl.

For the groups of precursor compounds that release flavor acetyls described above, R1 is selected as indicated herein-above or selected from heptyl, nonyl, undecyl, tridecyl, pentadecyl, and heptadecyl.

The following groups of compounds will release alkylaldehyde and alkenylaldehyde flavors:

A compound according to formula I wherein R2 is H and R3 is selected from methyl, ethyl, propyl, butyl, pentyl, hexyl, heptyl, octyl, nonyl, decyl, undecyl, dodecyl, tridecyl.

A compound according to formula I wherein R2 is H and R3 is a singly branched alkyl selected from isopropyl, methylbutyl, methylpentyl, methylhexyl, methylheptyl, methyloctyl, methylnonyl, methyldecyl, methylundecyl, methyldodecyl, methyltridecyl.

A compound according to formula I wherein R2 is H and R3 is 11-methyl-dodecyl.

A compound according to formula I wherein R2 is H and R3 is 1-methyl-propyl.

A compound according to formula I wherein R2 is H and R3 is 2-methyl-propyl.

A compound according to formula I wherein R2 is H and R3 is an alkenyl with a single double bond selected from propenyl, butenyl, pentenyl, hexenyl, heptenyl, octenyl, nonenyl, decenyl, undecenyl, dodecenyl, tridecenyl.

A compound according to formula I wherein R2 is H and R3 is an alkenyl with a single double bond selected from prop-1-enyl, but-1-enyl, buten-2-yl, but-3-enyl, pent-1-enyl, pent-2-enyl, pent-3-enyl, hex-1-enyl, hex-3-enyl, hept-1-enyl, oct-1-enyl, oct-5-enyl, non-1-enyl, non-3-enyl, non-8-enyl, dec-1-enyl, dec-8-enyl, dec-9-enyl, undec-1-enyl, dodec-1-enyl, tridec-1-enyl.

A compound according to formula I wherein R2 is H and R3 is an alkenyl with two double bonds selected from penta-1,3-dienyl, hexa-1,3-dienyl, hepta-1,3-dienyl, octa-1,3-dienyl, octa-1,5-dienyl, nona-1,3-dienyl, deca-1,3-dienyl, undeca-1,3-dienyl, dodeca-1,3-dienyl, trideca1,3-dienyl, octa-1,5-dienyl.

A compound according to formula I wherein R2 is H and R3 is an alkenyl selected from an alkenyl with multiple double bonds, and an alkenyl with three double bonds.

A compound according to formula I wherein R2 is H and R3 is an alkenyl with three double bonds octatrienyl, nonatrienyl, decatrienyl, undecatrienyl, dodecatrienyl.

A compound according to formula I wherein R2 is H and R3 is an alkenyl with three double bonds selected from octa-1,3,5-trienyl, nona-1,3,5-hienyl, deca-1,3,5-trienyl, undeca-1,3,5-trienyl, dodeca1,3,5-trienyl.

A compound according to formula I wherein R2 is H and R3 is an alkyl or alkenyl substituted with one, two, or more methyl groups.

A compound according to formula I wherein R2 is H and R3 is an alkyl with a methyl group including but not limited to 1-methyl-methyl, 1-methyl-ethyl, 1-methyl-propyl, 1-methyl-butyl, 1-methyl-pentyl, 1-methyl-hexyl, 1-methyl-heptyl, 1-methyl-octyl, 1-methyl-nonyl.

A compound according to formula I wherein R2 is H and R3 is an alkenyl with a methyl group including but not limited to 2-methyl-prop-2-enyl, 3-methylbut-1-enyl.

A compound according to formula I wherein R2 is H and R3 is an alkyl with two methyl groups including but not limited to 3,6-dimethylheptyl.

A compound according to formula I wherein R2 is H and R3 is an alkenyl with two double bonds and two methyl groups including but not limited to 3,6-dimethylhepta-1,5-dienyl.

A compound according to formula I wherein R2 is H and R3 is an alkenyl with a double bond and two methyl groups including but not limited to 3,6-dimethylhept-5-enyl.

A compound according to formula I wherein R2 is H and R3 is a branched alkyl or alkenyl substituted with one or two ethyl groups.

A compound according to formula I wherein R2 is H and R3 is an alkyl substituted with one ethyl group including but not limited to 1-ethyl-propyl.

A compound according to formula I wherein R2 is H and R3 is an alk-3-yl or alken-3-yl substituted with one or more methyl groups, including but not limited to 2, 6-dimethyl.hept-3-en-3-yl.

The above-described alkyl and alkenyl R3 residues include but are not limited to alk-1-yl, alk-1-enyl, alk-2-yl, alk-2-enyl, alk-3-yl, alk-3-enyl, alk-4-yl, and alk-4-enyl residues.

A compound according to formula I wherein R2 is H and R3 is pent-2-en-2-yl.

Alternatively to the above-described groups of compounds wherein R3 = alkyl or alkenyl, instead of the indicated R2 (H), R2 may be selected from methyl, ethyl, propyl, butyl, pentyl, hexyl, heptyl, octyl, pentyl, decyl, undecyl, dodecyl, tridecyl, tetradecyl, pentadecyl; still alternatively, R2 may be selected from H, methyl, ethyl, butyl, 2-butenyl, or vinyl. For the above-described compounds, R1 is selected as indicated herein-above or selected from heptyl, nonyl, undecyl, tridecyl, pentadecyl, and heptadecyl.

For the above-described groups of compounds wherein R3 is alkenyl, double bonds may be in cis or trans position.

### The following groups of precursor compounds will release alkylketone or alkenylketone flavors:

A compound according to formula I wherein R2 is methyl and R3 is an alkyl selected from ethyl, propyl, butyl, pentyl, hexyl, heptyl, octyl, nonyl, decyl, undecyl, dodecyl, tridecyl.

A compound according to formula I wherein R2 is methyl and R3 is an alkenyl selected from ethenyl, propenyl, butenyl, pentenyl, hexenyl, heptenyl, octenyl, nonenyl, decenyl, undecenyl, dodecenyl, tridecenyl, tetradecenyl, pentadecenyl.

A compound according to formula I wherein R2 is methyl and R3 is an alkenyl selected from prop-1-enyl, but-1-enyl, pent-1-enyl, hex-1-enyl, hept-1-enyl.

A compound according to formula I wherein R2 is methyl and R3 is a methylalkenyl selected from methylethenyl, methylpropenyl, methylbutenyl, methylpentenyl, methylhexenyl, methylheptenyl, methyloctenyl, methylnonenyl, methyldecenyl, methylundecenyl, methyldodecenyl, methyltridecenyl.

A compound according to formula I wherein R2 is methyl and R3 is a methylalkenyl as defined above including but not limited to 4-methyl-pent-3-enyl, 4-methyl-penta-1,3-dienyl.

A compound according to formula I wherein R2 is methyl and R3 is an alkadienyl selected from butadienyl, pentadienyl, hexadienyl, heptadienyl, octadienyl, nonadienyl, decadienyl, undecadienyl, dodecadienyl, tridecadienyl, tetradecadienyl, pentadecadienyl.

Acompound according to formula I wherein R2 is methyl and R3 is an alkadienyl selected from penta-1,3-dienyl, hexa-1,3-dienyl, hepta-1,3-dienyl, octa-1,3-dienyl.

A compound according to formula I wherein R2 is methyl and R3 is a hydroxy-alkyl including but not limited to hydroxy-ethyl, hydroxy-propyl, hydroxy-butyl, hydroxy-pentyl, hydroxy-hexyl, hydroxy-heptyl, 1-hydroxy-ethyl, 1-hydroxy-propyl, 1-hydroxy-butyl, 1-hydroxy-pentyl, 1-hydroxy-hexyl, 1-hydroxyheptyl.

A compound according to formula I wherein R2 is methyl and R3 is an oxo-alkyl including but not limited to oxo-ethyl, oxo-propyl, oxo-butyl, oxo-pentyl, oxo-hexyl, oxo-heptyl, 1-oxo-ethyl, 1-oxo-propyl, 1-oxo-butyl, 1-oxo-pentyl, 1-oxo-hexyl, 1-oxoheptyl.

Alternatively to the above-described groups of compounds wherein R3 is optionally substituted alkyl or alkenyl, instead of the indicated R2 (methyl), R2 may be selected from methyl, ethyl, propyl, butyl, pentyl, hexyl, heptyl, octyl, pentyl, decyl, undecyl, dodecyl, tridecyl, tetradecyl, pentadecyl; still alternatively, R2 may be selected from H, methyl, ethyl, butyl, 2-butenyl, or vinyl.

A compound according to formula I wherein R2 is ethyl and R3 is selected from ethyl, propyl, prop-1-enyl, butyl, pentyl, hexyl, heptyl.

A compound according to formula I wherein R2 is 2-butyl and R3 is prop-1-enyl

A compound according to formula I wherein R2 is vinyl and R3 is selected from ethyl, propyl, prop-1-enyl, butyl, pentyl, hexyl, heptyl.

For the above-described groups of compounds wherein R3 is optionally substituted alkyl or alkenyl, R1 is selected as indicated herein-above or selected from heptyl, nonyl, undecyl, tridecyl, pentadecyl, and heptadecyl.

For the above-described groups of compounds wherein R3 is optionally substituted alkenyl, double bonds may be in cis or trans position.

Some examples of useful flavor precursor compounds are given in table 2 below, further example compounds can be found in the examples herein-below:

| | | |
|---|---|---|
| 1 | 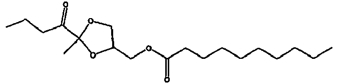 | example 3 |
| | m=0, n=1, R1=nonyl, R2=methyl, R3=butyroyl | |
| | -> releases 2,3-hexandione | |
| 2 | 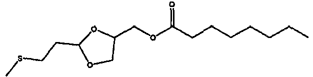 | example 4 |
| | m=0, n=1, R1=heptyl, R2=H, R3=2-methylthioethyl | |
| | -> releases methional | |
| 3 | 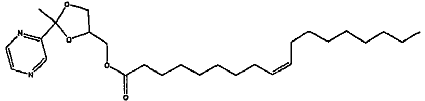 | example 5 |
| | m=0, n=1, R1=hepatadec-8-enyl, R2=methyl, R3=pyrazinyl | |
| | -> releases acetylpyrazine | |
| 4 | 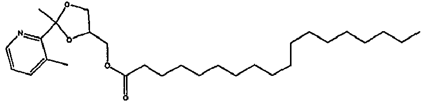 | example 6 |
| | m=0, n=1, R1=heptadecyl, R2=methyl, R3=3-methylpyridin-2-yl | |
| | -> releases 2-acetyl-3-methylpyridine | |
| 5 | 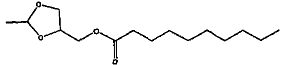 | example 7 |
| | m=0, n=1, R1=nonyl, R2=H, R3=methyl | |
| | -> releases acetaldehyde | |
| 6 | 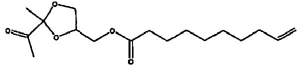 | example 8 |
| | m=0, n=1, R1=non-8-enyl, R2=methyl, R3=acetyl | |
| | -> releases diacetyl | |
| 7 | 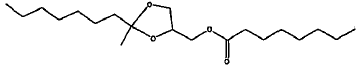 | example 9 |
| | m=0, n=1, R1=heptyl, R2=methyl, R3=heptyl | |
| | -> releases 2-nonanone | |
| 8 | 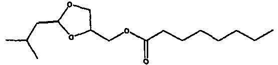 | example 10 |
| | m=0, n=1, R1=heptyl, R2=H, R3=2-methylpropyl | |
| | -> releases isovaleraldehyde | |
| 9 | 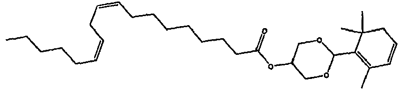 | |
| | m=1, n=0, R1=heptadeca-8,11-dienyl, R2=H, R3=2,2,5-Trimethyl-cyclohexa-4,6-dien1-yl | |
| | -> releases Saffranal | |
| 10 | 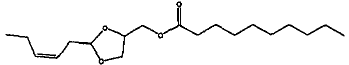 | example 2 |
| | m=0, n=1, R1=nonyl, R2=H, K3=2-pentenyl | |
| | -> releases cis 3-hexenal | |
| 11 | 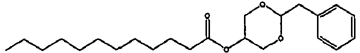 | |
| | m=1, n=0, R1=undecyl, R2=H, R3=benzyl | |
| | -> releases phenylacetaldehyde | |
| 12 | 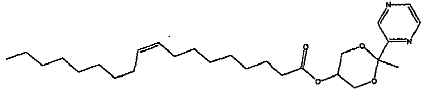 | |
| | m=1, n=0, R1=8-Heptadecenyl, R2=methyl, R3=pyrazinyl | |
| | -> releases acetylpyrazin | |
| 13 | 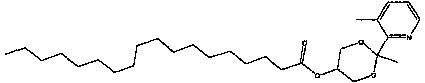 | |
| | m=1, n=0, R1=heptadecyl, R2=methyl, R3=3-methylpyridin-2-yl | |
| | -> releases 3-methyl-2-acetylpyridin | |
| 14 | 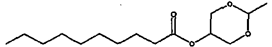 | |
| | m=1, n=0, R1=nonyl, R2=H, R3=methyl | |
| | -> releases acetaldehyde | |
| 15 | 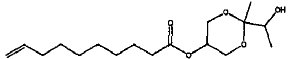 | |
| | m=1, n=0, R1=8-nonenyl, R2=methyl, R3=1-hydroxiethyl | |
| | -> releases acetoin | |
| 16 | 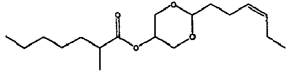 | |
| | m=1, n=0, R1=2-heptyl, R2=H, R3=3-hexenyl | |
| | -> releases cis-4-heptenal | |
| 17 | 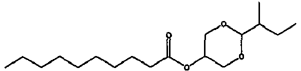 | |
| | m=1, n=0, R1=nonyl, R2=H, R3=2-butyl | |
| | -> releases 2-methylbutanal | |
| 18 | 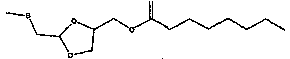 | |
| | m=0, n=1, R1=heptyl, R2=H, R3=methylthiomethyl | |
| | -> releases methylthioacetaldehyde | |
| 19 | 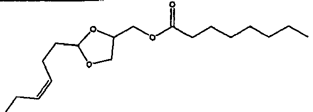 | |
| | m=0, n=1, R1=heptyl, R2=H, R3=3-hexenyl | |
| | -> releases cis-4-heptenal | |
| 20 | 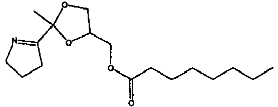 | |
| | m=0, n=1, R1=heptyl, R2=methyl, R3=1-pyrrolin-2-yl | |
| | -> releases 2-acetylpyrrolin | |
| 21 | 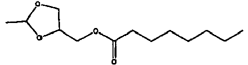 | |
| | m=0, n=1, R1=heptyl, R2=H, R3=methyl | |
| | -> releases acetaldehyde | |
| 22 | 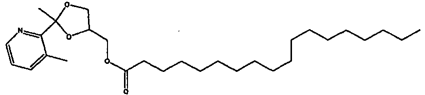 | |
| | m=0, n=1, R1= heptadecyl, R2=methyl, R3=3-methyl-pyridin-2-yl | |
| | -> releases 3-methyl-2-acetylpyridine | |
| 23 | 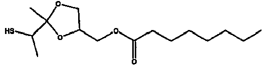 | |
| | m=0, n=1, R1= heptyl, R2=methyl, R3=1-thioethyl | |
| | -> releases 3-thio-2-butanone | |
| 24 | 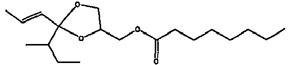 | |
| | m=0, n=1, R1= heptyl, R2=2-butyl, R3=1-propenyl | |
| | -> releases 3-methyl-hept-5-en-4-one | |
| 25 | 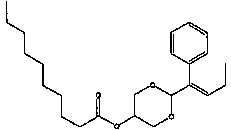 | |
| | m=1, n=0, R1=nonyl, R2=H, R3=1-phenyl-1-buten-1-yl | |
| | -> releases 2-phenyl-2-pentenal | |
| 26 | 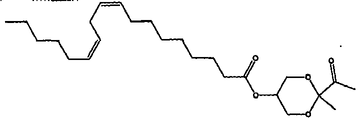 | |
| | m=1, n=0, R1=heptadeca-8,11-dienyl, R2=methyl, R3=acetyl | |
| | -> releases diacetyl | |
| 27 | 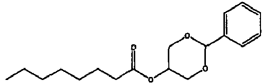 | |
| | m=1, n=0, R1=heptyl, R2=H, R3=phenyl | |
| | -> releases benzaldehyde | |
| 28 | 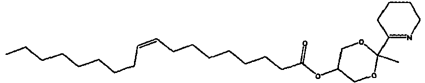 | |
| | m=1, n=0, R1=8-heptadecenyl, R2=methyl, R3=3,4,5,6-tetrahydropyridin-2-yl | |
| | -> releases 2-acetyl-tetrahydropyridin | |
| 29 | 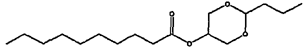 | |
| | m=1, n=0, R1=nonyl, R2=H, R3=propyl | |
| | -> releases butyraldehyd | |
| 30 | 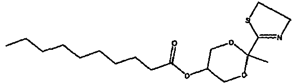 | |
| | m=1, n=0, R1=nonyl, R2=methyl, R3=1-thiazolin-2-yl | |
| | -> releases 2-acetylthiazolin | |
| 31 | 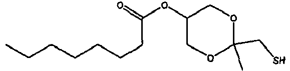 | |
| | m=1, n=0, R1=heptyl, R2=methyl, R3=thiomethyl | |
| | -> releases thioacetone | |
| 32 | 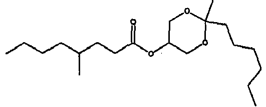 | |
| | m=1, n=0, R1=3-methylheptyl, R2=methyl, R3=hexyl | |
| | -> releases 2-octanone | |
| 33 | 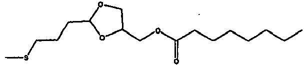 | |
| | m=0, n=1, R1=heptyl, R2=H, R3=3-methyl-thiopropyl | |
| | -> releases 4-methylthiobutanol | |
| 34 | 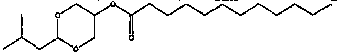 | |
| | m=1, n=0, R1= undecyl, R2=H, R3= 2-methylpropyl | |
| | ->releases isovaleraldehyde | |
| 35 | 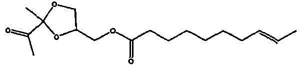 | |
| | m=0, n=1, R1=non-7-enyl, R2=methyl, R3=acetyl | |
| | -> releases diacetyl | |

Alkyl residues of formula I include, without limitation, methyl (C1), ethyl (C2), propyl and/or isopropyl (C3), butyl and/or isobutyl (C4), pentyl and/or isopentyl (C5), hexyl and/or isohexyl (C6), heptyl and/or isoheptyl (C7), octyl and/or isooctyl (C8), nonyl and/or isononyl (C9), decyl and/or isodecyl (C10), undecyl and/or isodecyl (C11), dodecyl and/or isododecyl (C12), tridecyl and/or isotridecyl (C13), tetradecyl and/or isotetradecyl (C14), pentadecyl and/or isopentadecyl (C15), hexadecyl and/or isohexadecyl (C16), heptadecyl and/or isoheptadecyl (C17), octadecyl and/or isooctadecyl (C18).

Alkenyl residues include, without limitation, ethenyl (C2), propenyl and/or isopropenyl (C3), butenyl and/or isobutenyl (C4), pentenyl and/or isopentenyl (C5), hexenyl and/or isohexenyl (C6), heptenyl and/or isoheptenyl (C7), octenyl and/or isooctenyl (C8), nonenyl and/or isononenyl (C9), decenyl and/or isodecenyl (C10), undecenyl and/or isoundecenyl (C11), dodecenyl and/or isododecenyl (C12), tridecenyl and/or isotridecenyl (C13), tetradecenyl and/or isotetradecenyl (C14), pentadecenyl and/or isopentadecenyl (C15).

The flavor precursor can be added directly to a food product, or can be provided as a flavor composition to be added to food products.

The term "food product" as used herein includes any food product, for example, without limitation, cereal products, rice products, tapioca products, sago products, baker's products, biscuit products, pastry products, bread products, confectionery products, desert products, gums, chewing gums, chocolates, ices, honey products, treacle products, yeast products, baking-powder, salt and spice products, savory products, mustard products, vinegar products, sauces (condiments), tobacco products, cigars, cigarettes, processed foods, cooked fruits and vegetable products, meat and meat products, jellies, jams, fruit sauces, egg products, milk and dairy products, cheese products, butter and butter substitute products, milk substitute products, soy products, edible oils and fat products, medicaments, beverages, alcoholic drinks, beers, soft drinks, mineral and aerated waters and other non-alcoholic drinks, fruit drinks, fruit juices, coffee, artificial coffee, tea, cocoa, including forms requiring reconstitution, food extracts, plant extracts, meat extracts, condiments, sweeteners, nutraceuticals, gelatins, pharmaceutical and non-pharmaceutical gums, tablets, lozenges, drops, emulsions, elixirs, syrups and other preparations for making beverages.

The flavor composition may comprise well known food additives, for example, without limitation, solvents, binders, diluents, disintegranting agents, lubricants, flavoring agents, coloring agents, preservatives, antioxidants, emulsifiers, stabilisers, flavor-enhancers, sweetening agents, anti-caking agents, and the like. Examples of such carriers ordiluents for flavor or fragrance compounds may be found e.g. in "Perfume and Flavor Materials of Natural Origin", S. Arctander, Ed., Elizabeth, N.J., 1960; in "Perfume and Flavor Chemicals", S. Arctander, Ed., Vol. I & II, Allured Publishing Corporation, Carol Stream, USA, 1994; in "Flavourings", E. Ziegler and H. Ziegler (ed.), Wiley-VCH Weinheim, 1998, and "CTFA Cosmetic Ingredient Handbook", J.M. Nikitakis (ed.), 1st ed., The Cosmetic, Toiletry and Fragrance Association, Inc., Washington, 1988.

The flavor composition may be added in any suitable form, for example as a liquid, as a paste, or in encapsulated form bound to or coated onto carriers/particles or as a powder.

There now follows a series of non-limiting examples that serve to illustrate the invention.

### Examples

Unless stated otherwise, all % indicated are in wt/wt.

### Example 1

### Preparation of precursor compounds from Glycerin-monodecanoate and release of aroma compound by heat hydrolysis or enzyme/acid catalysis in the mouth

The precursor is prepared as follows, 1g of Glycerin-monodecanoate (also known as CAS 2277-23-8 or 1-Monocaprin, commercially available from Indofine) and a suitable amount (500mg unless otherwise stated) of an aroma compound are dissolved in 20ml hexane with a trace of HCl conc. as catalyst in a Dean-Stark trap and boiled for about 2 hours until the formation of water stops. To the mixture, 0.5ml of a saturated brine solution is added, and the mixture is shaken. The organic phase (hexane) is separated and dried with a small amount of Magnesium sulfate. The hexane is distilled off under vacuum. The resulting precursor material is further purified by vacuum distillation, and may be further purified by chromatography.

When the precursor is boiled in water, a strong aroma (the aroma of the reacted aroma compound that is being hydrolysed) is released.

Upon tasting of the precursor (1-10 ppm) in an aqueous 5% sugar solution or a 0,3% salt solution at room temperature, a weak, bland aroma that slowly develops the aroma corresponding to the employed aroma compound is perceived.

### Example 2

### (2-((Z)-pent-2-enyl)-1,3-dioxolan-4-yl)methyl decanoate

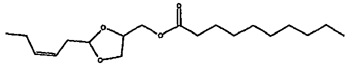

The precursor is a compound of formula I with n=1, m=0, R1=nonyl, R2=H, R3=2-pentenyl and is able to release cis 3-hexenal.

The precursor is prepared using Glycerin-monodecanoate and cis 3-hexenal as described in example 1. The purified precursor is a paste with a weak, green odor.

When the precursor is boiled in water, a strong, fresh, green, gras- or apple-like aroma (the aroma of cis 3-hexenal) is released.

Upon tasting of the precursor (1ppm) in an aqueous 5% sugar solution at room temperature, a weak, bland aroma that slowly develops a fresh green aroma is perceived.

### Example 3

### (2-butyryl-2-methyl-1,3-dioxolan-4-yl)methyl decanoate

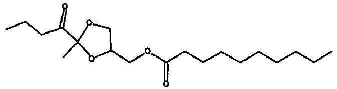

The precursor is a compound of formula I with n=1, m=0, R1=nonyl, R2=methyl, R3=butyroyl and is able to release 2,3-hexandione.

The precursor is prepared using Glycerin-monodecanoate and 2,3-hexandione as described in example 1.The purified precursor is a clear, yellow paste with a weak, buttery odor.

When the precursor is boiled in water, a fresh, buttery, creamy, fruity aroma (the aroma of 2,3-hexandione) is released.

Upon tasting of the precursor (1ppm) in an aqueous 5% sugar solution at room temperature, a weak, bland aroma that slowly develops a creamy, fruity aroma is perceived.

### Example 4

### (2-(2-(methylthio)ethyl)-1,3-dioxolan-4-yl)methyl octanoate

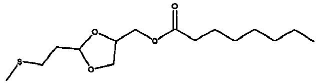

The precursor compound is a compound of formula I with n=1,m=0, R1=heptyl, R2=H, R3= 2-methylthioethyl and is able to release methional.

The precursor is prepared using Glycerin-monooctanoate and methional as described in example 1.The purified precursor is a clear, yellowish paste with a sulfurous odor.

When the precursor is boiled in water, a strong sulfuruos, potato, cooked tomato aroma (the aroma of methional) is released.

Upon tasting of the precursor in an aqueous 0,3% salt solution at room temperature, a weak, bland aroma that slowly develops a fresh cooked potato and tomato impression is perceived.

### Example 5

### (2-methyl-2-(pyrazin-2-yl)-1,3-dioxolan-4-yl)methyl oleate

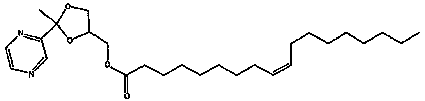

The precursor compound is a compound of formula I with n=1, m=0, R1=heptadec-8-enyl, R2=methyl, R3=pyrazinyl and is able to release acetylpyrazine.

The precursor is prepared using Glycerin-monooleate and acetylpyrazine as described in example 1. The purified precursor is a creamy paste with a weak, nutty roasted odor.

When the precursor is boiled in water, a nutty, roasted aroma (the aroma of acetylpyrazine) is released.

Upon tasting of the precursor (10 ppm) in an aqueous 5% sugar solution at room temperature, a weak, bland aroma that slowly develops a roasted bread crust aroma is perceived.

### Example 6

### (2-methyl-2-(3-methylpyridin-2-yl)-1,3-dioxolan-4-yl)methyl stearate

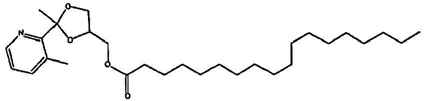

The precursor compound is a compound of formula I with n=1, m=0, R1= heptadecyl, R2=methyl, R3=3-methylpyridin-2-yl that is able to release 2-acetyl-3-methylpyridine.

The precursor is prepared using Glycerin-monostearate and 2-acetyl-3-methylpyridine as described in example 1. The purified precursor is a creamy paste with a weak, nutty roasted odor.

When the precursor is boiled in water, a nutty, roasted aroma (the aroma of 2-acetyl-3-methylpyridine) is released.

Upon tasting of the precursor (1ppm) in an aqueous 5% sugar solution at room temperature, a weak, bland aroma that slowly develops a roasted nutty aroma is perceived.

### Example 7

### (2-methyl-1,3-dioxolan-4-yl)methyl decanoate

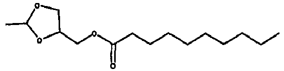

The precursor compound is a compound of formula I with n=1, m=0, R1=nonyl, R2=H, R3=methyl that is able to release acetaldehyde.

The precursor is prepared using Glycerin-monodecanoate as described in example 1 subject to the following modification:
5 g of cold (ca 10°C) Acetaldehyde (CAS 75-07-0 Supplier: Aldrich) are dissolved in 20ml cold hexane (ca 10°C) with 5g of glycerin monodecanaoate and a trace of HCl conc. as catalyst in a pressure reaction vessel and heated for ca. 2 hours. The purified precursor is a paste with a weak, chemical odor.

When the precursor is boiled in water, a fresh juicy, watery aroma (the aroma of acetaldehyde) is released.

Upon tasting of the precursor in an aqueous 20ppm sugar solution at room temperature, a weak, bland aroma that slowly develops a fresh juicy, watery aroma is perceived.

### Example 8

### (2-acetyl-2-methyl-1,3-dioxolan-4-yl)methyl dec-9-enoate

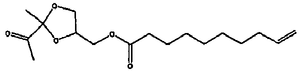

The precursor compound is a compound of formula I with n=1, m=0, R1=8-nonenyl, R2=methyl, R3= acetyl that is able to release diacetyl.

The precursor is prepared using 1-Glycerindec-9-enoate and diacetyl as described in example 1 subject to the following modification: 1g of Glycerin-monodecanoate is replaced with 1g 1-Glycerindec-9-enoate, which is synthesized as described below and reacted with 500mg of diacetyl (CAS 431-03-8, Aldrich) as described in example 1. The purified percursor is a yellowish paste with a weak, buttery odor.

When the precursor is boiled in water, a fresh, buttery, creamy, fruity aroma (the aroma of diacetyl) is released.

Upon tasting of the precursor (1 ppm) in an aqueous 5% sugar solution at room temperature, a weak, bland aroma that slowly develops a fresh, buttery, creamy, fruity aroma is perceived.

### Synthesis of 1-Glycerindec-9-enoate:

10 g of 9-Decenoic acid (CAS 14436-32-9, Bedoukian), 10 g of glycidol (CAS 556-52-5, Aldrich) and 100mg Amberlyst A-26(OH) (Aldrich) are boiled under reflux with 200ml hexane in a round bottom flask with reflux condensor and magnetic stirrer for 24hrs. The solution is filtered and the volatiles are distilled off under vacuum. The formed 1-Glycerindec-9-enoate can be used as such or purified further by chromatography.

### Example 9

### (2-heptyl-2-methyl-1,3-dioxolan-4-yl)methyl octanoate

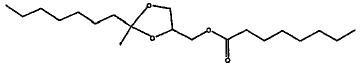

The precursor compound is a compound of formula I with n=1,m=0, R1= heptyl, R2=methyl, R3= heptyl (C7 saturated) that is able to release 2-nonanone.

The compound is prepared using Glycerin-monooctanoate and 2-nonanone as described in example 1 subject to the following modifications: 1g of Glycerin-monooctanoate (CAS 502-54-5, also known as 1-Monocaprylin, commercially available from indofine) and 500mg of 2-nonanone (CAS 821-55-6, Aldrich) are dissolved in 20 mlhexane.

The resulting precursor material may be further purified by chromatography. The purified precursor is a whitish paste with a weak, ketony aroma.

When the precursor is boiled in water, a fresh, bluecheese, creamy, fruity aroma (the aroma of 2-nonanone) is released.

Upon tasting of the precursor (1ppm) in an aqueous 5% sugar solution at room temperature, a weak, bland aroma that slowly develops a fresh, bluecheese, creamy, fruity aroma is perceived.

### Example 10

### (2-isobutyl-1,3-dioxolan-4-yl)methyl octanoate

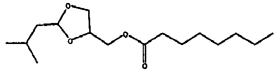

The precursor compound is a compound of formula I with n=1, m=0, R1= = heptyl, R2=H, R3= 2-methylpropyl that is able to release isovaleraldehyde.

The compound is prepared using Glycerin-monooctanoate and isovaleraldehyde as described in example 1 subject to the following modifications: 1g of Glycerin-monooctanoate (CAS 502-54-5, also known as 1-Monocaprylin, commercially available from Indofine) and 500mg of isovaleraldehyde (CAS 590-86-3, Aldrich) are dissolved in 20ml hexane. The purified precursor is a whitish paste with a weak, aldehydic aroma.

When the precursor is boiled in water, a fresh, cocoa aroma (the aroma of isovaleraldehyde) is released.

Upon tasting of the precursor (1ppm) in an aqueous 5% sugar solution at room temperature, a weak, bland aroma that slowly develops a cocoa aroma is perceived.

### Example 11

### (2-isobutyl-1,3-dioxan-5-yl) laurate

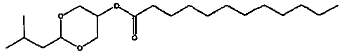

The precursor compound is a compound of formula I with n=0, m=1, R1= undecyl, R2=H, R3= 2-methylpropyl that is able to release isovaleraldehyde.

The compound is prepared using Glycerin-2-monododecanoate and isovaleraldehyde as described in example 1 subject to the following modifications: 1g of Glycerin-2-monododecanoate (CAS 1678-45-1, also known as 2-Monolaurin, commercially available from Indofine) and 500 mg of isovaleraldehyde (CAS 590-86-3 Supplier: Aldrich) are dissolved in 20 ml hexane. The purified precursor is a whitish paste with a weak, aldehydyc aroma.

When the precursor is boiled in water, a fresh, cocoa aroma (the aroma of isovaleraldehyde) is released.

Upon tasting of the precursor (1ppm) in an aqueous 5% sugar solution at room temperature, a weak, bland aroma that slowly develops a cocoa aroma is perceived.

### Example 12

### Cocoa flavor precursor

### Preparation of Cocoa-Monoglyceride:

A mixture of monoglycerides, derived from cocoabutter is prepared by stirring 60 g cocoabutter with 20 g glycerine, catalyzed by 100 mg HCl conc. in a roundbottom flask at 100°C for 20 hours.

For the topnote, 1.0 g Isovaleraldehyde, 5.0 g 2-Methylbutyraldehyde, and 1.0 g Phenylacetaldehyde are mixed together.

For the top aroma, 1g topnote are mixed with 99 g Migliol (vegetable oil). The top aroma has a very strong fresh cocoa aroma.

### Preparation of the precursors:

1 g topnote is mixed with 20 g of Cocoa-Monoglyceride and heated for 10 hours in a roundbottom flask with reflux cooler to form the precursors. The resulting cooled precursor mixture has a weak cocoa aroma.

### Comparison of precursors and top aroma in a milk drink:

Top aroma: A milk-drink powder is flavored with 0.1% (wt/wt) of the top aroma.
Precursor mixture: A milk-drink powder is flavored with 0.1% (wt/wt) of the Precursor mixture.

From the freshly prepared milk drink power, hot milk drinks are prepared and sensorically evaluated. The top aroma flavored milk drink has a strong, pungent cocoa aroma, while the precursor mixture flavored milk drink has a weaker balanced cocoa aroma.

After 2 months storage at room temperature of the drink powder, a sensory evaluation is performed by adding the powder to hot milk and stirring for 2 minutes. The top aroma milk drink is bland and has completely lost its original cocoa aroma. The precursor mixture milk drink shows a nice, balanced cocoa aroma and has a good mouthfeel.

### Examples 13

### Apple Flavor Precursor

### Preparation of Apple-Monoglyceride:

A mixture of 25 g Triacetin, 25 g Miglyol, 20 g glycerin, 25g 2-methylbutyric acid and 100mg HCl conc. is stirred in a roundbottom flask with distillation head at 110 °C for 6 hours. The formed water is distilled off. The formed product is used without further purification.

For the topnote, 5.0 g hexanal and 5.0 g 2-hexenal are mixed together.

For the top aroma, 1g topnote are mixed with 99 g migliol (vegetable oil). This resulting top aroma has a very strong fresh apple aroma.

### Preparation of the precursors:

1 g of topnote is mixed with 20 g of apple-monoglyceride and heated for 8 hours in a roundbottom flask with reflux cooler to form the precursors. The resulting cooled mixture has a weak apple aroma.

### Comparison of precursors and top aroma in a biscuits:

A biscuit short dough is prepared as follows:

| Ingredients: | % (wt/wt) |
|---|---|
| 1) Plain Flour (~10% (wt/wt) protein level) | 52.31 |
| 2) Vegetable Shortening BM 3030 (Woodlands Sunny Foods, Senoko, Singapore) | 17.26 |
| 3) Fine Milled Sugar | 17.40 |
| 4) Glucose Syrup 42 DE | 3.45 |
| 5) Skim Milk Powder | 1.49 |
| 6) Salt | 0.25 |
| 7) Sodium Bicarbonate | 0.31 |
| 8) Ammonium Bicarbonate | 0.21 |
| 10a) top aroma | 0.2 |
| 10b) precursor | 0.1 |
| | add 100.00 (water) |

Ingredients 2 - 6 are pre-blended in the mixing bowl, and mixed at low speed, then mixed further at medium speed for about 3 minutes. At low speed, the pre-dissolved solutions of sodium bicarbonate, ammonium carbonate, and 0.2% (wt/wt) of the top aroma or 0.1% (wt/wt) of the precursors are added; then the remaining water is added and mixed for 1 minute. Mixing is continued at medium speed for about 3 minutes to form a homogenous mixture. The flour is added at low speed to obtain a dough. The dough is formed to a sheet of a thickness of 4mm and cut into biscuit shapes using a stamp cutter. Biscuits are baked on a wire tray at a temperature of 200°C for about 8 to 10 minutes.

During the baking process a strong apple aroma from the baking oven is observed from the top aroma biscuits while the precursor biscuits develop a weaker aroma during baking. On sensory evaluation of the fresh biscuits, the precursor biscuits have a weaker aroma than the top aroma biscuits.

After 2 months of storage at room temperature a comparative sensory evaluation is performed. The top aroma biscuits are bland and have completely lost their original apple aroma. The precursor biscuits show a nice, balanced fruity apple aroma.

### Example 14

### Potato Flavor Precursor

### Preparation of Potato-Monoglyceride:

A mixture of monoglycerides derived from peanut oil is prepared by stirring 60 g peanut oil with 20 g glycerine, 1g of methionine, catalyzed by 100 mg HCl conc. in a roundbottom flask at 100 °C for 20 hours.

For the topnote, 0.05 g Methional, 1.0 g 2-Methyl-2-(methyldithio)propanal (FEMA3866), and 0.1g 3-Methylthiobutanal (FEMA3374) are mixed.

For the top aroma, 1 g topnote is mixed with 99 g peanut oil. The top aroma has a very strong potato aroma.

### Preparation of the precursors:

1 g of topnote is mixed with 20 g of Potato-Monoglyceride and heated for 10hours in a roundbottom flask with reflux cooler to form the precursor. The resulting cooled mixture has a weak potato aroma.

### Comparison of precursors and top aroma in potato flakes/mashed potato:

Potato flakes are flavored with 0.1 % (wt/wt) of the top aroma or the precursors and evaluated sensorically. The top aroma flakes (without cooking/heating) have a stronger potato aroma than the precursor.

After 2 months of storage at room temperature of the flavored flakes a comparative sensory evaluation is performed. The samples are cooked in water to mashed potato and compared. The top aroma sample is bland, has completely lost its original potato aroma and shows a starchy, watery aroma. The prescursor sample shows a nice, balanced potato aroma.

### Example 15

### Cheese Flavor Precursor

### Preparation of Cheese-Monoglyceride:

A mixture of 25 g Miglyol, 25g Tripropionin, 20 g glycerin, 25 g butyric acid and 100mg HCl conc. is stirred in a roundbottom flask with distillation head at 110 °C for 6 hours. The formed water is distilled off. The formed product is used without further purification.

For the topnote, 2g 2-Heptanone (FEMA2544), 0.5g 2-Nonanone FEMA2785), 1.0g 2-Octanone (FEMA2802) 2.0g 2-Undecanone (FEMA3093), 2.0g Diacetyl, and 0.5 g 2-Methyl-2-(methyldithio)propanal (FEMA3866) are mixed.

For the top aroma, 1g topnote are mixed with 99 g miglyol (vegetable oil). The top aroma has a very strong blue cheese aroma.

### Preparation of the precursors:

1 g of topnote is mixed with 20 g of Cheese-Monoglyceride and heated for 10hours in a roundbottom flask with reflux cooler to form the precursor. The resulting precursors have a weak blue cheese aroma.

### Comparison of precursors and top aroma In cracker biscuits:

A standard dough for crackers is prepared as follows:

| Ingredients: | % Weight |
|---|---|
| 1) Plain Flour (about 10% (wt/wt) protein) | 59.50 |
| 2) Monocalcium Phosphate | 0.60 |
| 3) Vegetable Shortening BM3030 (Woodlands Sunny Foods, Senoko, Singapore) | 7.14 |
| 4) Fine Milled Sugar | 2.23 |
| 5) Salt | 0.89 |
| 6) Glucose Syrup 42DE | 4.76 |
| 7) Sodium Bicarbonate | 0.60 |
| 8) Ammonium Bicarbonate | 2.23 |
| 9) Sodium Metabisulphite | 0.02 |
| 10a) top aroma | 0.2 |
| 10b) precursor | 0.2 |
| | add 100.00 (water) |

Ingredients 1 and 2 are sieved and dry-blended in a bowl. Ingredients 3 to 6 are placed in a mixing bowl, added to the above dry-blend and mixed at low speed for about 1 minute. The pre-dissolved solutions of sodium bicarbonate, ammonium carbonate and sodium metabisulphite are added at low speed. The top aroma or precursor is added. The remaining water is added and the cracker dough is mixed at medium speed at a temperature of 28-30°C for about 5 minutes until gluten development is achieved. The dough is left to rest at 28-30°C for 5 minutes. The dough is formed into a sheet and laminated twice until a final thickness of 1.5mm is obtained. The dough sheet is cut into cracker shapes and lightly dusted with fine salt The cracker dough is baked at 230oC for about 5 - 7 minutes. Baked crackers are brushed with warm oil (~8OoC).

During the baking process a strong blue cheese aroma is released from the baking oven for the top aroma crackers but not the precursor crackers. The resulting fresh biscuits with top aroma have a stronger flavor than the precursor crackers.

After 2 months storage at room temperature a comparative sensory evaluation is performed. The top aroma crackers are bland and have completely lost its original blue cheese aroma. The precursor crackers show a nice, balanced cheese aroma.

### Example 16

### Butter Flavor Precursor

### Preparation of Butter-Monoglyceride:

A mixture of monoglycerides, derived from butter fat is prepared by stirring 60 g butter fat with 20 g glycerine, catalyzed by 600mg of Lipozyme RM IM (Novozyms) in a roundbottom flask at 50°C for 24 hours.

For the topnote, 2.0 g Diacetyl and 0.001 g cis-4-Heptenal are mixed.

For the top aroma, 1g topnote are mixed with 99 g miglyol (vegetable oil). This flavor has a very strong butter aroma

### Preparation of the precursors:

1 g of top note is mixed with 20 g of Butter-Monoglyceride and heated for 10 hours in a round bottom flask with reflux cooler to form the precursor. The resulting cooled precursor mixture has a weak butter aroma.

### Comparison of precursors and top aroma in biscuits:

A standard dough for biscuit is prepared as in example 13.

0.2% of the top aroma or 0.1% of the precursors are homogenously mixed with the dough. Biscuits are formed and baked at an oven temperature of 200°C for 20min.

During the baking process a strong butter aroma from the baking oven is observed for the top aroma biscuits, but which is very weak for the precursor biscuits. The sensory evaluation shows a stronger aroma in the top aroma biscuits.

After 2 months storage at room temperature a sensory evaluation is performed. The top aroma sample is bland, has completely lost its original butter aroma, and has a dry texture and mouth feel.

The precursor biscuits show a nice, balanced butter aroma and smooth, lasting buttery mouth feel.

### Example 17

### Bread Flavor Precursor

### Preparation of bread-Monoglycerides:

A mixture of monoglycerides derived from butter fat is prepared by stirring 60 g butter fat, 2 g proline with 20 g glycerine, catalyzed by 600mg of Lipozyme RM IM (Novozyms) in a roundbottom flask at 50°C for 24 hours.

For the topnote, 2 g Acetylpyrazine, 1g 2-Acetylpyridine, 0.5 g 2-Acetylthiazole, and 2 g Butyraldehyde are mixed.

For the top aroma, 1g topnote are mixed with 99 g peanut oil. This top aroma has a very strong roasted aroma.

### Preparation of the precursors:

1 g of topnote is mixed with 20 g of bread-Monoglycerides and heated for 10 hours in a round bottom flask with reflux cooler to form the precursor. The resulting cooled precursors have a weak roasted aroma.

### Comparison of precursors and top aroma in bread buns:

A standard dough for bread bun is prepared as follows:

| **Ingredients** | **% Weight** |
|---|---|
| 1) Bread Flour | 53.81 |
| 2) Vegetable Shortening BM 3030 (Woodlands Sunny Foods, Senoko, Singapore) | 3.77 |
| 3) Castor Sugar | 5.92 |
| 4) Skim Milk Powder | 1.13 |
| 5) Salt | 1.21 |
| 6) Instant Yeast | 0.81 |
| 7a) top aroma | 0.2 |
| 7b) precursor | 0.2 |
| | add 100.00 (water) |

The instant yeast is hydrated using part of the water. At low speed, ingredients 1 to 4 and the remaining water are mixed in a mixing bowl. Salt is added. The dough is mixed at medium speed for about 10 minutes to form a soft and shiny dough with a temperature of about 24 to about 27oC.

The dough is formed into 65 g buns (determined with a scale, moulded, and the pieces are panned. The buns are set in a proofer for about 60 minutes at relative humidity 85 to 90% and temperature 43 to 46°C until full proof. Then the buns are baked at 230°C for about 7 minutes.

0.2% (wt/wt) of the top aroma or precursors are homogenously mixed with the dough. Buns are formed and baked at an oven temperature of 200°C for 20min.

During the baking process a strong roasted aroma from the baking oven is observed for the top aroma buns which is much weaker for the precursor buns. When evaluated sensorically directly after baking, the top aroma buns have a stronger roasted bread aroma than the precursor buns.

After 2 weeks storage at room temperature a sensory evaluation is performed. The top aroma version is bland and has lost its original roast aroma and has a starchy, dry "retrograded" aroma.

The precursor buns show a nice, balanced roasted aroma.

### Example 18

### Hazelnut Flavor Precursor

### Preparation of hazelnut-monoglycerides

A mixture of 50 g Miglyol, 20 g glycerin and 100mg HCl conc. is stirred in a roundbottom flask with distillation head at 110 °C for 6 hours. The formed water is distilled off. The formed product is used without further purification.

For the topnote, 1.0 g 2-Acetyl-3-ethylpyrazine, 1g 2-Acetyl-3-methylpyrazine, 0.5g 5-Methyl-hept-2-en-4-one (FEMA3761), 1.0 g Isovaleraldehyde are mixed.

For the top aroma, 1g topnote are mixed with 99 g peanut oil. The top aroma has a very strong roasted nutty aroma.

### Preparation of the precursors:

1 g of topnote is mixed with 20 g of Hazelnut-Monoglyceride and heated for 10 hours in a roundbottom flask with reflux cooler to form the precursor. The resulting cooled mixture has a weak roasted, nutty aroma.

### Comparison of precursors and top aroma in chocolate:

A chocolate mass is prepared as follows:

| **Ingredients:** | **% Weight** |
|---|---|
| 1) Compound dark super-coat chocolate (Barry Callebaut Asia Pacific, Singapore) | 92.59 |
| 2) Palm Kernel Stearin (Barry Callebaut Asia Pacific, Singapore) | 7.41 |
| 3a) top aroma, or alternatively precursor) | 0.2 |
| | add 100.00 (water) |

Compound dark super-coat chocolate and stearin are filled into a beaker and melted and stirred over a pot of warm water (about 35 to 38°C) until the chocolate mass is smooth and uniform (free of lumps). The top aroma or precursor are added. The resulting mass has a roasted nutty hazelnut chocolate aroma.

The chocolate mass is coated onto the nutritional bar product.

Nutritional bars are prepared as follows:

| **Ingredients Binder syrup:** | **% Weight** |
|---|---|
| 1) Isomalt (Palatinit Asia Pacific, Singapore) | 35.81 |
| 2) Sugar | 18.00 |
| 3) Dextrose | 4.40 |
| 4) Glucose Syrup 42 DE | 15.00 |
| 5) Condensed Milk | 2.50 |
| 6) Glycerine 85%, E-009 | 3.00 |
| 7) Salt | 0.20 |
| 8) Vegetable Shortening BM 3030 (Woodlands Sunny Foods, Senoko, Singapore) | 10.00 |
| 9) Lecithin, Topcithin™ N50 (Degussa texturants systems, Singapore) | 0.08 |
| | add 100.00 (water) |

| **Ingredients nutritional bar:** | **% Weight** |
|---|---|
| 1) Corn Flakes (Crushed) | 8.00 |
| 2) Rice Krispies | 10.71 |
| 3) Rolled Oats | 24.11 |
| 4) Binder Syrup (as above) | 57.18 |
| | add 100.00 (water) |

Ingredients 1 to 7 and water are mixed in a mixing bowl. The vegetable shortening is melted and stirred into the lecithin, then added to the mixing bowl and mixed at low speed for 1 minute or until homogenous. The mixture is further mixed at medium speed for 2 minutes until the mixture is almost opaque. The mixture is transfered to a pot and heated to 120°C (84.8°Brix) upon continuous stirring.

The cooked syrup is poured on top of the dry cereal mix and stirred gently ensuring that the binding syrup covers the cereals thoroughly. The syrup-cereal mix is emptied into a mould which is levelled out with a scrapwer. The syrup-cereal mixture is packed tightly, and rolled out with a rolling pin. The syrup-cereal mixture is cooled and cut into bars measuring 10 x 3.0 x 1.5cm. The bars are wrapped and packed.

The nutritional bars are coated with the chocolate mass and evaluated sensorically. The top aroma bar shows a stronger nutty aroma than the precursor bar.

After 2 months storage at room temperature a second sensory evaluation of the bars is performed. The top aroma bar has a bland neutral chocolate cover and has completely lost its original hazelnut aroma.

The precursor bar shows a nice, balanced hazelnut-chocolate aroma.

### Example 29

### Tomato Flavor Precursor

### Preparation of Tomato-Monoglyceride:

A mixture of 25 g Tripropionin, 25 g Miglyol, 20 g glycerin, 25 g 3-Methylbutyric acid and 100 mg HCl conz. is stirred in a roundbottom flask with distillation head at 110 °C for 6 hours. The formed water is distilled off. The formed product is used without further purification.

For the topnote, 0.05 g Methional, 1.0 g 2,4-Hexadienal (FEMA3429), 0.5 g cis-3-Hexenal, and 0.1g Isobutyl thiazole (FEMA3134) are mixed.

For the top aroma, 1g topnote are mixed with 99 g peanut oil. The top aroma has a very strong tomato aroma

### Preparation of the precursors:

1 g of topnote is mixed with 20 g of tomato-monoglyceride and heated for 4 hours in a round bottom flask with reflux cooler to form the precursor. The resulting cooled precursor mixture has a weak tomato aroma.

### Comparison of precursors and top aroma in tomato soup:

A tomato cream soup is prepared as follows:

| **Ingredients Instant cream soup base** | **% (wt/wt)** |
|---|---|
| Salt | 12.00 |
| Sugar | 8.50 |
| monosodiumglutamate | 1.50 |
| I+G (inosine monophosphate.guanosine monophosphate; nucleotides) | 0.10 |
| Palm fat | 5.00 |
| Chicken Roast Flavor 605-00015-53 (Givaudan, Cincinnati, USA) | 5.00 |
| Onion powder | 1.00 |
| Garlic powder | 0.80 |
| Novation 5600 (ex. National Starch) | 42.00 |
| Non-dairy creamer | 15.55 |
| Skimmed milk powder | 7.95 |
| Butter Flavor CF 8.08.07.L (Givaudan, Cincinnati, USA) | 0.15 |
| Vegetable Stock 473869 (Givaudan, Cincinnati, USA) | 0.50 |
| | Add 100.00 (water) |

Palm fat is melted and plated into salt, sugar, monosodiumglutamate and I+G. The rest of the ingredients is added and mixed until homogenous. The mixture is sieved and packed.

| **Ingredients tomato soup powder** | **% (wt/wt)** |
|---|---|
| Instant Cream Soup Base | 76.80 |
| Tomato powder (Spreda 707) | 20.00 |
| Citric acid | 0.40 |
| Lycopene 10 % ws (Roche) | 0.40 |
| Parsley flakes | 0.40 |
| Ultra Rhodigel™ (Rhodia) | 1.00 |
| top aroma or precursor | 0.1% (wt/wt)? |
| | Add 100.00 (water) |

To prepare the tomato soup, 6.5 g of the tomato soup powder is used and added to 100 ml hot boiling water. The soup is stirred and served in cups for sensorical evaluation,

The top aroma soup has a strong, pungent tomato aroma stronger than the precursor soup.

After 3 months storage at room temperature of the soup powder samples, a second evaluation is performed with prepared heated soup samples. The top aroma soup is bland and has completely lost its original tomato aroma. The precursor soup shows a nice, balanced tomato aroma.

## Claims

1. Method of providing a flavored food product, wherein at least one flavor precursor of formula I 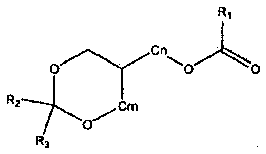 wherein n and m are selected from 0 and 1, and if n is 1 then m is 0, and if n is 0 then m is 1;
wherein R1 is selected from the group consisting of
a straight-chain C7 to C17 alkyl, a straight-chain C7 to C17 alkenyl, a branched C7 to C17 alkyl, a branched C7 to C17 alkenyl,
a straight-chain C7 to C17 monoalkenyl, a branched C7 to C17 monoalkenyl,
a straight-chain C7 to C17 alkadienyl, a branched C7 to C17 alkadienyl,
a straight-chain C7 to C17 alkatrienyl, a branched C7 to C17 alkatrienyl,
a straight-chain C9 to C17 alkatetraenyl, a branched C9 to C17 alkatetraenyl,
and a straight-chain C11 to C17 alkapentaenyl, a branched C11 to C17 alkapentaenyl;
wherein
R2 is selected from the group consisting of H, a C1 to C15 alkyl, a C1 to C15 oxoalkyl, a C1 to C15 hydroxyalkyl,
C2 to C15 alkenyl, a C2 to C15 oxoalkenyl, and a C2 to C15 hydroxyalkenyl, and
R3 is selected from the group consisting of C1 to C15 straight-chain alkyl comprising one or two substituents independently selected from 0, OH, N, NH, phenyl, furanyl, C9 aryl, C10 aryl, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15, C1 to C15 straight-chain alkyl, comprising one or two atoms independently selected from O,S or N within the alkyl chain,
C3 to C15 singly, doubly or multiply branched alkyl optionally substituted with one or two residues independently selected from 0, OH, N, NH, SH, and SR4, wherein R4 is a straight-chain or branched C1 to C5 alkyl residue optionally selected from methyl, ethyl, propyl, isopropyl, butyl, isobutyl, pentyl and isopentyl, and the total number of carbon atoms in R3 and R4 is up to 15,
C3 to C15 singly, doubly or multiply branched alkyl comprising one or two atoms independently selected from O,S or N within the alkyl chain, C2 to C15 straight-chain alkenyl, C3 to C15 straight-chain alkadienyl,
C2 to C15 straight-chain alkenyl optionally substitued with one or more residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C2 to C15 straight-chain alkenyl, optionally comprising one or more atoms independently selected from O.S and N within the alkenyl chain,
C3 to C15 branched alkenyl, optionally substituted with one or two alkyl groups,
C2 to C15 branched alkenyl comprising one or two alkyl groups optionally substituted with one or two residues independently selected from O, OH, N, NH, phenyl, furanyl, C9 aryl, C10 aryl, SH, SR4 wherein R4 is a straight-chain or branched C1 to C5 alkenyl residue optionally selected from ethenyl, propenyl, butenyl, and pentenyl, and the total number of carbon atoms in R3 and R4 is up to 15,
C3 to C15 branched alkenyl composing one or two alkyl groups and one or more atoms independently selected from O,S and N within the alkenyl chain.
C5 to 15 singly, doubly, or multiply branched alkadienyl, optionally substituted with one or two alkyl groups,
C4 to C15 singly, doubly, or multiply branched alkedienyl comprising one or more atoms independently selected from O,S and N within the alkenyl chain;
a 5- or 6-membered carbon ring residue comprising up to two heteroatoms independently selected from O, S, and N, optionally subsituted with one or more residues independently selected from O, OH, alkoxy, alkyl, alkenyl;
a C1 to C6 alkyl or alkenyl substituted with a 5- or 6-membered carbon ring residue comprising up to two heteroatoms independently selected from O, S, and N, optionally subsituted with one or more residues independently selected from O, OH, alkoxy, alkyl, alkenyl; or wherein
R2-C-R3 form a ring residue selected from oxacyclopentane optionally substituted with a residue selected from methyl, dimethyl, ethyl, hydroxy, and methoxy, oxacyclopentane optionally substituted with a residue selected from methyl, dimethyl, ethyl, hydroxy, and methoxy, hydroxycyclopentene, hydroxyalkylcyclopentene, hydroxymethylcyclopentene, hydroxydimethylcyclopentene, ethylhydroxycyclopentene, and ethylhydroxymethylcyclopentene, thiacyclopentane, alkylthiacyclopentane, and alkyl-3-thia-cyclopentane, oxa-cyclopentene, 2-oxacyclopentene, 3-oxacyclopentene, alkyloxacylcopentene, alkyl-3-oxacyclopentene, methyl-3-oxa-cyclopentene, alkyl-3-oxacyclopentene, dimethyl-3-oxacyclopentene, ethyl-3-oxacyclopentene, ethylmethyl-3-oxacyclopentene, hydroxyalkyl-3-oxacyclopentene, trydroxymethyl-3-oxacyclopentene, hydroxydimethyl-3-oxacyclopentene, hydroxyethyl-3-oxacyclopentene, ethythydroxymethyl-3-oxacyclopentene, oxacyclopentane, 3-oxacyclopentane, alkyl-3-oxacyclopentane, methyl-3-oxa-cyclopentane, dimethyl-3-oxacyclopentane, ethyl-3-oxacyclopentane, ethylmethyl alkyl-3-oxacyclopentane, alkylcyclopentane, alkenylcyclopentene, alkylalkenylcyclopentene, methylcylopentene, dimethylcyclopentene, ethylcyclopentene, propylmethylcyclopentene, butylmethylcyclopentene, butenylmethylcyclopentene, pentylmethylcyclopentene, pentenylmethylcyclopentene, alkylcarboxy-alkenyl-cyclopentane, alkylcarboxy-alkyl-cyclopentane, methylcarboxy-alkenyl-cyclopentane, methylcarboxy-alkyl-cyclopentane, alkylcarboxy-pentenyl-cyclopentane, alkylcarboxy-pentyl-cyclopentane, methylcarboxy-alkenyl-cyclopentane, and methylcarboxy-alkyl-cyclopentane, and wherein the alkyl is selected from methyl, ethyl, propyl, butyl, pentyl, pentenyl, hexyl, heptyl, and octyl, oxo-alkylcyclohexene, oxo-methylcyclohexene, oxo-trimethylcyclohexene, oxo-ethylcyclohexene, oxo-ethylmethylcyclohexene, oxo-propylcyclohexene, oxo-propylmethylcyclohexene, oxo-isopropocyclohexene, oxo-isopropylmethylcyclohexene, oxo-thiopropylcyclohexene, oxo-thiopropylmethylcyclohexene, alkylcyclohexene, methylcyclohexene, trimethylcyclohexene, ethylcyclohexene, ethylmethylcyclohexene, propylcyclohexene, propylmethylcyclohexene, isopropylcyclohexene,
isopropylmethylcyclohexene, thiopropylcyclohexene, thiopropylmethylcyclohexene, alkylcyclohexane, methylcyclohexane, trimethylcyclohexane, ethylcyclohexane, ethylmethylcyclohexane, propylcyclohexane, propylmethylcyclohexane, isopropylcyclohexane, isopropylmethylcyclohexane, thiopropylcyclohexane, thiopropylmethylcyclohexane;
and wherein R2 and R3 together have a total number of carbon atoms of up to 15, and R2-C-R3 has a total number of carbon atoms of up to 16,
is admixed to a food product in a sufficient concentration to release a flavor of noticeable aroma upon consumption and/or heating of the food product.

2. Method according to claim 1 wherein said flavor is selected from the group consisting of (+)-8,9-DEHYDRONOOTKATONE; (1R-CIS AND TRANS)-2-(1-ACETYLTHIO-1-METHYL)ETHYL-5-METHYLCYCLOHEXANONE; (E)-2-(2-OCTENYL)CYCLOPENTANONE; (E)-5-ISOPROPOXY-2-DECENAL; (E,E)-3,5-OCTADIEN-2-ONE; (METHYLTHIO)ACETONE; (RAC)-3-ACETYLOXY-5-METHYL-2-HEXANONE; 1-(2,5,5-TRIMETHYL-CYCLOPENT-1-EN-1-YL)-3-METHYL-2-BUTEN-1-ONE; 1-(3,5,5,-TRIMETHYL-1-CYCLOHEXENYL)-3-METHYL-2-BUTEN-1-ONE; 1(5-METHYLFURYL-2)-PROPANE-1,2-DIONE; 1-(METHYLTHIO)-2-BUTANONE; 1-(METHYLTHIO)-3-PENTANONE; 1-(METHYLTHIO)-OCTAN-3-ONE; 1-(P-METHOXYPHENYL)-2-PROPANONE; 1,4-DODEC-6-ENOLACTONE; 1,5,5-TRIMETHYL-8-ETHYL-G-OXA-BICYCLO(4,3,0)NON-6-EN-3-ONE; 1,5-[Z]-OCTADIEN-3-ONE; 10-UNDECENAL; 12-METHYL-TRIDECANAL; 14-METHYL-PENTADECANAL; 1-HYDROXY-2-BUTANONE; 1-MERCAPTO-2-PROPANONE; 1-MERCAPTO-3-PENTANONE; 1-PENTEN-3-ONE; 2-(3,3-DIMETHYLCYCLOHEXYLIDEN)-ETHANAL; 2-(METHYLTHIO)METHYL-2-BUTENAL; 2-(METHYLTHIOMETHYL)-3-PHENYLPROPENAL; 2,10-UNDECADIENAL; 2,3-DIHYDRO-2,3,3-TRIMETHYL-1H-INDEN-1-ONE; 2,4-DIMETHYL-HEPT-4-ENE-3-ONE; 2,4-PENTADIENAL; 2,5-DIETHYL-4-HYDROXY-5-METHYL-3(2H)-FURANONE; 2,5-DIMETHYL-3(2H)-FURANONE; 2,5-DIMETHYL-4-HYDROXY-6-HEPTANONE; 2,5-DIMETHYL-TETRAHYDRO-3-FURANONE; 2,6-DIMETHYL-3-HYDROXY-4-PYRONE; 2,6-DIMETHYL-4-HEPTANONE; 2,6-DIMETHYLBICYCLO[3.2.1]OCT-2-EN-7-ONE; 2-AMINO-ACETOPHENONE; 2-BUTYL-2-BUTENAL; 2-ETHYL-4-METHYL-PENT-2-ENAL; 2-HEPTEN-4-ONE; 2-HEPTYL-BUTYROLACTONE; 2-HEXENAL; 2-HYDROXY-1-(4-HYDROXY-3-METHOXYPHENYL)-ETHANONE; 2-HYDROXY-2-CYCLOHEXEN-1-ONE; 2-HYDROXY-3,4,5-TRIMETHYL-2-CYCLOPENTEN-1-ONE; 2-HYDROXY-3,5,5-TRIMETHYL-2-CYCLOHEXENONE; 2-HYDROXYACETOPHENONE; 2-ISOPROPYL-5-CAPROLACTONE; 2-ISOPROPYL-DIOXOLAN-4-ONE; 2-METHOXY-3,4,5-TRIMETHYL-2-CYCLOPENTEN-1-ONE; 2-METHYL-2-OCTENAL; 2-METHYL-3-(P-METHUYLPHENYL)PROPANAL; 2-METHYL-8-PHENYL-OCT-2-ENE-6-ONE; 2-METHYLCYCLOHEXANONE; 2-METHYLHEPTAN-3-ONE; 2-METHYLHEXAN-3-ONE; 2-METHYLNONANAL; 2-METHYL-SPIRO(5,5)-UNDECAN-1-ONE; 2-OCTEN-4-ONE; 2-PHENYL-3-(2-FURYL)PROP-2-ENAL; 2-PHENYL-4-PENTENAL; 2-TETRADECENAL; 2-TRANS-4-CIS-1-CIS-TRIDECATRIENAL; 3-((2-METHYL-3-FURYL)THIO)-4-HEPTANONE; 3(2)-HYDROXY-4-METHYL-HEXAN-2(3)-ONE; 3-(3-METHOXY-PHENYL)-PROPAN-2-ONE; 3-(5-METHYL-2-FURYL)BUTANAL; 3(Z),6(Z),9(Z)-DODECATRIENAL; 3(2),6(Z)-DODECADIENAL; 3,3-DIETHOXY-2-BUTANONE; 3,3-DIMETHYLCYCLOHEXANONE; 3,4,5,6-TETRAHYDRO PSEUDO IONONE; 3,4-DIMETHYL-BUTYROLACTONE; 3,5-DIMETHYL-4-THIA-HEPTAN-2,6-DIONE; 3,6,DIMETHYL-5,6-DIHYDRO-2(4H)BENZOFURANONE; 3,6-DIMETHYL-2-HYDROXY-2-CYCLOHEXEN-1-ONE; 3,7-DIMETHYL-6-OCTEN-2-ONE; 3A,4,5,7A-TETRAHYDRO-3,6-DIMETHYL-2(3H)BENZOFURANONE; 3-ACETYLOXY-2-OCTANONE; 3-ACETYLOXY-2-PENTANONE; 3-ACETYLSULFANYL-2-ETHYLHEXANAL; 3-DECANONE; 3-ETHOXY HEXANAL; 3-ETHYL-2-HYDROXY-4-METHYLCYCLOPENT-2-EN-1-ONE; 3-ETHYL-4,5,5-TRIMETHYL-2-(5H)-FURANONE; 3-HEPTYLDIHYDRO-5-METHYL-2(3H)-FURANONE; 3-HEXANONE; 3-HYDROXY-2-PENTANONE; 3-HYDROXY-4,5,5-TRIMETHYL-2-(5H)-FURANONE; 3-HYDROXY-4-METHYL-5-PENTYL-2(5H)-FURANONE; 3-HYDROXY-4-METHYL-ALPHA-PYRONE; 2-ACETYL4,5-DIMETHYL-THIAZOLE; 2-METHYL-3-ACETYLPYRIDINE; 1-ETHYL-2-ACETYL-PYRROLE; 1-METHYL-2-ACETYLPYRROLE; 2,4-DIMETHYL-5-ACETYL THIAZOLE; 3-ACETYL-2,5-DIMETHYLFURAN; 1,4-DIMETHYL-4-ACETYL-LCYCLOHEXENE; 4-ACETYL-6-T-BUTYL-1,1-DIMETHYLINDAN; 4-ACETYL-2-METHYLPYRIMIDINE; ACETYL-2-PYRAZINE_{;} ACETYL-2-FURAN; ACETYL-2-PYRROLE; ACETYL-2-PYRIDINE; ACETYL-2-DIMETHYL-PYRAZINE; ACETYL-2-THIAZOLE; ACETYL-3 PYRIDIN; AGETYL-3-DIMETHYL-2.5-THIOPHENE; ACETYL-2 METHYL-5 FURAN; ACETYL-2-THIAZOLINE-2; ACETYL-2 METHYL-3 PYRAZINE; PROPIONYL PYRAZINE; PROPIONYL-2-METHYL-3-FURANE; 2-PROPIONYLTHIAZOLE; 2-PROPIONYLPYRROLE; 3-HYDROXY-4-PHENYL-2-BUTANONE; 3-HYDROXY-5-HEXYL4-METHYL-2(5H)-FURANONE; 3-HYDROXY-6-HYDROXYMETHYL-PYRAN-2-ONE; 3-HYDROXYMETHYL-2-OCTANONE; 3-ISOAMYL-2,4-PENTANDIONE; 3-MERCAPTO-2-METHYLPENTANAL; 3-MERCAPTO-2-OCTANONE; 3-MERCAPTO-2-PENTANONE; 3-MERCAPTO-4-PHENYL-2-BUTANONE; 3-MERCAPTO-5-METHYL-HEXAN-2-ONE; 3-METHYL NOM-2(E)-EN-4ONE; 3-METHYL-1-CYCLOPENATADECANONE; 3-METHYL-2-CYCLOHEXEN-1-ONE; 3-METHYL-2-CYCLOPENTEN-1-ONE; 3-METHYL-2-HEPTEN-0,5-DIONE; 3-METHY4-3-(3-METHYL-BUT-2-ENYL)-DIHYDRO-FURAN-2-ONE; 3-METHYL4-PHENYL-3-BUTENE-2-ONE; 3-METHYL-5-PROPYL-2-CYCLOHEXEN-1-ONE; 3-METHYLCYCLOHEXANONE; 3-METHYLENE-2-OCTANONE; 3-METHYL-GAMMA-DECALACTONE; 3-METHYLPENTANAL; 3-METHYLTHIOHEXANAL; 3-NONANONE; 3-NONEN-2-ONE; 3-PENTANONE; 3-PENTEN-2-ONE; 3-PENTYL-4,5,5-TRIMETHYL-2-(5H)-FURANONE; 3-PHENYL-4-PENTENAL; 3-PROPYL-2-CYCLOPENTEN-1-ONE; 3-PROPYLIDEN-HEPTAN-2-ONE; 4-(2,6,6-TRIMETHYL-2-CYCLOHEXEN-1-YL)BUTAN-2-ONE; 4-(2,6,6-TRIMETHYL-CYCLOHEXA-1,3-DIENYL)-BUT-3-EN-2-ONE; 4-(FURAN-2-YL)-BUTAN-2-ONE; 4-(METHYLTHIO)BUTANAL; 4-(P-TOLYL)-2-BUTANONE; 4,4-DIMETHOXY-3-HEXANONE; 4.4-DIMETHYL-1,3-OXATHIANE-2-ONE; 4,8-DIMETHYL-3,7-NONADIEN-2-ONE; 4-((2-FURANMETHYL)THIO)-2-PENTANONE; 4-ACETOXY-HEX-4-EN-3-ONE; 4-EPOXY-1,1,3-TRIMETHYLCYCLOHEX-5-EN-2-ONE; 4-ETHYL-1,3-OXATHIAN-2-ONE; 4-ETHYL-5-METHYL-1,3-OXATHIAN-2-ONE; 4-HEPTYL-3-METHYLBUTYROLACTONE; 4-HYDROXY-2,2,5-TRIMETHYL-3(2H)-FURANONE; 4-HYDROXY-3,5,5 TRIMETHYL-CYCLOHEX-5,5-EN-1-ONE; 4-HYDROXY-4-METHYL-5-HEXENOIC-ACID LACTONE; 4-HYDROXY-4-METHYL-CIS-7-DECANOICACID-4-LACTONE; 4-MERCAPTO-4-METHYL-PENTAN-2-ONE; 4-METHYL-2-PENTENAL; 4-METHYL-3-PENTEN-2-ONE; 4-METHYL-4-FURFURYLTHIO-2-PENTANONE; 4-METHYL-5-PENTYL-DIHYDRO-2(3H)-FURANONE; 4-METHYLCYCLOHEXANONE; 4-METHYLTHIO-2-PENTANONE; 4-OXO-BETA DAMASCONE; 4-PHENYL-3,5-DITHIA-2-HEXANONE; 5 ALPHA-ANDROST-16-EN-3-ONE; 5.5-DIMETHYL-2(5H)-FURANONE; 5.5-DIMETHYL-2-METHOXY-CYCLOHEX-2-ENONE; 5-ETHYL-2-HYDROXY-3,4-DIMETHYL-2-CYCLOPENTEN-1-ONE; 5-ETHYL-2-METHOXY-3,4-DIMETHYL-2-CYCLOPENTEN-1-ONE; 5-ETHYLDIHYDRO-5-METHYL-2(3H)-FURANONE; 5-HYDROXY-4-OCTANONE; 5-HYDROXY-8-UNDECENOIC ACID D-LACTONE; 5-METHYL-2-PROPYL-[1,3]-DIOXOLAN-4-ONE; 5-METHYL-3-(3-METHYL-BUT-2-ENYL)-DIHYDRO-FURAN-2-ONE; 5-METHYL-3-HEXEN-2-ONE; 5-METHYL-4-HEXENE-2,3-DIONE; 5-METHYL-5-HEXEN-2-ONE; 5-PENTYL-ALPHA-PYRONE; 5-P-TOLUYL-2(3H)-FURANONE; 6-(1-PENTENYL)-2H-PYRAN-2-ONE; 6-HEPT-1-ENYL-5,6-DIHYDRO-PYRAN-2-ONE; 6-HYDROXY-3,7-DIMETHYLOCTANOIC ACID LACTONE; 6-METHYL-3-HEPTEN-2-ONE; 6-METHYL-NON-5-EN-4-ONE; 6-METHYL-OCTANAL; 6-PROPYL-[1,3]OXATHIAN-2-ONE; 6-UNDECANONE; 7-METHYL-2,3,4,4A,5,6-HEXAHYDRO-2-NAPHTHALENONE; 7-METHYL-6-OCTEN-2-ONE; 7-OCTENAL; 8-(2,2-DIMETHYLCYCLOPROPYL)-OCTA-3,5-DIEN-2-ONE; 8A-METHYL-3,4,4A,5,8,8A-HEXAHYDRO-1(2H)-NAPHTHALENONE 8-METHYL NONANAL; 8-METHYL-TRIDEC-7-EN-6-ONE; 8-NONEN-2-ONE; 8-NONENAL; 9-DECENAL; ACETONE; ACETOPHENONE; AR-TURMERONE, BENZOPHENONE; BENZOYLACETONE; BENZYL DIPROPYL KETONE; BENZYL-ACETONE; BENZYLIDENE ACETONE; BETA-IRONE; BICYCLO-IRONE; BICYCLONE; BICYCLONONALACTONE; BUTYRO-1,4-LACTONE; CARYOPHYLLEN-12-AL; CIS,CIS-4,7-DECADIEN-1-AL; CIS-2-NONENAL; CIS-3-HEXENAL; CIS-3-NONENAL; CIS-4-HEPTEN-2-ONE; CIS-4-HEXENAL; CIS-4-NONENAL; CIS-5-NONENAL; CIS-5-OCTENAL; CIS-7-NONENAL; CITRONELLENE LACTONE; CLONAL; COGNAC LACTONE; CYCLOBUTONE; CYCLOHEPTADECA-9-EN-1-ONE; CYCLONEXANONE; CYCLOIONONE; CYCLOPENTANONE; CYCLOHEPTADECANONE; DAMASCENONE; DECANAL; DECENYL CYCLOPENTANONE; DIBENZYL KETONE; DIBUTYL-4,4 GAMMA BUTYROLACTONE, DIHYDRO FILBERTONE; DIHYDRO-5-METHYL-5-PROPYL-2(3H)-FURANONE; DIHYDROCARVONE; DIHYDRO-DEHYDRO-GAMMA-IONONE; DIHYDROJASMONE LACTONE; DIHYDROLAVENDELLACTONE; DIMETHYL HYDROQUINONE; DIMETHYL METHOXY FURANONE; DIMETHYL-2,4 ACETOPHENONE; DIMETHYL-2,6 OCTANAL; DIMETHYL-3,6-BENZO-2(3H)-FURANONE; DODEC-2-EN-1,5-LACTONE; DODEC-2-EN-4-ONE; DODECANAL; E-DECALACTONE; E-DODECALACTONE; ETHYL-2-HEPTEN-2-AL; ETHYL-3 CYCLOPENTANDIONE; FARNESYL ACETONE; FURFURYLIDENEACETONE; GAMMA DAMASCONE; GAMMA IONONE; GERANYL ACETONE; GINGERONE; HEPT-3-EN-2-ONE; HEPTANAL; HEXANAL; HEXEN 5 ONE; HEXENYL METHYL KETONE; EXYLIDENE CYCLOPENTANONE; HOMOCORYLONE; HYDROBICYCLONE; HYDROXY-3(2)-HEPTAN-2(3)-ONE; HYDROXY-5-DECADIENOIC ACID-2.4 LACTONE; ISO JASMONE; ISOLONGIFOLENE-KETONE; ISOPHORONE; JASMIN LACTONE; LAVENDELLACTONE; MASSOIA LACTONE; MELONAL. DIMETHYL HEPTENAL; MENTHONE; METHIONAL; METHOXY-P-PHENYL-PENTEN-3-ONE, ETHONE; METHYL 2 OCTANAL; METHYL 2-PENTENE-1-AL; METHYL THIO-METHYLTHIOMETHYL-2-PENTENAL; METHYL-2 UNDECANAL; METHYL-2-BUTANAL; METHYL-2-PENT-2-ENAL; METHYL-2-TETRAHYDRO-FURANONE; METHYL-4-HYDROXY-3/2H/-FURANONE; METHYL-5-HEPT-2-EN-4-ONE; METHYL-6-HEPTA-3,5-DIENONE: METHYLTHIO-3-BUTANAL; METHYLTHIO-4-BUTAN-2-ONE; MINTLACTONE; MYRTENAL; NEOFOLIONE; NEOHESPERIDINE DIHYDROCHALCONE; NONANAL; NOOTKATONE; OCTAN-2-ONE; OCTANAL; PENNYROYAL OIL 84%PULEGONE; PENTADECANONE; PENTANAL; PENTYL 2-FURYL KETONE; PHENYL-2-BUTEN-2-AL; PHENYL-3-PROPANAL; PIPERITENONE; PIPERONYL ACETONE; P-MENTH-1-ENAL; P-MENTH-1-ENE-9-AL; P-MENTHAN-2-ONE; PRENAL; PROPANAL; PROPYL-ISO PARA ACETOPHENONE; RAC-3-HYDROXY-5-METRYL-2-(5H)-FURANONE; RASPBERRY KETONE; SAFRANAL; SAGE OIL 27% THUJONE; SOLANONE; SPIRO-(6,5)-DODECAN-1-ONE; TETRAHYDROIRONE; TETRAHYDRONOOTKATONE; TRANS-4-HEXENAL; TRANS-5-NONENAL; TRANS-6-DECEN-1-AL; TRANS-6-NONENAL; TRANS-7-METHYL-3-OCTEN-2-ONE; TRANS-7-NONENAL; TRIDECAN-2-ONE; TRIMETHYL-3,5,5-HEXANAL; TRITHIOACETONE; UNDECANAL; VANILLYLIDENE ACETONE; and VERBENONE.

3. A method according to any one of claims 1 to 2 wherein R1 is an alkyl selected from the group consisting of C7, C8, C9, C10, C11, C13, C15, and C17 alkyl.

4. A method according to any one of claims 1 to 2 wherein R1 is an alkenyl selected from the group consisting of C7, C8, C9, C10, C11, C13, C15, and C17 alkenyl, a C17-8en(oleic acid residue) alkenyl a C17-8,11 alka-dienyl (linoleic acid residue), and a C17-8,11,14-trienyl (linolenic acid residue).

5. A method according to any one of claims 1 to 2 wherein R2 is selected from the group consisting of H, methyl and ethyl.

6. A method according to any one of claims 1 to 2 wherein R3 is selected from the group consisting of a C1 to C8 straight-chain alkyl, a C1 to C8 branched alkyl comprising up to two alkyl groups, wherein the alkyl residue may contain one or more further alkyl residues, a C1 to C8 straight-chain alkyl comprising one or two substituents selected from O, and SR4, wherein R4 is an alkyl residue selected from methyl, and ethyl, a C2 to C8 straight-chain alkenyl, and a ring selected from a 5 membered and a 6 membered ring, comprising up to two N heteroatoms, wherein the ring may be further substituted with one or more alkyl groups selected from methyl, ethyl, propyl and isopropyl.

7. A method according to any one of claims 1 to 2 wherein R1 is an alkyl selected from the group consisting of C7, C9, C11, C13, C15 and C17 alkyl, R2 is selected from the group consisting of H, methyl and ethyl, and R3 is selected from the group consisting of a C1 to C8 straight-chain alkyl, a C1 to C8 branched alkyl comprising one or two alkyl groups, wherein the alkyl residue may contain one or more further alkyl residues, a C1 to C8 straight-chain alkyl comprising one or two substituents selected from O, and SR4, wherein R4 is an alkyl residue selected from methyl, and ethyl, a C2 to C8 straight-chain alkenyl, and a ring selected from a 5 membered and 6 membered ring comprising up to two N heteroatoms, wherein the ring may be further substituted with one or more alkyl groups selected from methyl, ethyl, propyl and isopropyl.

8. A flavor composition comprising at least one flavor precursor as defined in any one of claims 2 to 7.

9. A food product comprising at least one flavor precursor as defined in any one of claims 1 to 7.

10. A flavor precursor compound as defined in any one of claims 1 to 9 wherein m=1 and n=0.

11. A flavor precursor compound as defined in any one of claims 2 to 9 wherein m=0 and n=1.

12. A process of producing the flavor compounds precursor of claim 10 or claim 11 by reacting at least one flavor compound comprising one or more carbonyl group with at least one monoglyceride in an acid catalyzed reaction.

## Patentansprüche

1. Verfahren zur Bereitstellung eines geschmacksstoffhaltigen Lebensmittelprodukts, bei dem man mindestens einen Geschmacksstoffvorläufer der Formel I 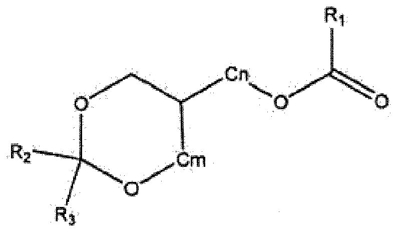 worin n und m aus 0 und 1 ausgewählt sind und m für 0 steht, wenn n für 1 steht, und m für 1 steht, wenn n für 0 steht;
worin R1 aus der Gruppe bestehend aus einem geradkettigen C7- bis C17-Alkyl, einem geradkettigen C7- bis C17-Alkenyl, einem verzweigten C7- bis C17-Alkyl, einem verzweigten C7- bis C17-Alkenyl,
einem geradkettigen C7-C17-Monoalkenyl, einem verzweigten C7-C17-Monoalkenyl,
einem geradkettigen C7-C17-Alkadienyl, einem verzweigten C7-C17-Alkadienyl,
einem geradkettigen C7-C17-Alkatrienyl, einem verzweigten C7-C17-Alkatrienyl,
einem geradkettigen Cg-C17-Alkatetraenyl, einem verzweigten Cg-C17-Alkatetraenyl,
und einem geradkettigen C11-C17-Alkapentaenyl, einem verzweigten C11-C17-Alkapentaenyl, ausgewählt ist;
worin
R2 aus der Gruppe bestehend aus H, einem C1- bis C15-Alkyl, einem C1- bis C15-Oxoalkyl, einem C1-bis C15-Hydroxyalkyl, einem C2- bis C15-Alkenyl, einem C2- bis C15-Oxoalkenyl und einem C2- bis C15-Hydroxyalkenyl ausgewählt ist und
R3 aus der Gruppe bestehend aus geradkettigem C1-bis C15-Alkyl mit einem oder zwei unabhängig voneinander aus O, OH, N, NH, Phenyl, Furanyl, C9-Aryl, C10-Aryl, SH und SR4, ausgewählten Substituenten, wobei R4 für einen geradkettigen oder verzweigten C1- bis C5-Alkylrest steht, der gegebenenfalls aus Methyl, Ethyl, Propyl, Isopropyl, Butyl, Isobutyl, Pentyl und Isopentyl ausgewählt ist, und die Gesamtzahl der Kohlenstoffatome in R3 und R4 bis zu 15 beträgt, geradkettigem C1- bis C15-Alkyl mit einem oder zwei Atomen, die unabhängig voneinander aus O, S oder N ausgewählt sind, in der Alkylkette, einfach, zweifach oder mehrfach verzweigtem C3-bis C15-Alkyl, das gegebenenfalls durch einen oder zwei unabhängig voneinander aus O, OH, N, NH, SH und SR4 ausgewählte Reste substituiert ist, wobei R4 für einen geradkettigen oder verzweigten C1-bis C5-Alkylrest steht, der gegebenenfalls aus Methyl, Ethyl, Propyl, Isopropyl, Butyl, Isobutyl, Pentyl und Isopentyl ausgewählt ist, und die Gesamtzahl der Kohlenstoffatome in R3 und R4 bis zu 15 beträgt,
einfach, zweifach oder mehrfach verzweigtem C3-bis C15-Alkyl mit einem oder zwei Atomen, die unabhängig voneinander aus O, S oder N ausgewählt sind, in der Alkylkette, geradkettigem C2- bis C15-Alkenyl, geradkettigem C3- bis C15-Alkadienyl, geradkettigem C2- bis C15-Alkenyl, das durch einen oder mehr unabhängig voneinander aus O, OH, N, NH, Phenyl, Furanyl, C9-Aryl, C10-Aryl, SH und SR4 ausgewählte Reste substituiert ist, wobei R4 für einen geradkettigen oder verzweigten C1- bis C5-Alkenylrest steht, der gegebenenfalls aus Ethenyl, Propenyl, Butenyl und Pentenyl ausgewählt ist,
und die Gesamtzahl der Kohlenstoffatome in R3 und R4 bis zu 15 beträgt,
geradkettigem C2- bis C15-Alkenyl gegebenenfalls mit einem oder mehr Atomen, die unabhängig voneinander aus O, S oder N ausgewählt sind, in der Alkenylkette,
verzweigtem C3-C15-Alkenyl, das gegebenenfalls durch eine oder zwei Alkylgruppen substituiert ist,
verzweigtem C2-C15-Alkenyl mit einer oder zwei Alkylgruppen, das gegebenenfalls durch einen oder zwei unabhängig voneinander aus O, OH, N, NH, Phenyl, Furanyl, Cg-Aryl, C10-Aryl, SH und SR4 ausgewählte Reste substituiert ist, wobei R4 für einen geradkettigen oder verzweigten C1- bis C5-Alkenylrest steht, der gegebenenfalls aus Ethenyl, Propenyl, Butenyl und Pentenyl ausgewählt ist, und die Gesamtzahl der Kohlenstoffatome in R3 und R4 bis zu 15 beträgt,
verzweigtem C3- bis C15-Alkenyl mit einer oder zwei Alkylgruppen und einem oder mehr Atomen, die unabhängig voneinander aus O, S und N ausgewählt sind, in der Alkenylkette,
einfach, zweifach oder mehrfach verzweigtem C5-C15-Alkadienyl, das gegebenenfalls durch eine oder zwei Alkylgruppen substituiert ist, einfach, zweifach oder mehrfach verzweigtem C4-C15-Alkadienyl mit einem oder mehr Atomen, die unabhängig voneinander aus O, S oder N ausgewählt sind, in der Alkenylkette,
einem 5- oder 6-gliedrigen Kohlenstoffringrest mit bis zu zwei Heteroatomen, die unabhängig voneinander aus O, S oder N ausgewählt sind, der gegebenenfalls durch einen odere mehrere unabhängig voneinander aus O, OH, Alkoxy, Alkyl und Alkenyl ausgewählte Reste substituiert ist; einem C1- bis C6-Alkyl oder Alkenyl, substituiert durch einen 5- oder 6-gliedrigen Kohlenstoffringrest mit bis zu zwei Heteroatomen, die unabhängig voneinander aus O, S oder N ausgewählt sind, der gegebenenfalls durch einen odere mehrere unabhängig voneinander aus O, OH, Alkoxy, Alkyl und Alkenyl ausgewählte Reste substituiert ist; ausgewählt ist; oder worin
R2-C-R3 einen Ringrest bilden, der aus Oxacyclopentan, das gegebenenfalls durch einen aus Methyl, Dimethyl, Ethyl, Hydroxy und Methoxy ausgewählten Rest substituiert ist, Oxacyclopenten, das gegebenenfalls durch einen aus Methyl, Dimethyl, Ethyl, Hydroxy und Methoxy ausgewählten Rest substituiert ist, Hydroxycyclopenten, Hydroxyalkylcyclopenten, Hydroxymethylcyclopenten, Hydroxydimethylcyclopenten, Ethylhydroxycyclopenten und Ethylhydroxymethylcyclopenten, Thiacyclopentan, Alkylthiacyclopentan und Alkyl-3-thiacyclopentan, Oxacyclopenten, 2-Oxacyclopenten, 3-Oxacyclopenten, Alkyloxacyclopenten, Alkyl-3-oxacyclopenten, Methyl-3-oxacyclopenten, Alkyl-3-oxacyclopenten, Dimethyl-3-oxacyclopenten, Ethyl-3-oxacyclopenten, Ethylmethyl-3-oxacyclopenten, Hydroxyalkyl-3-oxacyclopenten, Hydroxymethyl-3-oxacyclopenten, Hydroxydimethyl-3-oxacyclopenten, Hydroxyethyl-3-oxacyclopenten, Ethylhydroxymethyl-3-oxacyclopenten, Oxacyclopentan, 3-Oxacyclopentan, Alkyl-3-oxacyclopentan, Methyl-3-oxacyclopentan, Dimethyl-3-oxacyclopentan, Ethyl-3-oxacyclopentan, Ethylmethylalkyl-3-oxacyclopentan, Alkylcyclopenten, Alkenylcyclopenten, Alkylalkenylcyclopenten, Methylcyclopenten, Dimethylcyclopenten, Ethylmethylcyclopenten, Propylmethylcyclopenten, Butylmethylcyclopenten, Butenylmethylcyclopenten, Pentylmethylcyclopenten, Pentenylmethylcyclopenten, Alkylcarboxyalkenylcyclopentan, Alkylcarboxyalkylcyclopentan, Methylcarboxyalkenylcyclopentan, Methylcarboxyalkylcyclopentan, Alkylcarboxypentenylcyclopentan, Alkylcarboxypentylcyclopentan, Methylcarboxyalkenylcyclopentan und Methylcarboxyalkylcyclopentan, wobei das Alkyl aus Methyl, Ethyl, Propyl, Butyl, Pentyl, Pentenyl, Hexyl, Heptyl und Octyl ausgewählt ist, Oxoalkylcyclohexen, Oxomethylcyclohexen, Oxo-trimethylcyclohexen, Oxoethylcyclohexen, Oxoethylmethylcyclohexen, Oxopropylcyclohexen, Oxopropylmethylcyclohexen, Oxoisopropylcyclohexen, Oxoisopropylmethylcyclohexen, Oxothiopropylcyclohexen, Oxothiopropylmethylcyclohexen, Alkylcyclohexen, Methylcyclohexen, Trimethylcyclohexen, Ethylcyclohexen, Ethylmethylcyclohexen, Propylcyclohexen, Propylmethylcyclohexen, Isopropylcyclohexen, Isopropylmethylcyclohexen, Thiopropylcyclohexen, Thiopropylmethylcyclohexen, Alkylcyclohexan, Methylcyclohexan, Trimethylcyclohexan, Ethylcyclohexan, Ethylmethylcyclohexan, Propylcyclohexan, Propylmethylcyclohexan, Isopropylcyclohexan, Isopropylmethylcyclohexan, Thiopropylcyclohexan und Thiopropylmethylcyclohexan ausgewählt ist,
und worin R2 und R3 zusammen eine Gesamtzahl von Kohlenstoffatomen bis zu 15 aufweisen und R2-C-R3 eine Gesamtzahl von Kohlenstoffatomen bis zu 16 aufweist,
einem Lebensmittelprodukt in einer zur Freisetzung eines Geschmacksstoffs mit wahrnehmbarem Aroma beim Verzehr und/oder Erhitzen des Lebensmittelprodukts ausreichenden Konzentration beimischt.

2. Verfahren nach Anspruch 1, bei dem man den Geschmacksstoff aus der Gruppe bestehend aus (+)-8,9-DEHYDRONOOTKATONE; (1R-CIS AND TRANS)-2-(1-ACETYLTHIO-1-METHYL)ETHYL-5-METHYLCYCLOHEXANONE; (E)-2-(2-OCTENYL)CYCLOPENTANONE; (E)-5-ISOPROPOXY-2-DECENAL; (E,E)-3,5-OCTADIEN-2-ONE; (METHYLTHIO)ACETONE; (RAC)-3-ACETYLOXY-5-METHYL-2-HEXANONE; 1-(2,5,5-TRIMETHYL-CYCLOPENT-1-EN-1-YL)-3-METHYL-2-BUTEN-1-ONE; 1-(3,5,5,-TRIMETHYL-1-CYCLOHEXENYL)-3-METHYL-2-BUTEN-1-ONE; 1(5-METHYLFURYL-2)-PROPANE-1,2-DIONE; 1-(METHYLTHIO)-2-BUTANONE; 1-(METHYLTHIO)-3-PENTANONE; 1-(METHYLTHIO)-OCTAN-3-ONE; 1-(P-METHOXYPHENYL)-2-PROPANONE; 1,4-DODEC-6-ENOLACTONE; 1,5,5-TRIMETHYL-8-ETHYL-G-OXA-BICYCLO(4.3.O)NON-6-EN-3-ONE; 1,5-[Z]-OCTADIEN-3-ONE; 10-UNDECENAL; 12-METHYL-TRIDECANAL; 14-METHYL-PENTADECANAL; 1-HYDROXY-2-BUTANONE; 1-MERCAPTO-2-PROPANONE; 1-MERCAPTO-3-PENTANONE; 1-PENTEN-3-ONE; 2-(3,3-DIMETHYLCYCLOHEXYLIDEN)-ETHANAL; 2-(METHYLTHIO)METHYL-2-BUTENAL; 2-(METHYLTHIOMETHYL)-3-PHENYLPROPENAL; 2,10-UNDECADIENAL; 2,3-DIHYDRO-2,3,3-TRIMETHYL-1H-INDEN-1-ONE; 2,4-DIMETHYL-HEPT-4-ENE-3-ONE; 2,4-PENTADIENAL; 2,5-DIETHYL-4-HYDROXY-5-METHYL-3(2H)-FURANONE; 2,5-DIMETHYL-3 (2H)-FURANONE; 2,5-DIMETHYL-4-HYDROXY-6-HEPTANONE; 2,5-DIMETHYL-TETRAHYDRO-3-FURANONE; 2,6-DIMETHYL-3-HYDROXY-4-PYRONE; 2,6-DIMETHYL-4-HEPTANONE; 2,6-DIMETHYLBICYCLO[3.2.1]OCT-2-EN-7-ONE; 2-AMINO-ACETOPHENONE; 2-BUTYL-2-BUTENAL; 2-ETHYL-4-METHYL-PENT-2-ENAL; 2-HEPTEN-4-ONE; 2-HEPTYL-BUTYROLACTONE; 2-HEXENAL; 2-HYDROXY-1-(4-HYDROXY-3-METHOXY-PHENYL)-ETHANONE; 2-HYDROXY-2-CYCLOHEXEN-1-ONE; 2-HYDROXY-3,4,5-TRIMETHYL-2-CYCLOPENTEN-1-ONE; 2-HYDROXY-3,5,5-TRIMETHYL-2-CYCLOHEXENONE; 2-HYDROXYACETOPHENONE; 2-ISOPROPYL-5-CAPROLACTONE; 2-ISOPROPYL-DIOXOLAN-4-ONE; 2-METHOXY-3,4,5-TRIMETHYL-2-CYCLOPENTEN-1-ONE; 2-METHYL-2-OCTENAL; 2-METHYL-3-(P-METHYLPHENYL)PROPANAL; 2-METHYL-8-PHENYL-OCT-2-ENE-6-ONE; 2-METHYLCYCLOHEXANONE; 2-METHYLHEPTAN-3-ONE; 2-METHYLHEXAN-3-ONE; 2-METHYLNONANAL; 2-METHYL-SPIRO(5,5)-UNDECAN-1-ONE; 2-OCTEN-4-ONE; 2-PHENYL-3-(2-FURYL)PROP-2-ENAL; 2-PHENYL-4-PENTENAL; 2-TETRADECENAL; 2-TRANS-4-CIS-7-CIS-TRIDECATRIENAL; 3-((2-METHYL-3-FURYL)THIO)-4-HEPTANONE; 3(2)-HYDROXY-4-METHYL-HEXAN-2(3)-ONE; 3-(3-METHOXY-PHENYL)-PROPAN-2-ONE; 3-(5-METHYL-2-FURYL)BUTANAL; 3(Z),6(Z),9(Z)-DODECATRIENAL; 3(Z),6(Z)-DODECADIENAL; 3,3-DIETHOXY-2-BUTANONE; 3,3-DIMETHYLCYCLOHEXANONE; 3,4,5,6-TETRAHYDRO PSEUDO IONONE; 3,4-DIMETHYL-BUTYROLACTONE; 3,5-DIMETHYL-4-THIA-HEPTAN-2,6-DIONE; 3,6,DIMETHYL-5,6-DIHYDRO-2(4H)BENZOFURANONE; 3,6-DIMETHYL-2-HYDROXY-2-CYCLOHEXEN-1-ONE; 3,7-DIMETHYL-6-OCTEN-2-ONE; 3A,4,5,7A-TETRAHYDRO-3,6-DIMETHYL-2(3H)BENZOFURANONE; 3-ACETYLOXY-2-OCTANONE; 3-ACETYLOXY-2-PENTANONE; 3-ACETYLSULFANYL-2-ETHYLHEXANAL; 3-DECANONE; 3-ETHOXY HEXANAL; 3-ETHYL-2-HYDROXY-4-METHYLCYCLOPENT-2-EN-1-ONE; 3-ETHYL-4,5,5-TRIMETHYL-2-(5H)-FURANONE; 3-HEPTYLDIHYDRO-5-METHYL-2(3H)-FURANONE; 3-HEXANONE; 3-HYDROXY-2-PENTANONE; 3-HYDROXY-4,5,5-TRIMETHYL-2-(5H)-FURANONE; 3-HYDROXY-4-METHYL-5-PENTYL-2(5H)-FURANONE; 3-HYDROXY-4-METHYL-ALPHA-PYRONE; 2-ACETYL-4,5-DIMETHYL-THIAZOLE; 2-METHYL-3-ACETYLPYRIDINE; 1-ETHYL-2-ACETYL-PYRROLE; 1-METHYL-2-ACETYLPYRROLE; 2,4-DIMETHYL-5-ACETYL THIAZOLE; 3-ACETYL-2,5-DIMETHYLFURAN; 1,4-DIMETHYL-4-ACETYL-1-CYCLOHEXENE; 4-ACETYL-6-T-BUTYL-1,1-DIMETHYLINDAN; 4-ACETYL-2-METHYLPYRIMIDINE; ACETYL-2-PYRAZINE; ACETYL-2-FURAN; ACETYL-2-PYRROLE; ACETYL-2-PYRIDINE; ACETYL-2-DIMETHYL-PYRAZINE; ACETYL-2-THIAZOLE; ACETYL-3 PYRIDIN; ACETYL-3-DIMETHYL-2.5-THIOPHENE; ACETYL-2 METHYL-5 FURAN; ACETYL-2-THIAZOLINE-2; ACETYL-2 METHYL-3 PYRAZINE; PROPIONYL PYRAZINE; PROPIONYL-2-METHYL-3-FURANE; 2-PROPIONYLTHIAZOLE; 2-PROPIONYLPYRROLE; 3-HYDROXY-4-PHENYL-2-BUTANONE; 3-HYDROXY-5-HEXYL-4-METHYL-2(5H)-FURANONE; 3-HYDROXY-6-HYDROXYMETHYL-PYRAN-2-ONE; 3-HYDROXYMETHYL-2-OCTANONE; 3-ISOAMYL-2,4-PENTANDIONE; 3-MERCAPTO-2-METHYLPENTANAL; 3-MERCAPTO-2-OCTANONE; 3-MERCAPTO-2-PENTANONE; 3-MERCAPTO-4-PHENYL-2-BUTANONE; 3-MERCAPTO-5-METHYL-HEXAN-2-ONE; 3-METHYL NON-2(E)-EN-40NE; 3-METHYL-1-CYCLOPENATADECANONE; 3-METHYL-2-CYCLOHEXEN-1-ONE; 3-METHYL-2-CYCLOPENTEN-1-ONE; 3-METHYL-2-HEPTEN-4,5-DIONE; 3-METHYL-3-(3-METHYL-BUT-2-ENYL)-DIHYDRO-FURAN-2-ONE; 3-METHYL-4-PHENYL-3-BUTENE-2-ONE; 3-METHYL-5-PROPYL-2-CYCLOHEXEN-1-ONE; 3-METHYLCYCLOHEXANONE; 3-METHYLENE-2-OCTANONE; 3-METHYL-GAMMA-DECALACTONE; 3-METHYLPENTANAL; 3-METHYLTHIOHEXANAL; 3-NONANONE; 3-NONEN-2-ONE; 3-PENTANONE; 3-PENTEN-2-ONE; 3-PENTYL-4,5,5-TRIMETHYL-2-(5H)-FURANONE; 3-PHENYL-4-PENTENAL; 3-PROPYL-2-CYCLOPENTEN-1-ONE; 3-PROPYLIDEN-HEPTAN-2-ONE; 4-(2,6,6-TRIMETHYL-2-CYCLOHEXEN-1-YL)BUTAN-2-ONE; 4-(2,6,6-TRIMETHYL-CYCLOHEXA-1,3-DIENYL)-BUT-3-EN-2-ONE; 4-(FURAN-2-YL)-BUTAN-2-ONE; 4-(METHYLTHIO)BUTANAL; 4-(P-TOLYL)-2-BUTANONE; 4,4-DIMETHOXY-3-HEXANONE; 4,4-DIMETHYL-1,3-OXATHIANE-2-ONE; 4,8-DIMETHYL-3,7-NONADIEN-2-ONE; 4-[(2-FURANMETHYL)THIO]-2-PENTANONE; 4-ACETOXY-HEX-4-EN-3-ONE; 4-EPOXY-1,1,3-TRIMETHYLCYCLOHEX-5-EN-2-ONE; 4-ETHYL-1,3-OXATHIAN-2-ONE; 4-ETHYL-5-METHYL-1,3-OXATHIAN-2-ONE; 4-HEPTYL-3-METHYLBUTYROLACTONE; 4-HYDROXY-2,2,5-TRIMETHYL-3[2H]-FURANONE; 4-HYDROXY-3,5,5-TRIMETHYL-CYCLOHEX-5,5-EN-1-ONE; 4-HYDROXY-4-METHYL-5-HEXENOIC-ACID LACTONE; 4-HYDROXY-4-METHYL-CIS-7-DECANOICACID-4-LACTONE; 4-MERCAPTO-4-METHYL-PENTAN-2-ONE; 4-METHYL-2-PENTENAL; 4-METHYL-3-PENTEN-2-ONE; 4-METHYL-4-FURFURYLTHIO-2-PENTANONE; 4-METHYL-5-PENTYL-DIHYDRO-2(3H)-FURANONE; 4-METHYLCYCLOHEXANONE; 4-METHYLTHIO-2-PENTANONE; 4-OXO-BETA DAMASCONE; 4-PHENYL-3,5-DITHIA-2-HEXANONE; 5 ALPHA-ANDROST-16-EN-3-ONE; 5,5-DIMETHYL-2(5H)-FURANONE; 5,5-DIMETHYL-2-METHOXY-CYCLOHEX-2-ENONE; 5-ETHYL-2-HYDROXY-3,4-DIMETHYL-2-CYCLOPENTEN-1-ONE; 5-ETHYL-2-METHOXY-3,4-DIMETHYL-2-CYCLOPENTEN-1-ONE; 5-ETHYLDIHYDRO-5-METHYL-2(3H)-FURANONE; 5-HYDROXY-4-OCTANONE; 5-HYDROXY-8-UNDECENOIC ACID D-LACTONE; 5-METHYL-2-PROPYL-[1,3]-DIOXOLAN-4-ONE; 5-METHYL-3-(3-METHYL-BUT-2-ENYL)-DIHYDRO-FURAN-2-ONE; 5-METHYL-3-HEXEN-2-ONE; 5-METHYL-4-HEXENE-2,3-DIONE; 5-METHYL-5-HEXEN-2-ONE; 5-PENTYL-ALPHA-PYRONE; 5-P-TOLUYL-2(3H)-FURANONE; 6-(1-PENTENYL)-2H-PYRAN-2-ONE; 6-HEPT-1-ENYL-5,6-DIHYDRO-PYRAN-2-ONE; 6-HYDROXY-3,7-DIMETHYLOCTANOIC ACID LACTONE; 6-METHYL-3-HEPTEN-2-ONE; 6-METHYL-NON-5-EN-4-ONE; 6-METHYL-OCTANAL; 6-PROPYL-[1,3]OXATHIAN-2-ONE; 6-UNDECANONE; 7-METHYL-2,3,4,4A,5,6-HEXAHYDRO-2-NAPHTHALENONE; 7-METHYL-6-OCTEN-2-ONE; 7-OCTENAL; 8-(2,2-DIMETHYLCYCLOPROPYL)-OCTA-3,5-DIEN-2-ONE; 8A-METHYL-3,4,4A,5,8,8A-HEXAHYDRO-1(2H)-NAPHTHALENONE; 8-METHYL NONANAL; 8-METHYL-TRIDEC-7-EN-6-ONE; 8-NONEN-2-ONE; 8-NONENAL; 9-DECENAL; ACETONE; ACETOPHENONE; AR-TURMERONE; BENZOPHENONE; BENZOYLACETONE; BENZYL DIPROPYL KETONE; BENZYL-ACETONE; BENZYLIDENE ACETONE; BETA-IRONE; BICYCLO-IRONE; BICYCLONE; BICYCLONONALACTONE; BUTYRO-1,4-LACTONE; CARYOPHYLLEN-12-AL; CIS,CIS-4,7-DECADIEN-1-AL; CIS-2-NONENAL; CIS-3-HEXENAL; CIS-3-NONENAL; CIS-4-HEPTEN-2-ONE; CIS-4-HEXENAL; CIS-4-NONENAL; CIS-5-NONENAL; CIS-5-OCTENAL; CIS-7-NONENAL; CITRONELLENE LACTONE; CLONAL; COGNAC LACTONE; CYCLOBUTONE; CYCLOHEPTADECA-9-EN-1-ONE; CYCLOHEXANONE; CYCLOIONONE; CYCLOPENTANONE; CYCLOHEPTADECANONE; DAMASCENONE; DECANAL; DECENYL CYCLOPENTANONE; DIBENZYL KETONE; DIBUTYL-4,4 GAMMA BUTYROLACTONE; DIHYDRO FILBERTONE; DIHYDRO-5-METHYL-5-PROPYL-2(3H)-FURANONE; DIHYDROCARVONE; DIHYDRO-DEHYDRO-GAMMA-IONONE; DIHYDROJASMONE LACTONE; DIHYDROLAVENDELLACTONE; DIMETHYL HYDROQUINONE; DIMETHYL METHOXY FURANONE; DIMETHYL-2,4 ACETOPHENONE; DIMETHYL-2,6 OCTANAL; DIMETHYL-3,6-BENZO-2(3H)-FURANONE; DODEC-2-EN-1,5-LACTONE; DODEC-2-EN-4-ONE; DODECANAL; E-DECALACTONE; E-DODECALACTONE; ETHYL-2-HEPTEN-2-AL; ETHYL-3 CYCLOPENTANDIONE; FARNESYL ACETONE; FURFURYLIDENE ACETONE; GAMMA DAMASCONE; GAMMA IONONE; GERANYL ACETONE; GINGERONE; HEPT-3-EN-2-ONE; HEPTANAL; HEXANAL; HEXEN 5 ONE; HEXENYL METHYL KETONE; HEXYLIDENE CYCLOPENTANONE; HOMOCORYLONE; HYDROBICYCLONE; HYDROXY-3(2)-HEPTAN-2(3)-ONE; HYDROXY-5-DECADIENOIC ACID-2,4 LACTONE; ISO JASMONE; ISOLONGIFOLENE-KETONE; ISOPHORONE; JASMIN LACTONE; LAVENDELLACTONE; MASSOIA LACTONE; MELONAL, DIMETHYL HEPTENAL; MENTHONE; METHIONAL; METHOXY-P-PHENYL-PENTEN-3-ONE, ETHONE; METHYL 2 OCTANAL; METHYL 2-PENTENE-1-AL; METHYL THIO-METHYLTHIOMETHYL-2-PENTENAL; METHYL-2 UNDECANAL; METHYL-2-BUTANAL; METHYL-2-PENT-2-ENAL; METHYL-2-TETRAHYDRO-FURANONE; METHYL-4-HYDROXY-3/2H/- FURANONE; METHYL-5-HEPT-2-EN-4-ONE; METHYL-6-HEPTA-3,5-DIENONE; METHYLTHIO-3-BUTANAL; METHYLTHIO-4-BUTAN-2-ONE; MINTLACTONE; MYRTENAL; NEOFOLIONE; NEOHESPERIDINE DIHYDROCHALCONE; NONANAL; NOOTKATONE; OCTAN-2-ONE; OCTANAL; PENNYROYAL OIL 84% PULEGONE; PENTADECANONE; PENTANAL; PENTYL 2-FURYL KETONE; PHENYL-2-BUTEN-2-AL; PHENYL-3-PROPANAL; PIPERITENONE; PIPERONYL ACETONE; P-MENTH-1-ENAL; P-MENTH-1-ENE-9-AL; P-MENTHAN-2-ONE; PRENAL; PROPANAL; PROPYL-ISO PARA ACETOPHENONE; RAC-3-HYDROXY-5-METHYL-2-(5H)-FURANONE; RASPBERRY KETONE; SAFRANAL; SAGE OIL 27% THUJONE; SOLANONE; SPIRO-(6,5)-DODECAN-1-ONE; TETRAHYDROIRONE; TETRAHYDRONOOTKATONE; TRANS-4-HEXENAL; TRANS-5-NONENAL; TRANS-6-DECEN-1-AL; TRANS-6-NONENAL; TRANS-7-METHYL-3-OCTEN-2-ONE; TRANS-7-NONENAL; TRIDECAN-2-ONE; TRIMETHYL-3,5,5-HEXANAL; TRITHIOACETONE; UNDECANAL; VANILLYLIDENE ACETONE; und VERBENONE auswählt.

3. Verfahren nach einem der Ansprüche 1 bis 2, bei dem R1 für ein Alkyl aus der Gruppe bestehend aus C7-, C8-, C9-, C10-, C11-, C13-, C15- und C17-Alkyl steht.

4. Verfahren nach einem der Ansprüche 1 bis 2, bei dem R1 für ein Alkenyl aus der Gruppe bestehend aus C7-, C8-, C9-, C10-, C11-, C13-, C15- und C17-Alkenyl, ein C17-8-En-Alkenyl (Ölsäurerest), ein C17-8,11-Alkadienyl (Linolsäurerest) und ein C17-8,11,14-trienyl (Linolensäurerest) steht.

5. Verfahren nach einem der Ansprüche 1 bis 2, bei dem R2 aus der Gruppe bestehend aus H, Methyl und Ethyl ausgewählt ist.

6. Verfahren nach einem der Ansprüche 1 bis 2, bei dem R3 aus der Gruppe bestehend aus einem geradkettigen C1- bis C8-Alkyl, einem verzweigten C1- bis C8-Alkyl mit bis zu zwei Alkylgruppen, wobei der Alkylrest einen oder mehrere weitere Alkylreste enthalten kann, einem geradkettigen C1-bis C8-Alkyl mit einem oder zwei aus O und SR4 ausgewählten Substituenten, wobei R4 für einen aus Methyl und Ethyl ausgewählten Alkylrest steht, einem geradkettigen C2- bis C8-Alkenyl und einem Ring, der aus einem 5- oder 6-gliedrigen Ring mit bis zu zwei N-Heteroatomen ausgewählt ist, wobei der Ring ferner durch eine oder mehrere aus Methyl, Ethyl, Propyl und Isopropyl ausgewählte Alkylgruppen substituiert sein kann, ausgewählt ist.

7. Verfahren nach einem der Ansprüche 1 bis 2, bei dem R1 für ein Alkyl aus der Gruppe bestehend aus C7-, C9-, C11-, C13-, C15- und C17-Alkyl steht, R2 aus der Gruppe bestehend aus H, Methyl und Ethyl ausgewählt ist und R3 aus der Gruppe bestehend aus einem geradkettigen C1- bis C8-Alkyl, einem verzweigten C1- bis C8-Alkyl mit bis zu zwei Alkylgruppen, wobei der Alkylrest einen oder mehrere weitere Alkylreste enthalten kann, einem geradkettigen C1- bis C8-Alkyl mit einem oder zwei aus O und SR4 ausgewählten Substituenten, wobei R4 für einen aus Methyl und Ethyl ausgewählten Alkylrest steht, einem geradkettigen C2- bis C8-Alkenyl und einem Ring, der aus einem 5- oder 6-gliedrigen Ring mit bis zu zwei N-Heteroatomen ausgewählt ist, wobei der Ring ferner durch eine oder mehrere aus Methyl, Ethyl, Propyl und Isopropyl ausgewählte Alkylgruppen substituiert sein kann, ausgewählt ist.

8. Geschmacksstoffzusammensetzung, die mindestens einen Geschmacksstoffvorläufer gemäß einem der Ansprüche 2 bis 7 umfasst.

9. Lebensmittelprodukt, das mindestens einen Geschmacksstoffvorläufer gemäß einem der Ansprüche 1 bis 7 umfasst.

10. Geschmacksstoffvorläuferverbindung gemäß einem der Ansprüche 1 bis 9, worin m = 1 und n = 0.

11. Geschmacksstoffvorläuferverbindung gemäß einem der Ansprüche 2 bis 9, worin m = 0 und n = 1.

12. Verfahren zur Herstellung der Geschmacksstoffvorläuferverbindung nach Anspruch 10 oder 11 durch Umsetzung mindestens einer Geschmacksstoffverbindung mit einer oder mehreren Carbonylgruppen mit mindestens einem Monoglycerid in einer säurekatalysierten Reaktion.

## Revendications

1. Procédé de préparation d'un produit alimentaire aromatisé, **caractérisé en ce qu'**au moins un précurseur d'arôme de formule I 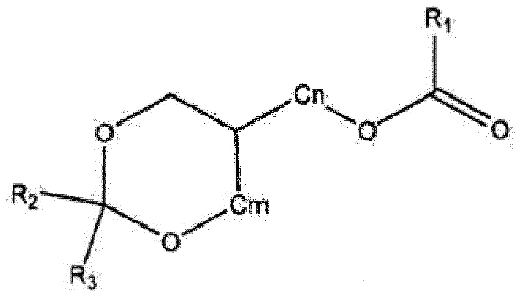 dans laquelle n et m sont choisis parmi 0 et 1, et si n est 1, alors m est 0, et si n est 0, alors m est 1 ;
dans laquelle R₁ est choisi dans le groupe constitué par
un alkyle en C₇ à C₁₇ à chaîne droite, un alcényle en C₇ à C₁₇ à chaîne droite, un alkyle en C₇ à C₁₇ ramifié, un alcényle en C₇ à C₁₇ ramifié,
un monoalcényle en C₇ à C₁₇ à chaîne droite, un monoalcényle en C₇ à C₁₇ ramifié,
un alcadiényle en C₇ à C₁₇ à chaîne droite, un alcadiényle en C₇ à C₁₇ ramifié,
un alcatriényle en C₇ à C₁₇ à chaîne droite, un alcatriényle en C₇ à C₁₇ ramifié,
un alcatétraényle en C₉ à C₁₇ à chaîne droite, un alcatétraényle en C₉ à C₁₇ ramifié,
et un alcapentaényle en C₁₁ à C₁₇ à chaîne droite, un alcapentaényle en C₁₁ à C₁₇ ramifié ;
dans laquelle
R₂ est choisi dans le groupe constitué par H, un alkyle en C₁ à C₁₅, un oxoalkyle en C₁ à C₁₅, un hydroxyalkyle en C₁ à C₁₅, un alcényle en C₂ à C₁₅, un oxoalcényle en C₂ à C₁₅, et un hydroxyalcényle en C₂ à C₁₅ et
R₃ est choisi dans le groupe constitué par alkyle en C₁ à C₁₅ à chaîne droite comprenant un ou deux substituants choisis indépendamment parmi O, OH, N, NH, phényle, furanyle, aryle en C₉, aryle en C₁₀, SH et SR₄, R₄ étant un résidu alkyle en C₁ à C₅ à chaîne droite ou ramifiée choisi éventuellement parmi méthyle, éthyle, propyle, isopropyle, butyle, isobutyle, pentyle et isopentyle, et le nombre total d'atomes de carbone dans R₃ et R₄ étant jusqu'à 15,
alkyle en C₁ à C₁₅ à chaîne droite comprenant un ou deux atomes choisis indépendamment parmi O, S ou N au sein de la chaîne alkyle, alkyle en C₃ à C₁₅ à un ou deux branchements ou plus éventuellement substitué par un ou deux résidus choisis indépendamment parmi O, OH, N, NH, SH, et SR₄, R₄ étant un résidu alkyle en C₁ à C₅ à chaîne droite ou ramifiée choisi éventuellement parmi méthyle, éthyle, propyle, isopropyle, butyle, isobutyle, pentyle et isopentyle, et le nombre total d'atomes de carbone dans R₃ et R₄ étant jusqu'à 15,
alkyle en C₃ à C₁₅ à un ou deux branchements ou plus comprenant un ou deux atomes choisis indépendamment parmi O, S ou N au sein de la chaîne alkyle, alcényle en C₂ à C₁₅ à chaîne droite, alcadiényle en C₃ à C₁₅ à chaîne droite, alcényle en C₂ à C₁₅ à chaîne droite éventuellement substitué par un ou plusieurs résidus choisis indépendamment parmi O, OH, N, NH, phényle, furanyle, aryle en C₉, aryle en C₁₀, SH, SR₄, R₄ étant un résidu alcényle en C₁ à C₅ à chaîne droite ou ramifiée choisi éventuellement parmi éthényle, propényle, butényle et pentényle, et le nombre total d'atomes de carbone dans R₃ et R₄ étant jusqu'à 15,
alcényle en C₂ à C₁₅ à chaîne droite comprenant éventuellement un ou plusieurs atomes choisis indépendamment parmi O, S et N au sein de la chaîne alcényle,
alcényle en C₃ à C₁₅ ramifié éventuellement substitué par un ou deux groupements alkyle, alcényle en C₂ à C₁₅ ramifié comprenant un ou deux groupements alkyle éventuellement substitués par un ou deux résidus choisis indépendamment parmi O, OH, N, NH, phényle, furanyle, aryle en C₉, aryle en C₁₀, SH, SR₄, R₄ étant un résidu alcényle en C₁ à C₅ à chaîne droite ou ramifiée choisi éventuellement parmi éthényle, propényle, butényle et pentényle, et le nombre total d'atomes de carbone dans R₃ et R₄ étant jusqu'à 15,
alcényle en C₃ à C₁₅ ramifié comprenant un ou deux groupements alkyle et un ou plusieurs atomes choisis indépendamment parmi O, S et N au sein de la chaîne alcényle,
alcadiényle en C₅ à C₁₅ à un ou deux branchements ou plus, éventuellement substitué par un ou deux groupements alkyle,
alcadiényle en C₄ à C₁₅ à un ou deux branchements ou plus comprenant un ou plusieurs atomes choisis indépendamment parmi O, S et N au sein de la chaîne alcényle ;
un résidu de cycle carboné à 5 ou 6 chaînons comprenant jusqu'à deux hétéroatomes choisis indépendamment parmi O, S et N, éventuellement substitué par un ou plusieurs résidus choisis indépendamment parmi O, OH, alcoxy, alkyle, alcényle, alkyle ou alcényle en C₁ à C₆ substitué par un résidu de cycle carboné à 5 ou 6 chaînons comprenant jusqu'à deux hétéroatomes choisis indépendamment parmi O, S et N, éventuellement substitué par un ou plusieurs résidus choisis indépendamment parmi O, OH, alcoxy, alkyle, alcényle ;
ou dans laquelle
R₂-C-R₃ forment un résidu cyclique choisi parmi l'oxacyclopentane éventuellement substitué par un résidu choisi parmi méthyle, diméthyle, éthyle, hydroxy et méthoxy, l'oxacyclopentène éventuellement substitué par un résidu choisi parmi méthyle, diméthyle, éthyle, hydroxy et méthoxy, l'hydroxycyclopentène, l'hydroxyalkylcyclopentène, l'hydroxyméthylcyclopentène, l'hydroxydiméthylcyclopentène, l'éthylhydroxycyclopentène, et l'éthylhydroxyméthylcyclopentène, la thiacyclopentane, l'alkylthiacyclopentane et l'alkyl-3-thiacyclopentane, l'oxacyclopentène, le 2-oxacyclopentène, le 3-oxacyclopentène, l'alkyloxacyclopentène, l'alkyl-3-oxacyclopentène, le méthyl-3-oxacyclopentène, l'alkyl-3-oxacyclopentène, le diméthyl-3-oxacyclopentène, l'éthyl-3-oxacyclopentène, l'éthylméthyl-3-oxacyclopentène, l'hydroxyalkyl-3-oxacyclopentène, l'hydroxyméthyl-3-oxacyclopentène, l'hydroxydiméthyl-3-oxacyclopentène, l'hydroxyéthyl-3-oxacyclopentène, l'éthylhydroxyméthyl-3-oxacyclopentène, l'oxacyclopentane, la 3-oxacyclopentane, l'alkyl-3-oxacyclopentane, la méthyl-3-oxacyclopentane, la diméthyl-3-oxacyclopentane, l'éthyl-3-oxacyclopentane, l'éthylméthylalkyl-3-oxacyclopentane, l'alkylcyclopentène, l'alcénylcyclopentène, l'alkylalcénylcyclopentène, le méthylcyclopentène, le diméthylcyclopentène, l'éthylméthylcyclopentène, le propylméthylcyclopentène, le
butylméthylcyclopentène, le buténylméthylcyclopentène, le pentylméthylcyclopentène, le penténylméthylcyclopentène, l'alkylcarboxyalcénylcyclopentane, l'alkylcarboxyalkylcyclopentane, la méthylcarboxyalcénylcyclopentane, la méthylcarboxyalkylcyclopentane, l'alkylcarboxypenténylcyclopentane, l'alkylcarboxy-pentylcyclopentane, la méthylcarboxyalcénylcyclopentane et la méthylcarboxyalkylcyclopentane, et l'alkyle étant choisi parmi méthyle, éthyle, propyle, butyle, pentyle, pentényle, hexyle, heptyle et octyle, l'oxo-alkylcyclohexène, l'oxo-méthylcyclohexène, l'oxo-triméthylcyclohexène, l'oxo-éthylcyclohexène, l'oxo-éthylméthylcyclohexène, l'oxo-propylcyclohexène, l'oxo-propylméthylcyclohexène, l'oxo-isopropylcyclohexène, l'oxo-isopropylméthylcyclohexène, l'oxo-thiopropylcyclohexène, l'oxo-thiopropylméthylcyclohexène l'alkylcyclohexène, le méthylcyclohexène, le triméthylcyclohexène, l'éthylcyclohexène, l'éthylméthylcyclohexène, le propylcyclohexène, le propylméthylcyclohexène, l'isopropylcyclohexène, l'isopropylméthylcyclohexène, le thiopropylcyclohexène, le thiopropylméthylcyclohexène, l'alkylcyclohexane,
la méthylcyclohexane, la triméthylcyclohexane, l'éthylcyclohexane, l'éthylméthylcyclohexane, la propylcyclohexane, la propylméthylcyclohexane, l'isopropylcyclohexane, l'isopropylméthylcyclohexane, la thiopropylcyclohexane et la thiopropylméthylcyclohexane,
et dans laquelle R₂ et R₃ ont ensemble un nombre total d'atomes de carbone allant jusqu'à 15, et R₂-C-R₃ a un nombre total d'atomes de carbone allant jusqu'à 16,
est ajouté à un produit alimentaire dans une concentration suffisante pour libérer un arôme ayant un parfum susceptible d'être remarqué dès la consommation et/ou le chauffage du produit alimentaire.

2. Procédé selon la revendication 1, **caractérisé en ce que** ledit arôme est choisi dans le groupe constitué par LA (+)-8,9-DESHYDRONOOTKATONE ; LA (1R-CIS ET TRANS)-2-(1-ACETYLTHIO-1-METHYL)ETHYL-5-METHYLCYCLOHEXANONE ; LA (E)-2-(2-OCTENYL)CYCLOPENTANONE ; LE (E)-5-ISOPROPOXY-2-DECENAL ; LA (E,E)-3,5-OCTADIEN-2-ONE ; LA (METHYLTHIO)ACETONE ; LA (RAC)-3-ACETYLOXY-5-METHYL-2-HEXANONE ; LA 1-(2,5,5-TRIMETHYL-CYCLOPENT-1-EN-1-YL)-3-METHYL-2-BUTEN-1-ONE ; LA 1-(3,5,5,-TRIMETHYL-1-CYCLOHEXENYL)-3-METHYL-2-BUTEN-1-ONE ; LA 1(5-METHYLFURYL-2)-PROPANE-1,2-DIONE ; LA 1-(METHYLTHIO)-2-BUTANONE ; LA 1-(METHYLTHIO)-3-PENTANONE ; LA 1-(MÉTHYLTHIO)-OCTAN-3-ONE ; LA 1-(P-METHOXYPHENYL)-2-PROPANONE ; LA 1,4-DODEC-6-ENOLACTONE ; LA 1,5,5-TRIMETHYL-8-ETHYL-G-OXA-BICYCLO(4,3,0)NON-6-EN-3-ONE ; LA 1,5-[Z]-OCTADIEN-3-ONE ; LE 10-UNDECENAL ; LE 12-METHYL-TRIDECANAL ; LE 14-METHYL-PENTADECANAL ; LA 1-HYDROXY-2-BUTANONE ; LA 1-MERCAPTO-2-PROPANONE ; LA 1-MERCAPTO-3-PENTANONE ; LA 1-PENTEN-3-ONE ; LE 2-(3,3-DIMETHYLCYCLOHEXYLIDEN)-ETHANAL ; LE 2-(METHYLTHIO)METHYL-2-BUTENAL ; LE 2-(METHYLTHIOMETHYL)-3-PHENYLPROPENAL ; LE 2,10-UNDECADIENAL ; LA 2,3-DIHYDRO-2,3,3-TRIMETHYL-1H-INDEN-1-ONE ; LA 2,4-DIMETHYL-HEPT-4-ENE-3-ONE ; LE 2,4-PENTADIENAL ; LA 2,5-DIETHYL-4-HYDROXY-5-METHYL-3(2H)-FURANONE ; LA 2,5-DIMETHYL-3 (2H)-FURANONE ; LA 2,5-DIMETHYL-4-HYDROXY-6-HEPTANONE ; LA 2,5-DIMETHYL-TETRAHYDRO-3-FURANONE ; LA 2,6-DIMETHYL-3-HYDROXY-4-PYRONE ; LA 2,6-DIMETHYL-4-HEPTANONE ; LA 2,6-DIMETHYLBICYCLO[3,2,1]OCT-2-EN-7-ONE ; LA 2-AMINO-ACETOPHENONE ; LE 2-BUTYL-2-BUTENAL ; LE 2-ETHYL-4-METHYL-PENT-2-ENAL ; LA 2-HEPTEN-4-ONE ; LA 2-HEPTYL-BUTYROLACTONE ; LE 2-HEXENAL ; LA 2-HYDROXY-1-(4-HYDROXY-3-METHOXYPHENYL)-ETHANONE ; LA 2-HYDROXY-2-CYCLOHEXEN-1-ONE ; LA 2-HYDROXY-3,4,5-TRIMETHYL-2-CYCLOPENTEN-1-ONE ; LA 2-HYDROXY-3,5,5-TRIMETHYL-2-CYCLOHEXENONE ; LA 2-HYDROXYACETOPHENONE ; LA 2-ISOPROPYL-5-CAPROLACTONE ; LA 2-ISOPROPYL-DIOXOLAN-4-ONE ; LA 2-METHOXY-3,4,5-TRIMETHYL-2-CYCLOPENTEN-1-ONE ; LE 2-METHYL-2-OCTENAL ; LE 2-METHYL-3-(P-METHYLPHENYL)PROPANAL ; LA 2-METHYL-8-PHENYL-OCT-2-ENE-6-ONE ; LA 2-METHYLCYCLOHEXANONE ; LA 2-METHYLHEPTAN-3-ONE ; LA 2-METHYLHEXAN-3-ONE ; LE 2-METHYLNONANAL ; LA 2-METHYL-SPIRO(5,5)-UNDECAN-1-ONE ; LA 2-OCTEN-4-ONE ; LE 2-PHENYL-3-(2-FURYL)PROP-2-ENAL ; LE 2-PHENYL-4-PENTENAL ; LE 2-TETRADECENAL ; LE 2-TRANS-4-CIS-7-CIS-TRIDECATRIENAL ; LA 3-((2-METHYL-3-FURYL)THIO)-4-HEPTANONE ; LA 3(2)-HYDROXY-4-METHYL-HEXAN-2(3)-ONE ; LA 3-(3-METHOXYPHENYL)-PROPAN-2-ONE ; LE 3-(5-METHYL-2-FURYL)BUTANAL ; LE 3(Z),6(Z),9(Z)-DODECATRIENAL ; LE 3(Z),6(Z)-DODECADIENAL ; LA 3,3-DIETHOXY-2-BUTANONE ; LA 3,3-DIMETHYLCYCLOHEXANONE ; LA 3,4,5,6-TETRAHYDRO PSEUDO IONONE ; LA 3,4-DIMETHYL-BUTYROLACTONE ; LA 3,5-DIMETHYL-4-THIA-HEPTANE-2,6-DIONE ; LA 3,6,DIMETHYL-5,6-DIHYDRO-2(4H)BENZOFURANONE ; LA 3,6-DIMETHYL-2-HYDROXY-2-CYCLOHEXEN-1-ONE ; LA 3,7-DIMETHYL-6-OCTEN-2-ONE ; LA 3A,4,5,7A-TETRAHYDRO-3,6-DIMETHYL-2(3H)BENZOFURANONE ; LA 3-ACETYLOXY-2-OCTANONE ; LA 3-ACETYLOXY-2-PENTANONE ; LE 3-ACETYLSULFANYL-2-ETHYLHEXANAL ; LA 3-DECANONE ; LE 3-ETHOXY HEXANAL ; LA 3-ETHYL-2-HYDROXY-4-METHYLCYCLOPENT-2-EN-1-ONE ; LA 3-ETHYL-4,5,5-TRIMETHYL-2-(5H)-FURANONE ; LA 3-HEPTYLDIHYDRO-5-METHYL-2(3H)-FURANONE ; LA 3-HEXANONE ; LA 3-HYDROXY-2-PENTANONE ; LA 3-HYDROXY-4,5,5-TRIMETHYL-2-(5H)-FURANONE ; LA 3-HYDROXY-4-METHYL-5-PENTYL-2(5H)-FURANONE ; LA 3-HYDROXY-4-METHYL-ALPHA-PYRONE ; LE 2-ACETYL-4,5-DIMETHYL-THIAZOLE ; LA 2-METHYL-3-ACETYLPYRIDINE ; LE 1-ETHYL-2-ACETYL-PYRROLE ; LE 1-METHYL-2-ACETYLPYRROLE ; LE 2,4-DIMETHYL-5-ACETYL-THIAZOLE ; LE 3-ACETYL-2,5-DIMETHYLFURANE ; LE 1,4-DIMETHYL-4-ACETYL-1-CYCLOHEXENE ; LE 4-ACETYL-6-T-BUTYL-1,1-DIMETHYLINDANE ; LA 4-ACETYL-2-METHYLPYRIMIDINE ; L'ACETYL-2-PYRAZINE ; L'ACETYL-2-FURANE ; L'ACETYL-2-PYRROLE ; L'ACETYL-2-PYRIDINE ; L'ACETYL-2-DIMETHYL-PYRAZINE ; L'ACETYL-2-THIAZOLE ; L'ACETYL-3-PYRIDINE ; L'ACETYL-3-DIMETHYL-2,5-THIOPHENE ; L'ACETYL-2-METHYL-5-FURANE ; L'ACETYL-2-THIAZOLINE-2 ; L'ACETYL-2-METHYL-3-PYRAZINE ; LA PROPIONYL-PYRAZINE ; LE PROPIONYL-2-METHYL-3-FURANE ; LE 2-PROPIONYLTHIAZOLE ; LE 2-PROPIONYLPYRROLE ; LA 3-HYDROXY-4-PHENYL-2-BUTANONE ; LA 3-HYDROXY-5-HEXYL-4-METHYL-2(5H)-FURANONE ; LA 3-HYDROXY-6-HYDROXYMETHYL-PYRAN-2-ONE ; LA 3-HYDROXYMETHYL-2-OCTANONE ; LA 3-ISOAMYL-2,4-PENTANDIONE ; LE 3-MERCAPTO-2-METHYLPENTANAL ; LA 3-MERCAPTO-2-OCTANONE ; LA 3-MERCAPTO-2-PENTANONE ; LA 3-MERCAPTO-4-PHENYL-2-BUTANONE ; LA 3-MERCAPTO-5-METHYL-HEXAN-2-ONE ; LA 3-METHYL-NON-2(E)-EN-4-ONE ; LA 3-METHYL-1-CYCLOPENATADECANONE ; LA 3-METHYL-2-CYCLOHEXEN-1-ONE ; LA 3-METHYL-2-CYCLOPENTEN-1-ONE ; LA 3-METHYL-2-HEPTEN-4,5-DIONE ; LA 3-METHYL-3-(3-METHYL-BUT-2-ENYL)-DIHYDRO-FURAN-2-ONE ; LA 3-METHYL-4-PHENYL-3-BUTEN-2-ONE ; LA 3-METHYL-5-PROPYL-2-CYCLOHEXEN-1-ONE ; LA 3-METHYLCYCLOHEXANONE ; LA 3-METHYLENE-2-OCTANONE ; LA 3-METHYL-GAMMA-DECALACTONE ; LE 3-METHYLPENTANAL ; LE 3-METHYLTHIOHEXANAL ; LA 3-NONANONE ; LA 3-NONEN-2-ONE ; LA 3-PENTANONE ; LA 3-PENTEN-2-ONE ; LA 3-PENTYL-4,5,5-TRIMETHYL-2-(5H)-FURANONE ; LE 3-PHENYL-4-PENTENAL ; LA 3-PROPYL-2-CYCLOPENTEN-1-ONE ; LA 3-PROPYLIDEN-HEPTAN-2-ONE ; LA 4-(2,6,6-TRIMETHYL-2-CYCLOHEXEN-1-YL)BUTAN-2-ONE ; LA 4-(2,6,6-TRIMETHYL-CYCLOHEXA-1,3-DIENYL)-BUT-3-EN-2-ONE ; LA 4-(FURAN-2-YL)-BUTAN-2-ONE ; LE 4-(METHYLTHIO)BUTANAL ; LA 4-(P-TOLYL)-2-BUTANONE ; LA 4,4-DIMETHOXY-3-HEXANONE ; LA 4,4-DIMETHYL-1,3-OXATHIANE-2-ONE ; LA 4,8-DIMETHYL-3,7-NONADIEN-2-ONE ; LA 4-[(2-FURANMETHYL)THIO]-2-PENTANONE ; LA 4-ACETOXY-HEX-4-EN-3-ONE ; LA 4-EPOXY-1,1,3-TRIMETHYLCYCLOHEX-5-EN-2-ONE ; LA 4-ETHYL-1,3-OXATHIAN-2-ONE ; LA 4-ETHYL-5-METHYL-1,3-OXATHIAN-2-ONE ; LA 4-HEPTYL-3-METHYLBUTYROLACTONE ; LA 4-HYDROXY-2,2,5-TRIMETHYL-3[2H]-FURANONE ; LA 4-HYDROXY-3,5,5-TRIMETHYL-CYCLOHEX-5,5-EN-1-ONE ; LA LACTONE D'ACIDE 4-HYDROXY-4-METHYL-5-HEXENOIQUE ; LA LACTONE D'ACIDE 4-HYDROXY-4-METHYL-CIS-7-DECANOIQUE ; LA 4-MERCAPTO-4-METHYL-PENTAN-2-ONE ; LE 4-METHYL-2-PENTENAL ; LA 4-METHYL-3-PENTEN-2-ONE ; LA 4-METHYL-4-FURFURYLTHIO-2-PENTANONE ; LA 4-METHYL-5-PENTYL-DIHYDRO-2(3H)-FURANONE ; LA 4-METHYLCYCLOHEXANONE ; LA 4-METHYLTHIO-2-PENTANONE ; LA 4-OXO-BETA-DAMASCONE ; LA 4-PHENYL-3,5-DITHIA-2-HEXANONE ; LA 5-ALPHA-ANDROST-16-EN-3-ONE ; LA 5,5-DIMETHYL-2(5H)-FURANONE ; LA 5,5-DIMETHYL-2-METHOXY-CYCLOHEX-2-ENONE ; LA 5-ETHYL-2-HYDROXY-3,4-DIMETHYL-2-CYCLOPENTEN-1-ONE ; LA 5-ETHYL-2-METHOXY-3,4-DIMETHYL-2-CYCLOPENTEN-1-ONE ; LA 5-ETHYLDIHYDRO-5-METHYL-2(3H)-FURANONE ; LA 5-HYDROXY-4-OCTANONE ; LA D-LACTONE D'ACIDE 5-HYDROXY-8-UNDECENOIQUE ; LA 5-METHYL-2-PROPYL-[1,3]-DIOXOLAN-4-ONE ; LA 5-METHYL-3-(3-METHYL-BUT-2-ENYL)-DIHYDRO-FURAN-2-ONE ; LA 5-METHYL-3-HEXEN-2-ONE ; LA 5-METHYL-4-HEXENE-2,3-DIONE ; LA 5-METHYL-5-HEXEN-2-ONE ; LA 5-PENTYL-ALPHA-PYRONE ; LA 5-P-TOLUYL-2(3H)-FURANONE ; LA 6-(1-PENTENYL)-2H-PYRAN-2-ONE ; LA 6-HEPT-1-ENYL-5,6-DIHYDRO-PYRAN-2-ONE ; LA LACTONE D'ACIDE 6-HYDROXY-3,7-DIMETHYLOCTANOIQUE ; LA 6-METHYL-3-HEPTEN-2-ONE ; LA 6-METHYL-NON-5-EN-4-ONE ; LE 6-METHYL-OCTANAL ; LA 6-PROPYL-[1,3]OXATHIAN-2-ONE ; LA 6-UNDECANONE ; LA 7-METHYL-2,3,4,4A,5,6-HEXAHYDRO-2-NAPHTALENONE ; LA 7-METHYL-6-OCTEN-2-ONE ; LE 7-OCTENAL ; LA 8-(2,2-DIMETHYLCYCLOPROPYL)-OCTA-3,5-DIEN-2-ONE ; LA 8A-METHYL-3,4,4A,5,8,8A-HEXAHYDRO-1(2H)-NAPHTHALENONE ; LE 8-METHYLNONANAL ; LA 8-METHYL-TRIDEC-7-EN-6-ONE ; LA 8-NONEN-2-ONE ; LE 8-NONENAL ; LE 9-DECENAL ; L'ACETONE ; L'ACETOPHENONE ; L'AR-TURMERONE ; LA BENZOPHENONE ; LA BENZOYLACETONE ; LA BENZYLDIPROPYLCETONE ; LA BENZYL-ACETONE ; LA BENZYLIDENE ACETONE ; LA BETA-IRONE ; LA BICYCLO-IRONE ; LA BICYCLONE ; LA BICYCLONONALACTONE ; LA BUTYRO-1,4-LACTONE ; LE CARYOPHYLLEN-12-AL ; LE CIS,CIS-4,7-DECADIEN-1-AL ; LE CIS-2-NONENAL ; LE CIS-3-HEXENAL ; LE CIS-3-NONENAL ; LA CIS-4-HEPTEN-2-ONE ; LE CIS-4-HEXENAL ; LE CIS-4-NONENAL ; LE CIS-5-NONENAL ; LE CIS-5-OCTENAL ; LE CIS-7-NONENAL ; LA CITRONELLENE-LACTONE ; LE CLONAL ; LA COGNAC-LACTONE ; LA CYCLOBUTONE ; LA CYCLOHEPTADECA-9-EN-1-ONE ; LA CYCLOHEXANONE ; LA CYCLOIONONE ; LA CYCLOPENTANONE ; LA CYLCOHEPTADECANONE ; LA DAMASCENONE ; LE DECANAL ; LA DECENYLCYCLOPENTANONE ; LA DIBENZYLCETONE ; LA 4,4-DIBUTYL-GAMMA-BUTYROLACTONE ; LA DIHYDROFILBERTONE ; LA DIHYDRO-5-METHYL-5-PROPYL-2(3H)-FURANONE ; LA DIHYDROCARVONE ; LA DIHYDRO-DESHYDRO-GAMMA-IONONE ; LA LACTONE DE DIHYDROJASMONE ; LA DIHYDROLAVENDELLACTONE ; LA DIMETHYLHYDROQUINONE ; LA DIMETHYLMETHOXYFURANONE ; LA 2,4-DIMETHYLACETOPHENONE ; LE 2,6-DIMETHYLOCTANAL ; LA 3,6-DIMETHYL-2(3H)-BENZOFURANONE ; LA DODEC-2-ENE-1,5-LACTONE ; LA DODEC-2-EN-4-ONE ; LE DODECANAL ; LA E-DECALACTONE ; LA E-DODECALACTONE ; LE 2-ETHYL-HEPTEN-2-AL ; LA 3-ETHYL-CYCLOPENTANDIONE ; LA FARNESYLACETONE ; LA FURFURYLIDENE-ACETONE ; LA GAMMA-DAMASCONE ; LA GAMMA-IONONE ; LA GERANYLACETONE ; LA GINGERONE ; L'HEPT-3-EN-2-ONE ; L'HEPTANAL ; L'HEXANAL ; L'HEXEN-5-ONE ; LA HEXENYLMETHYLCETONE ; L'HEXYLIDENECYCLOPENTANONE ; L'HOMOCORYLONE ; L'HYDROBICYCLONE ; L'HYDROXY-3(2)-HEPTAN-2(3)-ONE ; LA LACTONE D'ACIDE 5-HYDROXY-2,4-DECADIENOIQUE ; L'ISOJASMONE ; L'ISOLONGIFOLENE-CETONE ; L'ISOPHORONE ; LA LACTONE DE JASMIN ; LA LACTONE DE LAVANDE ; LA LACTONE DE MASSOIA ; LE MELONAL, LE DIMETHYLHEPTENAL ; LA MENTHONE ; LE METHIONAL ; LA METHOXY-P-PHENYLPENTEN-3-ONE, L'ETHONE ; LE 2-METHYLOCTANAL ; LE 2-METHYLPENTENE-1-AL ; LE METHYL-THIOMETHYLTHIOMETHYL-2-PENTENAL ; LE 2-METHYLUNDECANAL ; LE 2-METHYLBUTANAL ; LE 2-METHYLPENT-2-ENAL ; LA 2-METHYLTETRAHYDROFURANONE ; LA 4-METHYL-3(2H)-HYDROXYFURANONE ; LA 5-METHYLHEPT-2-EN-4-ONE ; LA 6-METHYL-3,5-HEPTADIENONE ; LE 3-METHYLTHIOBUTANAL ; LA 4-METHYLTHIOBUTAN-2-ONE ; LA LACTONE DE MENTHE ; LE MYRTENAL ; LA NEOFOLIONE ; LA DIHYDROCHALCONE DE NEOHESPERIDINE ; LE NONANAL ; LA NOOTKATONE ; L'OCTAN-2-ONE ; L'OCTANAL ; L'HUILE DE PENNYROYAL 84 % DE PULEGONE ; LA PENTADECANONE ; LE PENTANAL ; LA PENTYL-2-FURYLCETONE ; LE 2-PHENYLBUTEN-2-AL ; LE 3-PHENYLPROPANAL ; LA PIPERITENONE ; LA PIPERONYLACETONE ; LE P-MENTH-1-ENAL ; LE P-MENTH-1-EN-9-AL ; LA P-MENTHAN-2-ONE ; LE PRENAL ; LE PROPANAL ; L'ISOPROPYL-PARA-ACETOPHENONE ; LA RAC-3-HYDROXY-5-METHYL-2-(5H)-FURANONE ; LA CETONE DE FRAMBOISE ; LE SAFRANAL ; L'HUILE DE SAUGE 27 % DE THUJONE ; LA SOLANONE ; LA SPIRO-(6,5)-DODECAN-1-ONE ; LA TETRAHYDROIRONE ; LA TETRAHYDRONOOTKATONE ; LE TRANS-4-HEXENAL ; LE TRANS-5-NONENAL ; LE TRANS-6-DECEN-1-AL ; LE TRANS-6-NONENAL ; LE TRANS-7-METHYL-3-OCTEN-2-ONE ; LE TRANS-7-NONENAL ; LA TRIDECAN-2-ONE ; LE 3,5,5-TRIMETHYLHEXANAL ; LA TRITHIOACETONE ; L'UNDECANAL ; LA VANILLYLIDENE-ACETONE ; et LA VERBENONE.

3. Procédé selon l'une ou l'autre des revendications 1 et 2, **caractérisé en ce que** R₁ est un alkyle choisi dans le groupe constitué d'alkyle en C₇, C₈, C₉, C₁₀, C₁₁, C₁₃, C₁₅ et C₁₇.

4. Procédé selon l'une ou l'autre des revendications 1 et 2, **caractérisé en ce que** R₁ est un alcényle choisi dans le groupe constitué d'alcényle en C₇, C₈, C₉, C₁₀, C₁₁, C₁₃, C₁₅ et C₁₇, d' alcényle de (résidu d'acide oléique) en C₁₇-8ène, de 8,11-alcadiényle en C₁₇ (résidu d'acide linoléique), et de 8,11,14-triényle en C₁₇ (résidu d'acide linolénique).

5. Procédé selon l'une ou l'autre des revendications 1 et 2, **caractérisé en ce que** R₂ est choisi dans le groupe constitué de H, de méthyle et d'éthyle.

6. Procédé selon l'une ou l'autre des revendications 1 et 2, **caractérisé en ce que** R₃ est choisi dans le groupe constitué par un alkyle en C₁ à C₈ à chaîne droite, un alkyle en C₁ à C₈ ramifié comprenant jusqu'à deux groupements alkyle, le résidu alkyle pouvant contenir un ou plusieurs autres résidus alkyle, un alkyle en C₁ à C₈ à chaîne droite comprenant un ou deux substituants choisis parmi O, et SR₄, R₄ étant un résidu alkyle choisi parmi méthyle et éthyle, un alcényle en C₂ à C₈ à chaîne droite, et un cycle choisi parmi un cycle à 5 chaînons et à 6 chaînons, comprenant jusqu'à deux hétéroatomes de N, le cycle pouvant être en outre substitué par un ou plusieurs groupements alkyle choisis parmi méthyle, éthyle, propyle et isopropyle.

7. Procédé selon l'une ou l'autre des revendications 1 et 2, **caractérisé en ce que** R₁ est un alkyle choisi dans le groupe constitué par alkyle en C₇, C₉, C₁₁, C₁₃, C₁₅ et C₁₇, R₂ est choisi dans le groupe constitué par H, méthyle et éthyle, et R₃ est choisi dans le groupe constitué par un alkyle en C₁ à C₈ à chaîne droite, un alkyle en C₁ à C₈ ramifié comprenant un ou deux groupements alkyle, le résidu alkyle pouvant contenir un ou plusieurs autres résidus alkyle, un alkyle en C₁ à C₈ à chaîne droite comprenant un ou deux substituants choisis parmi O, et SR₄, R₄ étant un résidu alkyle choisi parmi méthyle et éthyle, un alcényle en C₂ à C₈ à chaîne droite, et un cycle choisi parmi un cycle à 5 chaînons et à 6 chaînons comprenant jusqu'à deux hétéroatomes de N, le cycle pouvant être en outre substitué par un ou plusieurs groupements alkyle choisis parmi méthyle, éthyle, propyle et isopropyle.

8. Composition d'arôme comprenant au moins un précurseur d'arôme tel que défini dans l'une quelconque des revendications 2 à 7.

9. Produit alimentaire comprenant au moins un précurseur d'arôme tel que défini dans l'une quelconque des revendications 1 à 7.

10. Composé précurseur d'arôme tel que défini dans l'une quelconque des revendications 1 à 9, **caractérisé en ce que** m = 1 et n = 0.

11. Composé précurseur d'arôme tel que défini dans l'une quelconque des revendications 2 à 9, **caractérisé en ce que** m = 0 et n = 1.

12. Procédé de production des composés précurseurs d'arôme selon la revendication 10 ou la revendication 11, en faisant réagir au moins un composé d'arôme comprenant un ou plusieurs groupements carbonyle avec au moins un monoglycéride dans une réaction catalysée par un acide.
